# EUROPEAN PATENT APPLICATION

(11) **EP 4 703 376 A1**
(43) Date of publication of application: **04.03.2026**
(21) Application number: 24795707.9
(22) Date of filing: 20.03.2024
(51) Int. Cl.: C07K 14/78, C07K 19/00, C12N 15/62, C12N 15/12, C12N 1/19, C12N 15/81, A61K 8/65, A61Q 19/00, A61K 38/39

(54) **RECOMBINANT MICROMOLECULAR COLLAGEN, EXPRESSION SYSTEM THEREOF AND PREPARATION METHOD THEREFOR**

(30) Priority: 28.04.2023 CN 202310481706
(71) Applicant: JIANGSU TRAUTEC MEDICAL TECHNOLOGY CO., LTD., Changzhou, Jiangsu 213251 (CN)
(72) Inventor: LI, Jiajia, Changzhou, Jiangsu 213251 (CN); CHENG, Pengfei, Changzhou, Jiangsu 213251 (CN); LIU, Huimin, Changzhou, Jiangsu 213251 (CN); QIAN, Chenming, Changzhou, Jiangsu 213251 (CN); JIANG, Wenwen, Changzhou, Jiangsu 213251 (CN); FAN, Xiaoju, Changzhou, Jiangsu 213251 (CN); LI, Haihang, Changzhou, Jiangsu 213251 (CN); QIAN, Song, Changzhou, Jiangsu 213251 (CN)
(74) Representative: Lorenz Seidler Gossel Part. mbB
(86) International application number: PCT/CN2024/082594
(87) International publication number: WO 2024/222324

(57) **Abstract**

The present invention provides a recombinant small-molecule collagen, an expression system thereof and a preparation method thereof, and belongs to the fields of synthetic biology, genetic engineering, and biotechnology. The present invention provides a recombinant small-molecule collagen derived from a plurality of sources of type-III and type-XVII collagens, and successfully expresses the recombinant small-molecule collagen. The present invention establishes one expression system of a small-molecule collagen and a preparation method of the small-molecule collagen, and includes an engineered exclusive chassis cell, a designed tandem repeat sequence of the small-molecule collagen, and a recombinant vector. The expression system or method of the present invention significantly enhances the yield of the small-molecule collagen. The present invention avoids the cost caused by in vitro protease digestion and the risk of exogenous protein residues, while also reducing the time and cost of a subsequent purification process.

## Description

### TECHNICAL FIELD

The present invention relates to a recombinant small-molecule collagen, an expression system thereof, and a preparation method thereof, and belongs to the fields of synthetic biology, genetic engineering, and biotechnology.

### BACKGROUND

A collagen possesses a plurality of biological functions, excellent biocompatibility, bioactivity, degradability, and other unique functional features, is an ideal source of a biomaterial, finds extensive applications across numerous sectors including chemical engineering, a pharmaceutical, food, and a cosmetic, and has broad application prospects. Currently, an animal-derived collagen dominates the market. In recent years, a genetically engineered recombinant collagen has begun to emerge. However, both the animal-derived collagen and a recombinant collagen are proteins with typically long amino acid sequences and high molecular weights. The animal-derived collagen generally has a molecular weight of 100-300 kDa or more, while the molecular weight of the recombinant collagen typically exceeds 20 kDa (over 200 amino acids), with 50 kDa being common. Such high-molecular-weight collagen is suitable for biomaterial applications in tissue regeneration and repair, an implantable medical device, and a delivery-type aesthetic medical product but is not ideal for an application scenario requiring transdermal absorption.

The animal-derived collagen with a smaller molecular weight is predominantly a small-molecule collagen peptide, which is frequently used in transdermal absorption application, such as a small-molecule collagen peptide sourced from an aquatic product. However, these products are typically prepared through enzymolysis, hydrolysis, or acid-base degradation. The resulted small-molecule collagen peptide is not a small-molecule collagen with an amino acid sequence, a defined molecular weight and a uniform molecular size. Instead, the resulted small-molecule collagen peptide represents a broad category encompassing everything from an extremely small oligopeptide to a small-molecule collagen peptide with a specific amino acid sequence length. When the type and quantity of the collagen peptide are virtually impossible to determine, the amino acid sequence is random (unknown). The quality of the small-molecule collagen peptides produced in different batches is also largely random, and the amino acid sequences, molecular weight distributions, and proportions of the small-molecule collagen peptides are all uncontrollable. In summary, an existing small-molecule collagen product has been extensively studied, but most are compounds derived from the animal-derived collagen processed through enzymolysis and hydrolysis. While the existing small-molecule collagen product has applications related to transdermal absorption, factors such as the amino acid sequence, the molecular weight, and batch-to-batch variability remain difficult to effectively control.

Disclosed in Patent application CN202211579848.5 is a small-molecule recombinant collagen peptide and a preparation method thereof. Essentially, the method remains a preparation method similar to that for an animal-derived small collagen peptide. The resulted small-molecule recombinant collagen peptide is a mixture of collagen peptides with diverse amino acid sequences and varying molecular weights (only the average molecular weight thereof can be calculated) but is not a single recombinant small-molecule collagen with an amino acid sequence and a single defined molecular weight (a polypeptide). Other existing recombinant small-molecule collagen peptides struggle to achieve a balance between a low molecular weight and biological activity. A recombinant collagen produced through synthetic biology and genetic engineering are predominantly medium-to-high molecular weight products (some R&D outcomes are labeled as peptides but contain well over 100 amino acids, exceeding the small-molecule category). Only the recombinant collagens of a handful of recombinant small-molecule collagen peptide study outcomes feature amino acid sequences with relatively low molecular weights. As an active substance, the collagen requires specific amino acid sequences and sequence lengths to support the biological activity thereof. That is, a balanced state between low molecular weight and biological activity is essential for being meaningful. However, the existing small-molecule recombinant collagen peptide lacks corresponding evidence demonstrating good biological activity.

Similar challenges exist for other small-molecule proteins or peptides during recombinant expression. The recombinant small-molecule collagen, characterized by fewer total amino acids and low molecular weight, faced significant limitations in expression efficiency and yield in recombinant expression systems-particularly eukaryotic expression systems like Pichia pastoris or mammalian cell expression systems. Some technical means, such as tandem expression to increase gene copy numbers, often introduce a non-essential amino acid into the small-molecule protein or peptide. The collagen is a unique protein characterized by the typical amino acid sequence of a G-X-Y triplet repeat structure. Introducing the non-essential amino acids frequently disrupts this triplet repeat structure, impairing the functional activity thereof.

Existing research on the recombinant small-molecule collagen or a multiple-recombinant small-molecule collagen peptide is scarce. A defined recombinant small-molecule collagen products is virtually nonexistent, and expression level issues are even less addressed. Some studies employ a method for fusing a leader peptide to enhance the expression amount of the small-molecule protein, such as Patent Publication CN108148114B. However, the small-molecule protein to be expressed requires protease digestion in vitro (in a cell) to remove the leader peptide. A foreign protein is artificially introduced into an expression product, which increases purification steps and costs while introducing a risk of a foreign protein residue. Additionally, some studies employ a method of tandem expression of small proteins to enhance expression efficiency and yield, such as CN110305890A, which employs recombinant expression for five antimicrobial peptides in tandem, but uses formic acid to cut a tandem polypeptide. A cleavage site can only be positioned between two amino acid residues of DP, which restricts a design space. Further, the cleaved DP cannot be effectively removed, becoming a non-natural amino acid-thus also introducing an exogenous protein. None of these solutions has been applied to the expression and production of the recombinant small-molecule collagen.

Therefore, the expression of the recombinant small-molecule collagen in the technical field requires a balanced method addressing a plurality of challenges: achieving a low molecular weight, maintaining biological activity, enabling scalable production, ensuring a high expression level, and ensuring a single, clearly defined amino acid sequence and molecular weight rather than a mixture. Therefore, an effective solution is urgently needed.

### SUMMARY

An objective of the present invention provides a recombinant small-molecule collagen, an expression system thereof, and a preparation method thereof to overcome some technical problems in the prior art.

To achieve the above objective, the present invention employs the following technical solutions:
The present invention first provides **a recombinant small-molecule collagen,** including an amino acid sequence shown in SEQ ID NO: 1, SEQ ID NO: 3, SEQ ID NO: 5, SEQ ID NO: 7, SEQ ID NO: 63, or SEQ ID NO: 67, or an amino acid sequence sharing identity of 80% or more, 85% or more, 90% or more, 95% or more, 96% or more, 97% or more, 98% or more, or 99% or more with SEQ ID NO: 3, SEQ ID NO: 5, SEQ ID NO: 7, SEQ ID NO: 63, or SEQ ID NO: 67, while maintaining the biological activity of the collagen.

Furthermore, a carboxy-terminus of the recombinant small-molecule collagen has a plurality of Hises capable of forming a 6×His Tag.

Furthermore, the recombinant small-molecule collagen includes the amino acid sequence shown in SEQ ID NO: 2, SEQ ID NO: 4, SEQ ID NO: 6, or SEQ ID NO: 8, or the amino acid sequence sharing identity of 80% or more, 85% or more, 90% or more, 95% or more, 96% or more, 97% or more, 98% or more, or 99% or more with SEQ ID NO: 2, SEQ ID NO: 4, SEQ ID NO: 6, or SEQ ID NO: 8, while maintaining the biological activity of the collagen. The present invention further provides a nucleic acid for encoding a recombinant small-molecule collagen. Furthermore, the nucleic acid includes nucleotide sequences shown in SEQ ID NOS: 9-16, or degenerate sequences thereof.

The present invention further provides a recombinant vector containing a nucleic acid. The vector includes pPICZαB, pFLDα, and pPIC9K, with a connecting site positioned between a cleavage site sequence of a Kex2 enzyme and a cleavage site of a NotI enzyme.

The present invention further provides a recombinant engineered strain containing a nucleic acid or a recombinant vector or expressing a recombinant small-molecule collagen.

Furthermore, host strains of the engineered strain include Pichia pastoris, Saccharomyces cerevisiae, and hansenula polymorpha, with Pichia pastoris being preferred.

According to an embodiment of the present invention, the engineered strain is deposited at China General Microbiological Culture Collection Center, with deposit numbers of CGMCC No.25811, CGMCC No.25823, CGMCC No. 25812, CGMCC No. 25824, CGMCC No. 25825, CGMCC No. 25813, CGMCC No. 25826, and CGMCC No. 25814.

The present invention further provides a **tandem repeat expression sequence of a recombinant small-molecule collagen.** The tandem repeat expression sequences are connected in series and repeated by taking as basic units the recombinant small-molecule collagen, an artificially designed collagen with a typical G-X-Y triplet structure, or a protein combined by two or more regions of a human collagen sequence. Sites recognized and cleaved by Kex2 and CPB enzymes are provided between every the two adjacent recombinant small-molecule collagens in the tandem repeat expression sequence, and sites recognized and cleaved by a Ste13 enzyme can additionally be provided between every the two adjacent recombinant small-molecule collagens in the tandem repeat expression sequence at the same time.

The tandem repeat expression sequence includes sequences shown in SEQ ID NO: 30, SEQ ID NO: 33, SEQ ID NO: 36, SEQ ID NO: 39, SEQ ID NO: 42, SEQ ID NO: 45, SEQ ID NO: 48, SEQ ID NO: 51, SEQ ID NO: 54, SEQ ID NO: 57, SEQ ID NO: 60, SEQ ID NO: 64, and SEQ ID NO: 68, or amino acid sequences sharing identity of 80% or more, 85% or more, 90% or more, 95% or more, 96% or more, 97% or more, 98% or more, or 99% or more with the above sequences.

According to an embodiment of the present invention, the site recognized and cleaved by the Kex2 enzyme includes a KR or RR dibasic amino acid residue, followed by an EA, an EAEA, or other amino acid residues that facilitate recognition and cleavage by the Kex2 enzyme or the Ste13 enzyme.

According to an embodiment of the present invention, the site recognized and cleaved by the CPB enzyme includes a basic amino acid residue at a carboxy terminus of a protein, including K and R.

According to an embodiment of the present invention, an amino acid sequence of a tandem repeat small-molecule collagen monomer (or fragment) can be any sequence, with no restrictions on the specific order of amino acid residues, sequence length, or the number of tandem repeats in the monomer (or the fragment), provided that the amino acid sequence possesses the typical G-X-Y triplet structure.

The present invention further provides a nucleic acid for encoding a tandem repeat expression sequence. Furthermore, the nucleic acid further includes sequences shown in SEQ ID NOS: 71-81 or degenerate sequences thereof.

The present invention further provides a recombinant vector containing a nucleic acid for encoding a tandem repeat expression sequence. According to an embodiment of the present invention, the vector includes but is not limited to expression vectors such as pPICZαB, pFLDα, and pPIC9K.

The present invention further provides an engineered strain containing a nucleic acid or a recombinant vector.

According to an embodiment of the present invention, a host strain of the engineered strain includes Pichia pastoris, Saccharomyces cerevisiae, and hansenula polymorpha, with Pichia pastoris being preferred. According to an embodiment of the present invention, the engineered strain is deposited at China General Microbiological Culture Collection Center, with deposit numbers of CGMCC No.25819, CGMCC No.25821, CGMCC No.25827, CGMCC No.25829, CGMCC No.25828, CGMCC No.25820 and CGMCC No.25822.

The present invention further provides a **method for obtaining a recombinant small-molecule collagen,** including:
constructing a tandem repeat expression sequence of the recombinant small-molecule collagen described herein.
constructing a positioning fusion functional protein; and transforming a positioning fusion functional protein-linked vector into a host strain to obtain a chassis cell or a chassis engineered strain;
transferring the tandem repeat expression sequence of the recombinant small-molecule collagen into a chassis cell or a chassis engineered strain after being connected to an expression vector to obtain a recombinant engineered strain containing or expressing the recombinant small-molecule collagen; inducing an expression through fermentation to obtain the recombinant small-molecule collagen.

According to an embodiment of the present invention, a positioning fusion functional protein includes a CPB enzyme and a functional region with intracellular membrane positioning or conversion and transport functions among respective organelles.

Furthermore, the positioning fusion functional protein further includes a connecting sequence for connecting the CPB enzyme to the functional region sequence with the intracellular membrane positioning or the conversion and transport functions among the respective organelles during fusion expression. Preferably, the connecting sequence is a Linker sequence such as GGSGSGSGGS as shown in SEQ ID NO: 21.

According to an embodiment of the present invention, preferably, the CPB enzyme is derived from human or a rat. Sequences of the CPB enzyme are as shown in SEQ ID NOS: 17-18.

The functional region sequence with the intracellular membrane positioning or the conversion and transport functions among the respective organelles is derived from the Kex2 enzyme of Saccharomyces cerevisiae or Pichia pastoris, or other functional regions of a protein with similar functions, such as a Ste13 protease.

Furthermore, the functional region sequences with the intracellular membrane positioning or the conversion and transport functions among the respective organelles are as shown in SEQ ID NOS: 19-20.

According to an embodiment of the present invention, sites recognized and cleaved by the Kex2 enzyme and the CPB enzyme are provided between every the two adjacent recombinant small-molecule collagens in the tandem repeat expression sequence, and sites recognized and cleaved by a Ste13 enzyme can additionally be included between every the two adjacent recombinant small-molecule collagens in the tandem repeat expression sequence. The small-molecule collagen is preferably the recombinant small-molecule collagen described herein.

According to an embodiment of the present invention, the site recognized and cleaved by the Kex2 enzyme includes a KR or RR dibasic amino acid residue, followed by an EA, an EAEA, or other amino acid residues that facilitate recognition and cleavage by the Kex2 enzyme or the Ste13 enzyme.

According to an embodiment of the present invention, the site recognized and cleaved by the CPB enzyme includes a basic amino acid residue at a carboxy terminus of a protein, including K and R.

Furthermore, the recombinant small-molecule collagen has a typical G-X-Y triplet structure. Preferably, the small-molecule collagen is the recombinant small-molecule collagen described herein, an artificially designed collagen with the typical G-X-Y triplet structure, or a collagen combined by two or more regions of a human collagen sequence.

According to an embodiment of the present invention, the vector includes but is not limited to expression vectors such as pPICZαB, pFLDα, and pPIC9K. According to an embodiment of the present invention, the recombinant engineered strain containing or expressing the recombinant small-molecule collagen is deposited at China General Microbiological Culture Collection Center, with deposit numbers of CGMCC No. 25819, CGMCC No. 25821, CGMCC No. 25827, CGMCC No. 25828, CGMCC No. 25829, CGMCC No. 25820, and CGMCC No. 25822.

According to an embodiment of the present invention, the recombinant small-molecule collagen expressed by the above method includes an amino acid sequence shown in SEQ ID NO: 32, SEQ ID NO: 35, SEQ ID NO: 38, SEQ ID NO: 41, SEQ ID NO: 44, SEQ ID NO: 47, SEQ ID NO: 50, SEQ ID NO: 53, SEQ ID NO: 56, SEQ ID NO: 59, SEQ ID NO: 62, SEQ ID NO: 66, or SEQ ID NO: 70, or an amino acid sequence sharing identity of 80% or more, 85% or more, 90% or more, 95% or more, 96% or more, 97% or more, 98% or more, 99% or more of SEQ ID NO: 32, SEQ ID NO: 35, SEQ ID NO: 38, SEQ ID NO: 41, SEQ ID NO: 44, SEQ ID NO: 47, SEQ ID NO: 50, SEQ ID NO: 53, SEQ ID NO: 56, SEQ ID NO: 59, SEQ ID NO: 62, SEQ ID NO: 66, or SEQ ID NO: 70.

**The present invention further provides a positioning fusion functional protein.** The positioning fusion functional protein is configured for the cleavage and removal of C-terminus basic amino acid residues (K, R) after a plurality of tandem repeat recombinant small-molecule collagens are cleaved into monomers by a Kex2 enzyme.

The positioning fusion functional protein includes a mature peptide sequence of a CPB enzyme and a functional region sequence with intracellular membrane positioning or conversion and transport functions among respective organelles.

Furthermore, the positioning fusion functional protein further includes a connecting sequence for connecting the CPB enzyme to the functional region sequence with the intracellular membrane positioning or the conversion and transport functions among the respective organelles during fusion expression.

According to an embodiment of the present invention, the CPB enzyme is derived from human or a rat. Furthermore, the CPB enzyme is preferably sequences shown in SEQ ID NOS: 17-18.

According to an embodiment of the present invention, the functional region sequence with the intracellular membrane positioning or the conversion and transport functions among the respective organelles is derived from a Kex2 enzyme of Saccharomyces cerevisiae or Pichia pastoris, or other functional regions of a protein with similar functions, such as a Ste13 protease.

Furthermore, the sequences of the CPB enzyme are as shown in SEQ ID NOS: 17-18. The functional region sequences with the intracellular membrane positioning or the conversion and transport functions among the respective organelles are as shown in SEQ ID NOS: 19-20.

According to an embodiment of the present invention, a connecting sequence is a Linker sequence such as GGSGSGSGGS as shown in SEQ ID NO: 21.

According to an embodiment of the present invention, the positioning fusion functional protein includes an amino acid sequence shown in SEQ ID NO: 22, SEQ ID NO: 24, SEQ ID NO: 26, or SEQ ID NO: 28, or an amino acid sequence sharing identity of 80% or more, 85% or more, 90% or more, 95% or more, 96% or more, 97% or more, 98% or more, or 99% or more with SEQ ID NO: 22, SEQ ID NO: 24, SEQ ID NO: 26, or SEQ ID NO: 28.

The present invention further provides a nucleic acid for encoding a positioning fusion functional protein described herein.

According to an embodiment of the present invention, the nucleic acid for encoding the positioning fusion functional protein of the present invention includes a nucleotide sequence shown in SEQ ID NO: 23, SEQ ID NO: 25, SEQ ID NO: 27, or SEQ ID NO: 29, or a degenerate sequence thereof.

The present invention further provides a recombinant vector containing a nucleic acid for encoding a positioning fusion functional protein of the present invention.

According to an embodiment of the present invention, the vector includes but is not limited to expression vectors such as pPICZαB, pFLDα, and pPIC9K, with a connecting site positioned between a site sequence of a Kex2 enzyme and a termination codon "TGA" on the vector.

The present invention further provides **a chassis cell or a chassis engineered strain.** The chassis cell or the chassis engineered strain contains a nucleic acid for encoding a positioning fusion functional protein of the present invention, contains a recombinant vector of a nucleic acid for encoding a positioning fusion functional protein of the present invention, or expresses a positioning fusion functional protein.

According to an embodiment of the present invention, a host strain of the engineered strain includes Pichia pastoris, Saccharomyces cerevisiae, or hansenula polymorpha, with Pichia pastoris being preferred. The constructed chassis cell or the chassis engineered strain is deposited at China General Microbiological Culture Collection Center, with deposit numbers of CGMCC No. 25815, CGMCC No. 25817, CGMCC No. 25816, CGMCC No. 25818.

**The present invention further provides an expression system for a recombinant small-molecule collagen.** The expression system includes a positioning fusion functional protein, a chassis cell or a chassis engineered strain, and the tandem repeat expression sequence of the recombinant small-molecule collagen.

The present invention further provides application of a positioning fusion functional protein, a chassis cell or a chassis engineered strain, and a tandem repeat expression sequence of a recombinant small-molecule collagen in expressing the recombinant small-molecule collagen.

The recombinant small-molecule collagen described in the present invention, or the small-molecule collagen obtained by the above method, has cell adhesion activity, cell proliferation-promoting activity, and good skin permeability.

The present invention further provides use of a recombinant small-molecule collagen, a nucleic acid, a recombinant vector, a host cell or a recombinant engineered strain, or a composition, or a product in preparation of a pharmaceutical, a medical device, a biomaterial, a tissue engineering product, a cosmetic, or a health care product.

### Advantages of the present invention:

(1) The present invention first provides a recombinant small-molecule collagen derived from a plurality of sources of Type-III and Type-XVII collagens, and expresses the recombinant small-molecule collagen from the plurality of sources of Type-III and Type-XVII collagens. Although the amino acid sequence lengths and molecular weights of the collagens differ, the collagens can all be expressed as the small-molecule collagen with a single band and a clearly defined amino acid sequence. At the same time, the collagens have excellent transdermal absorption performance and biological activity, achieving a balance among a small molecular size, transdermal absorption, and biological activity. Furthermore, large-scale production has been realized in a 500L-scale fermenter, enabling the widespread application of the recombinant small-molecule collagen. The present invention addresses the problem of lack of a current product of a recombinant small-molecule collagen, particularly the recombinant small-molecule collagens derived from Type-III and Type-XVII collagens.
(2) The present invention establishes one expression system for the recombinant small-molecule collagen and a preparation method for the small-molecule collagen, including the construction of a dedicated chassis cell and a design method for a collagen tandem repeat sequence. The technical solution of the present invention significantly enhances the yield of the expression of the small-molecule collagen and avoids introducing an exogenous protein or employing any in vitro enzymatic cleavage. During intracellular secretion, the small-molecule collagen removes a residual amino acid residue of the cleavage site of the enzyme during repeat, tandem, and design, yielding recombinant small-molecule collagen free of a non-collagen sequence or exhibiting 100% homology with a corresponding region of a natural collagen. The entire expression system avoids the cost associated with in vitro protease enzymatic cleavage and the risk of an exogenous protein residue, while also reducing the time and cost of a subsequent purification process.

The small-molecule collagen obtained from the present invention is underwent with N-terminus and C-terminus as well as full-sequence verification, demonstrating the efficacy of the entire expression system-particularly the chassis cell engineered strain as a host strain for expressing the recombinant small-molecule collagen. Furthermore, validation through fermentation and purification in a 500L-scale fermentation tank confirms the feasibility for large-scale industrial production. Results indicate that the expression system constructed using a repeat tandem expression strategy can increase the yield of the recombinant small-molecule collagen by 4-5 times.

(3) The cell adhesion activity, cell proliferation-promoting activity, and transdermal absorption of the recombinant small molecules of the present invention are evaluated. The results indicate that, in addition to exhibiting excellent transdermal absorption, the recombinant small-molecule collagen possesses cell adhesion activity comparable to those of a natural collagen and a homologous high-molecular-weight recombinant collagen, along with cell proliferation-promoting activity.

### BRIEF DESCRIPTION OF THE DRAWINGS

FIG. 1 shows the Tricine-PAGE inspection results of eight purified recombinant small-molecule collagens: 3A5D1NT, 3A5D1, 3A5D2NT, 3A5D2, 17S1NNT, 17S1N, 17S3NT, and 17S3.
FIG. 2 shows the deconvoluted molecular weight results obtained by LC-MS analysis of recombinant small-molecule collagens 3A5D1NT and 3A5D1 expressed in the present invention.
FIG. 3 shows the deconvoluted molecular weight results obtained by LC-MS analysis of recombinant small-molecule collagens 3A5D2 expressed in the present invention.
FIG. 4 shows the deconvoluted molecular weight results obtained by LC-MS analysis of recombinant small-molecule collagens 17S1NNT and 17S1N expressed in the present invention.
FIG. 5 shows the deconvoluted molecular weight results obtained by LC-MS analysis of recombinant small-molecule collagens 17S3NT and 17S3 expressed in the present invention.
FIG. 6 shows the Western blot inspection results for the N-terminus 6×His tags of 3A5D1, 3A5D2, 17S1N, and 17S3 using an anti-6×His Tag antibody. In the figure, + indicates positive control, and - indicates negative control.
FIG. 7 shows the results of comparing a peptide fragment obtained by trypsin enzymolysis of a lyophilized product of recombinant small-molecule collagens 3A5D1 and 3A5D1NT from Embodiment 1 of the present invention with an original sequence thereof after Nano-HPLC-MS/MS mass spectrometry inspection.
FIG. 8 shows the results of comparing a peptide fragment obtained by trypsin enzymolysis of a lyophilized product of recombinant small-molecule collagens 3A5D2 and 3A5D2NT from Embodiment 1 of the present invention with an original sequence thereof after Nano-HPLC-MS/MS mass spectrometry inspection.
FIG. 9 shows the results of comparing a peptide fragment obtained by trypsin enzymolysis of a lyophilized product of recombinant small-molecule collagens 17S1N and 17S1NNT from Embodiment 1 of the present invention with an original sequence thereof after Nano-HPLC-MS/MS mass spectrometry inspection.
FIG. 10 shows the results of comparing a peptide fragment obtained by trypsin enzymolysis of a lyophilized product of recombinant small-molecule collagens 17S3 and 17S3NT from Embodiment 1 of the present invention with an original sequence thereof after Nano-HPLC-MS/MS mass spectrometry inspection.
FIG. 11 shows a map of a recombinant expression vector pPICZαB-RCPB-SCKEX2 in the present invention.
FIG. 12 shows a map of a recombinant expression vector pPICZαB-HCPB-SCKEX2 in the present invention.
FIG. 13 shows a map of a recombinant expression vector pPICZαB-RCPB-PPKEX2 in the present invention.
FIG. 14 shows a map of a recombinant expression vector pPICZαB-HCPB-PPKEX2 in the present invention.
FIG. 15 shows detection results of CPB positioning fusion functional proteins HCPB-PPKEX2, HCPB-SCKEX2, RCPB-PPKEX2, and RCPB-SCKEX2 expressed in an intracellular lysate and a culture supernatant.
   Lanes in the left figure: 1: HCPB-SCKEX2 strain lysate; 2: HCPB-SCKEX2 strain culture supernatant; 3: 6: HCPB-PPKEX2 culture supernatant; 4, 5: HCPB-PPKEX2 strain lysate; (-): Negative control; (+): Positive control, recombinant human CPB enzyme; /: Non-sample well.
   Lanes in the right figure: (-): Negative control; (+): Positive control, a CPB enzyme of a recombinant rat; /: Non-sample well; 7: an RCPB-SCKEX2 strain culture supernatant; 8: a RCPB-SCKEX2 strain lysate; 9, 10, 12, 13: an RCPB-PPKEX2 culture supernatant; 11, 14: an RCPB-PPKEX2 strain lysate.

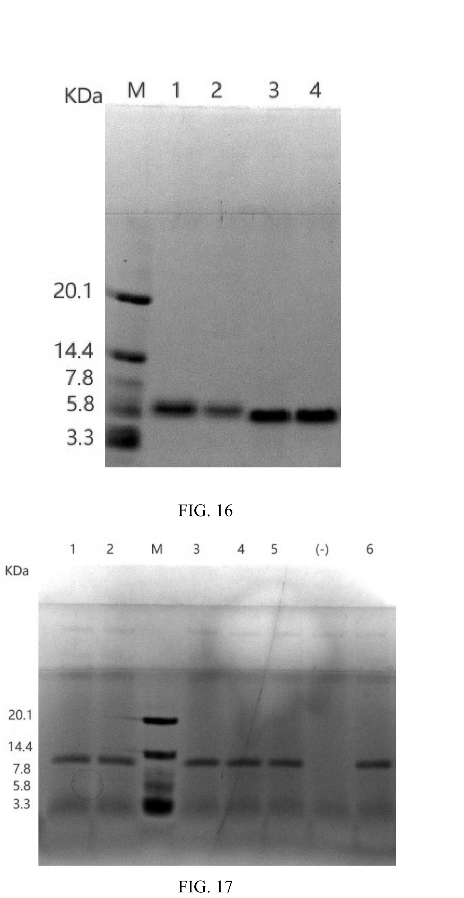

FIG. 16 shows the SDS-PAGE inspection results of a supernatant from expression of 3A5D29N-9 and 17S28-8 in HCPB-PPKEX2 and RCPB-PPKEX2 chassis cell engineered strains, respectively.
   The lanes in the figure represent: 1: the supernatant from expression of 3A5D29N-9 in the HCPB-PPKEX2 chassis cell engineered strain; 2: the supernatant from expression of 3A5D29N-9 in the RCPB-PPKEX2 chassis cell engineered strain; 3: the supernatant from expression of 17S28-8 in the HCPB-PPKEX2 chassis cell engineered strain; 4: the supernatant from expression of 17S28-8 in the RCPB-PPKEX2 chassis cell engineered strain.
FIG. 17 shows the SDS-PAGE inspection results of a supernatant from expression of 17S1N6-6, 17S1N7-7 and 17S1NK-7 in HCPB-PPKEX2 and RCPB-PPKEX2 chassis cell engineered strains, respectively.
   The lanes in the figure are: 1: the supernatant from expression of 17S1N6-6 in the HCPB-PPKEX2 chassis cell engineered strain; 2: the supernatant from expression of 17S1N6-6 in an RCPB-PPKEX2 chassis cell engineered strain; 3: the supernatant from expression of 17S1N7-7 in the HCPB-PPKEX2 chassis cell engineered strain; 4: the supernatant from expression in 17S1N7-7 in the RCPB-PPKEX2 chassis cell engineered strain; 5: the supernatant from expression of 17S1NK-7 in the HCPB-PPKEX2 chassis cell engineered strain; 6: the supernatant from expression of 17S1NK-7 in the RCPB-PPKEX2 chassis cell engineered strain; (-) Negative control: a pPIC9K empty vector transferring into the HCPB-PPKEX2 chassis cell engineered strain to express the supernatant.

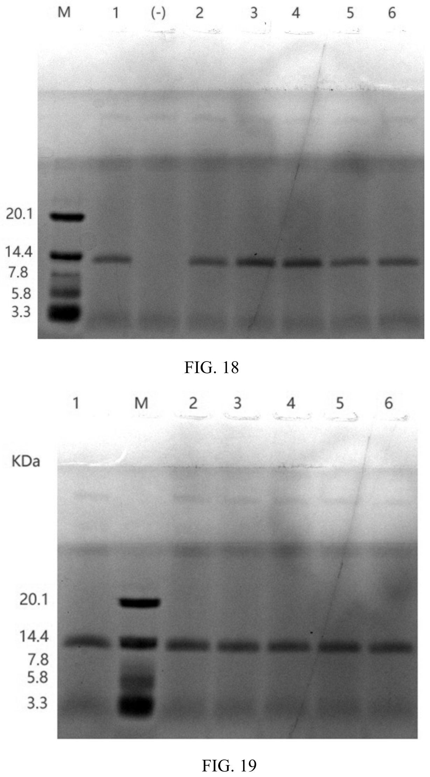

FIG. 18 shows a supernatant from expression of 3A5D15D-5, 3A5D15E-5, 3A5D15G-6, and 3A5D15EKR-6 in the RCPB-PPKEX2 chassis cell engineered strain, and SDS-PAGE inspection results of a supernatant from expression of 3A5D15R-6 in an HCPB-RPKEX2 chassis cell engineered strain.
   The lanes in the figure are: 1: the supernatant from expression of 3A5D15D-5 in the RCPB-PPKEX2 chassis cell engineered strain; 2: the supernatant from expression of 3A5D15E-5 in the RCPB-PPKEX2 chassis cell engineered strain; 3: the supernatant from expression of 3A5D15G-6 in the RCPB-PPKEX2 chassis cell engineered strain; 4: the supernatant from expression of 3A5D15EKR-6 in the RCPB-PPKEX2 chassis cell engineered strain; 5: the supernatant from expression of 3A5D15KR-6 in the RCPB-PPKEX2 chassis cell engineered strain; 6: the supernatant from expression of 3A5D15R-6 in the HCPB-RPKEX2 chassis cell engineered strain; (-): Negative control, the supernatant from expression of a pPIC9K empty vector transferred into the RCPB-RPKEX2 chassis cell engineered strain.
FIG. 19 shows SDS-PAGE inspection results of a supernatant from expression of 3A5D15D-5, 3A5D15E-5, 3A5D15G-6, A5D15EKR-6, 3A5D15KR-6, and 3A5D15R-6 in HCPB-PPKEX2 chassis cell engineered strains.
   The lanes in the figure represent: 1: the supernatant from expression of 3A5D15D-5 in the HCPB-PPKEX2 chassis cell engineered strain; 2: the supernatant from expression of 3A5D15E-5 in the HCPB-PPKEX2 chassis cell engineered strain; 3: the supernatant from expression of 3A5D15G-6 in the HCPB-PPKEX2 chassis cell engineered strain; 4: the supernatant from expression of 3A5D15EKR-6 in the HCPB-PPKEX2 chassis cell engineered strain; 5: the supernatant from expression of 3A5D15KR-6 in the HCPB-PPKEX2 chassis cell engineered strain; 6: the supernatant from expression of 3A5D15R-6 in the HCPB-PPKEX2 chassis cell engineered strain.

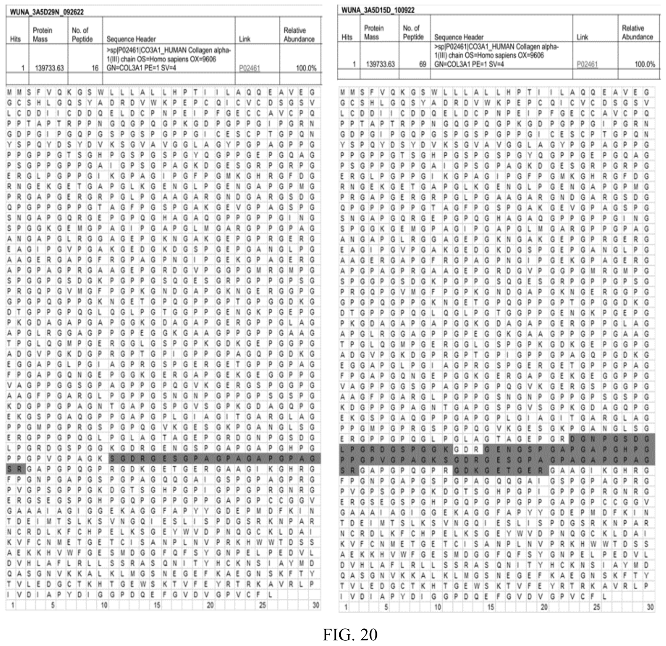

FIG. 20 shows the results of comparing a peptide fragment obtained by trypsin enzymolysis of lyophilized products of 3A5D29N and 33A5D15D with original sequences thereof after Nano-HPLC-MS/MS mass spectrometry inspection.

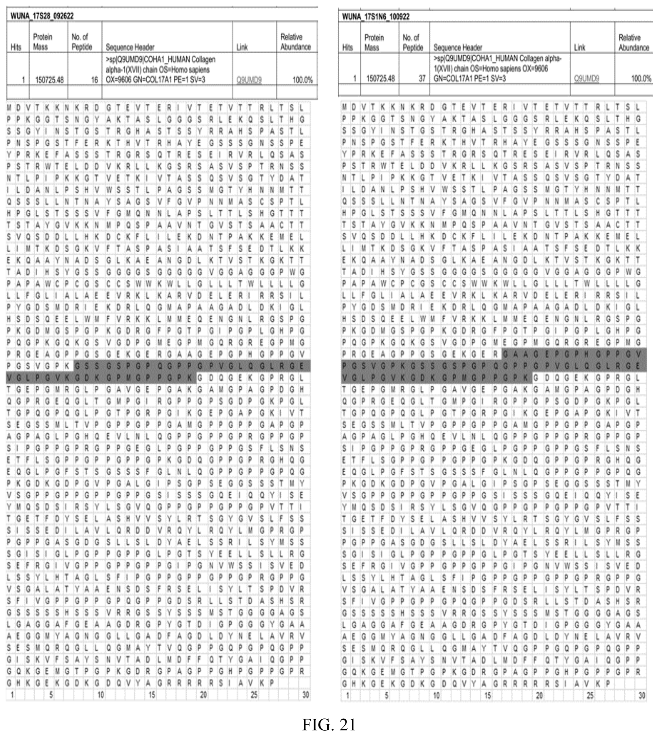

FIG. 21 shows the results of comparing a peptide fragment obtained by trypsin enzymolysis of lyophilized products of 17S28 and 17S1N6 with original sequences thereof after Nano-HPLC-MS/MS mass spectrometry inspection.

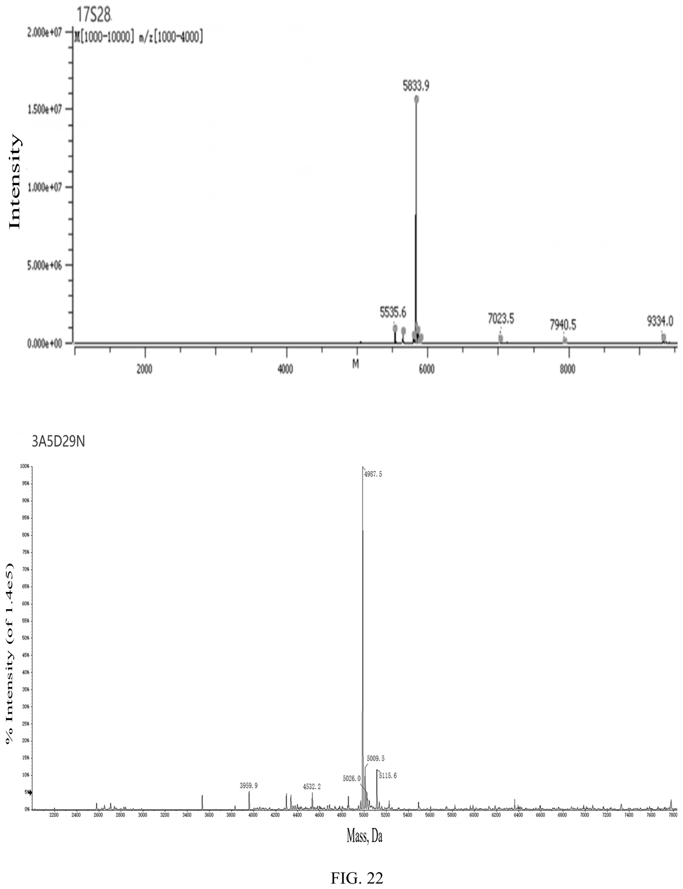

FIG. 22 shows the deconvoluted molecular weight results of 3A5D29N and 17S28 obtained by LC-MS analysis.

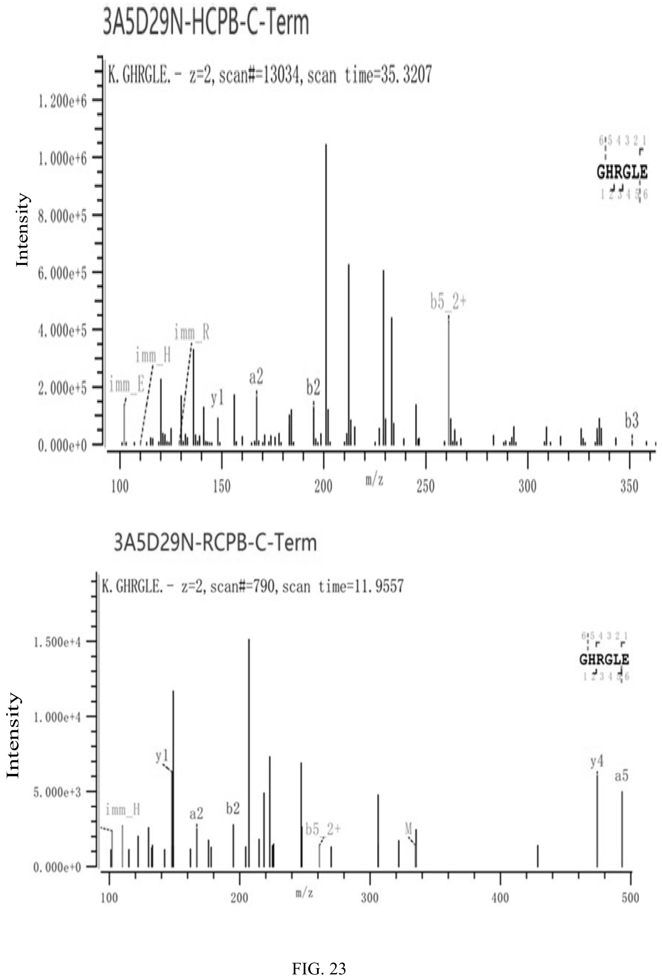

FIG. 23 shows a secondary mass spectrum of a C-terminus peptide fragment of recombinant small-molecule collagen 3A5D29N expressed in the HCPB-PPKEX2 and RCPB-PPKEX2 chassis cell engineered strains.

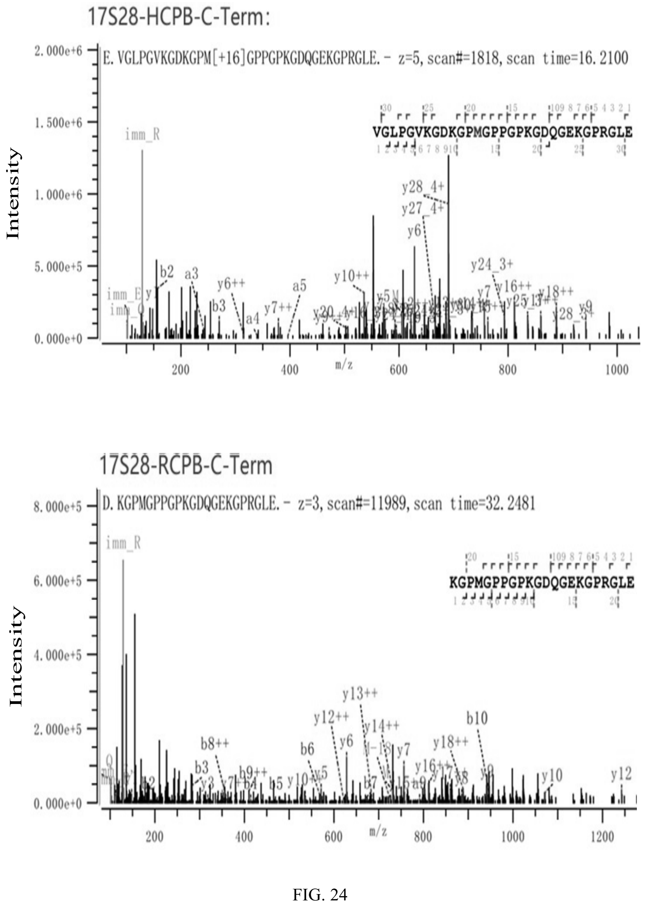

FIG. 24 shows a secondary mass spectrum of a C-terminus peptide fragment of recombinant small-molecule collagen 17S28 expressed in the HCPB-PPKEX2 and RCPB-PPKEX2 chassis cell engineered strains.

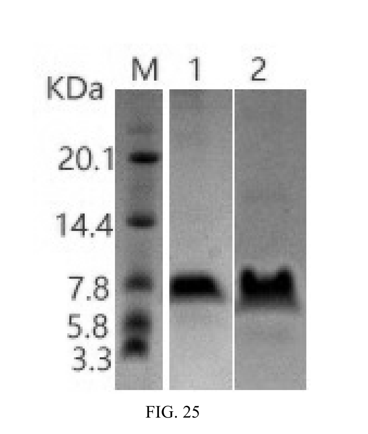

FIG. 25 shows SDS-PAGE inspection results of a fermentation supernatant from an engineered strain expressing 3A5D2NT and 3A5D29N after induction for a specific duration.
   The lanes in the figure represent: Lane 1: a fermentation supernatant from an engineered strain expressing 3A5D2NT (CGMCC No. 25812) after induction for 48 hours; Lane 2: a fermentation supernatant from an engineered strain expressing 3A5D29N (CGMCC No. 25819) after induction for 43 hours.

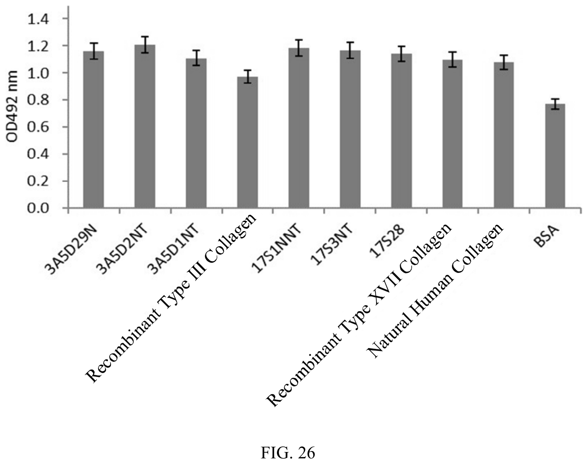

FIG. 26 shows the comparative results of cell adhesion activity for recombinant small-molecule collagens 3A5D29N, 3A5D2NT, 3A5D1NT, 17S1NNT, 17S3NT, 17S28, a recombinant type III collagen, a recombinant type XVII collagen, and a natural human collagen.

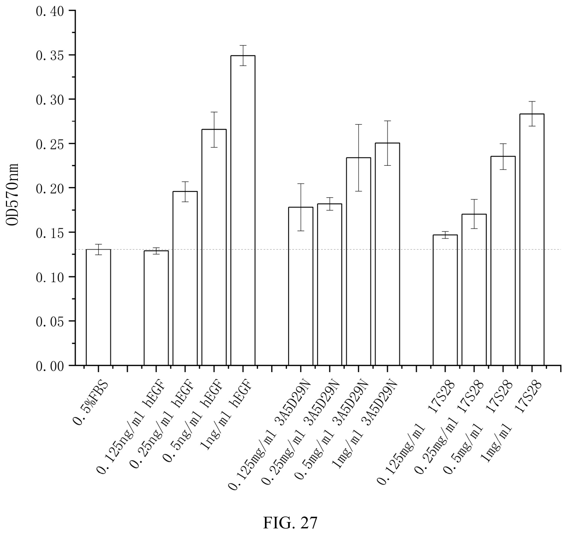

FIG. 27 shows the validation results of the cell proliferation-promoting effect of recombinant small-molecule collagens 3A5D29N and 17S28.

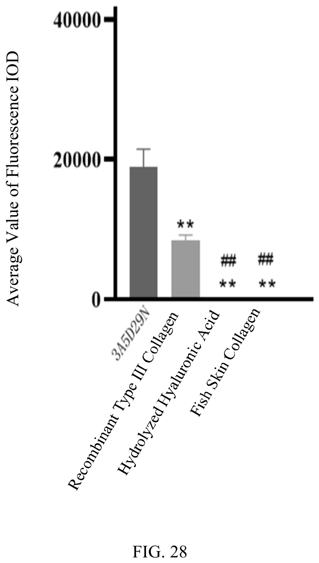

FIG. 28 shows the average values of fluorescence IOD detected in a full-thickness skin model tissue after a 1 mg/mL recombinant small-molecule collagen 3A5D29N, a recombinant type III collagen, a hydrolyzed hyaluronic acid, and a fish skin collagen are applied for 1 hour. Here, * denotes significance relative to the 1 mg/mL recombinant small-molecule collagen 3A5D29N (* indicates significant difference, P < 0.05; ** indicates highly significant difference, P < 0.01), while # denotes significance relative to the recombinant type III collagen (# indicates significant difference, P < 0.05; ## indicates highly significant difference, P *<* 0.01).

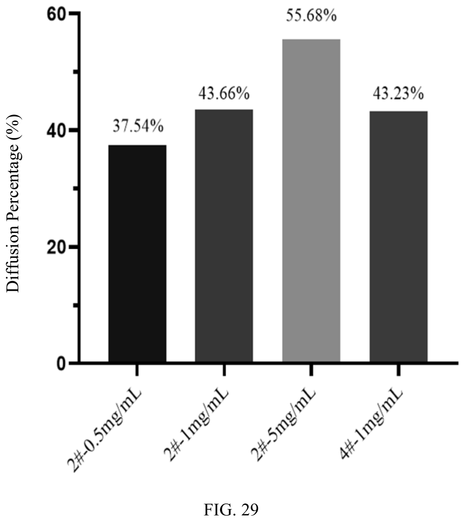

FIG. 29 shows the percentage diffusion (i.e., cumulative permeability) of a sample permeated into a culture solution after 12-hour application of a recombinant small-molecule collagen 3A5D29N in different concentrations and a 1 mg/mL hydrolyzed hyaluronic acid. 2# represents the recombinant small-molecule collagen 3A5D29N, and 4# represents the hydrolyzed hyaluronic acid.

### DETAILED DESCRIPTION OF THE EMBODIMENTS

To enable a person skilled in the art to better understand the technical solutions of the present invention, the following detailed description provides preferred embodiments of the present invention. However, the scope of protection of the present invention is not limited to the following embodiments.

In the embodiments of the present invention, unless otherwise specified, conventional experimental methods are employed. Processes described herein that are not elaborated upon are understood and readily achievable by a person skilled in the art based on product manuals or fundamental knowledge in the art, and thus are not described in detail again.

### Embodiment 1: Single-sequence recombinant expression of recombinant small-molecule collagen

### (1) The amino acid sequence design of the single-sequence recombinant expression strategy of a recombinant small-molecule collagen

Amino acid sequences from positions 990 to 1085 of a full-length human type III collagen (including an N-terminus propeptide, a mature polypeptide chain, and a C-terminus propeptide) was selected (refer to the protein sequence with ID P02461 in Uniprot database, https://www.uniprot.org/uniprotkb/P02461). The recombinant small-molecule collagen expressed from this sequence was designated as 3A5D1NT, including 96 amino acids with a theoretical molecular weight of 8596.08 Da. A sequence thereof was shown in SEQ ID NO: 1:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIKGH

By adding five histidine (H) residues to a C-terminus of the 3A5D1NT sequence, the recombinant small-molecule collagen expressed from this sequence was designated as 3A5D1, including 101 amino acids with a theoretical molecular weight of 9281.78 Da. A sequence thereof is shown in SEQ ID NO: 2:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIKGHHHHHH

Amino acid sequences from positions 1036 to 1085 of a full-length human type III collagen (including an N-terminus propeptide, a mature polypeptide chain, and a C-terminus propeptide) was selected (refer to the protein sequence with ID P02461 in Uniprot database, https://www.uniprot.org/uniprotkb/P02461). The recombinant small-molecule collagen expressed from this sequence was designated as 3A5D2NT, including 50 amino acids with a theoretical molecular weight of 4532.83Da. A sequence thereof was shown in SEQ ID NO: 3:
GKSGDRGESGPAGPAGAPGPAGSRGAPGPQGPRGDKGETGERGAAGIKGH

By adding five histidine (H) residues to a C-terminus of the 3A5D2NT sequence, the recombinant small-molecule collagen expressed from this sequence was designated as 3A5D2, including 55 amino acids with a theoretical molecular weight of 5218.54 Da. A sequence thereof is shown in SEQ ID NO: 4:

GKSGDRGESGPAGPAGAPGPAGSRGAPGPQGPRGDKGETGERGAAGIKGHHHHH
H

The amino acid sequence from positions 636 to 718 of a full-length human collagen XVII sequence (refer to protein ID Q9UMD9-1 in the Uniprot database, https://www.uniprot.org/uniprot/Q9UMD9) was selected. The recombinant small-molecule collagen expressed from this sequence fragment was designated as 17S1NNT, including 82 amino acids with a theoretical molecular weight of 7483.28 Da. A sequence thereof was shown in SEQ ID NO: 5:

GPPGSGEKGERGAAGEPGPHGPPGVPGSVGPKGSSGSPGPQGPPGPVGLQGLRGE
VGLPG VKGDKGPMGPPGPKGDQGEKGP

By adding six histidine (H) residues to a C-terminus of the 17S1NNT sequence, the recombinant small-molecule collagen expressed from this sequence was designated as 17S1N, including 88 amino acids with a theoretical molecular weight of 8306.13 Da. A sequence thereof is shown in SEQ ID NO: 6:

GPPGSGEKGERGAAGEPGPHGPPGVPGSVGPKGSSGSPGPQGPPGPVGLQGLRGE
VGLPG VKGDKGPMGPPGPKGDQGEKGPHHHHHH

The amino acid sequence from positions 659 to 717 of a full-length human collagen XVII sequence (refer to protein ID Q9UMD9-1 in the Uniprot database, https://www.uniprot.org/uniprot/Q9UMD9) was selected. The recombinant small-molecule collagen expressed from this sequence fragment was designated as 17S3NT, including 59 amino acids with a theoretical molecular weight of 5406.08 Da. A sequence thereof was shown in SEQ ID NO: 7:

GVPGSVGPKGSSGSPGPQGPPGPVGLQGLRGEVGLPGVKGDKGPMGPPGPKGDQ
GEKGP

By adding six histidine (H) residues to a C-terminus of the 17S3NT sequence, the recombinant small-molecule collagen expressed from this sequence was designated as 17S3, including 65 amino acids with a theoretical molecular weight of 6228.93 Da. A sequence thereof is shown in SEQ ID NO: 8:

GVPGSVGPKGSSGSPGPQGPPGPVGLQGLRGEVGLPGVKGDKGPMGPPGPKGDQ
GEKGP HHHHHH

### (2) Construction of a recombinant expression vector and an engineered strain for the single-sequence recombinant expression strategy of the small-molecule collagen

A DNA sequence for encoding 3A5D1NT was codon-optimized with Pichia pastoris as a host. An optimized sequence was shown in SEQ ID NO: 9:

gacgggaacccagggagtgatggcctgccaggacgcgatggctccccgggagggaagggggaccggggcgaaaatggttc
gcccggagcgccgggtgctcctggtcaccctggtccgcccgggcctgtcggaccagccggcaaatctggcgatcgaggagagagcg
gcccagctggtcccgctggggcaccaggtccggctgggtcaaggggtgcacctggccctcagggacccagaggagacaagggcga
gaccggtgaacgtggagccgcgggtattaaaggtcactaa

A DNA sequence for encoding 3A5D1 was codon-optimized with Pichia pastoris as a host. An optimized sequence was shown in SEQ ID NO: 10:

gacgggaacccagggagtgatggcctgccaggacgcgatggctccccgggagggaagggggaccggggcgaaaatggttc
gcccggagcgccgggtgctcctggtcaccctggtccgcccgggcctgtcggaccagccggcaaatctggcgatcgaggagagagcg
gcccagctggtcccgctggggcaccaggtccggctgggtcaaggggtgcacctggccctcagggacccagaggagacaagggcga
gaccggtgaacgtggagccgcgggtattaaaggtcaccatcatcatcaccactaa

A DNA sequence for encoding 3A5D2NT was codon-optimized with Pichia pastoris as a host. An optimized sequence was shown in SEQ ID NO: 11:

gaggcagggaagtccggtgacaggggggagagcggacctgctggtcctgctggagccccgggtcctgctggctctcgcggcg
cgccag gaccgcaaggcccacgtggtgataaaggagaaacaggggaaagaggggccgcaggtattaaaggtcactaa

A DNA sequence for encoding 3A5D2 was codon-optimized with Pichia pastoris as a host. An optimized sequence was shown in SEQ ID NO: 12:

gaggcagggaagtccggtgacaggggggagagcggacctgctggtcctgctggagccccgggtcctgctggctctcgcggcg
cgccag
gaccgcaaggcccacgtggtgataaaggagaaacaggggaaagaggggccgcaggtattaaaggtcaccatcaccatcatcactaa

A DNA sequence for encoding 17S1NNT was codon-optimized with Pichia pastoris as a host. An optimized sequence was shown in SEQ ID NO: 13:

gaggccggccctccaggttcgggcgagaaaggggaaagaggggcggcaggagaacccggaccccacggtcctcctggagt
cccgggttccgtaggcccaaaaggcagctctggaagtccggggccacaagggcctccaggccccgttggtttacagggtctacgcggc
gaagtgggattgccaggggtaaagggcgacaagggtccgatgggacctcccggtcctaagggtgatcaaggggagaaaggaccgta

a

A DNA sequence for encoding 17S1N was codon-optimized with Pichia pastoris as a host. An optimized sequence was shown in SEQ ID NO: 14:

gaggccggccctccaggttcgggcgagaaaggggaaagaggggcggcaggagaacccggaccccacggtcctcctggagt
cccgggttccgtaggcccaaaaggcagctctggaagtccggggccacaagggcctccaggccccgttggtttacagggtctacgcggc
gaagtgggattgccaggggtaaagggcgacaagggtccgatgggacctcccggtcctaagggtgatcaaggggagaaaggaccgca
tcaccaccatcatcattaa

A DNA sequence for encoding 17S3NT was codon-optimized with Pichia pastoris as a host. An optimized sequence was shown in SEQ ID NO: 15:

gaagccggtgtccctggctctgttggaccgaagggtagttcgggaagcccagggcctcaggggccgcccggacccgtaggtct
gcaggggctcagaggagaggtgggcctacctggcgtcaagggcgacaaaggtccaatggggccgcctggaccaaaaggggatcaa
ggtgaaaaaggcccctaa

A DNA sequence for encoding 17S3 was codon-optimized with Pichia pastoris as a host. An optimized sequence was shown in SEQ ID NO: 16:

gaagccggtgtccctggctctgttggaccgaagggtagttcgggaagcccagggcctcaggggccgcccggacccgtaggtct
gcaggggctcagaggagaggtgggcctacctggcgtcaagggcgacaaaggtccaatggggccgcctggaccaaaaggggatcaa
ggtgaaaaaggcccccaccatcatcaccatcactaa

DNA sequences SEQ ID NO: 9-SEQ ID NO: 16 were synthesized by Nanjing GenScript Biotechnology Co., Ltd. The synthesized gene was cloned into the pPIC9K expression vector (Thermo Fisher Scientific (China) Co., Ltd.) between the Kex2 enzyme cleavage site sequence (terminus DNA sequence of Kex2 enzyme cleavage site: AAAGAAGAAGGGGTATCTCTCGAGAAAAGA) in the α-factor secretion signal sequence (a secrete signal peptide) and an NotI enzyme cleavage site. Recombinant expression vectors pPIC9K-3A5D1NT, pPIC9K-3A5D1, pPIC9K-3A5D2NT, pPIC9K-3A5D2, pPIC9K-17S1NNT, pPIC9K-17SIN, pPIC9K-17S3NT, and pPIC9K-17S3 were obtained. Theoretically, other expression vectors suitable for Pichia pastoris, such as pPICZαB and pFLDα, exhibited similar effects to pPIC9K. The corresponding recombinant expression vectors were all in the scope of protection of the present invention.

The above recombinant expression vector plasmids (pPIC9K-3A5D1NT, pPIC9K-3A5D1, pPIC9K-3A5D2NT, pPIC9K-3A5D2, pPIC9K-17S1NNT, pPIC9K-17S1N, pPIC9K-17S3NT, pPIC9K-17S3), 10 µg each, were individually digested overnight at 37°C with SacI (purchased from TaKara (Dalian); specific operations were performed according to kit instructions), and linearized. The linearized plasmids were then purified using a PCR Purification Kit (purchased from Sangon Biotech (Shanghai) Co., Ltd.), and the final volume was adjusted to approximately 10 µL.

The linearized plasmid was electrotransformed into Pichia pastoris GS115 (purchased from China Center of Industrial Culture Collection) of an empty host strain in a competent cell. An electrotransformed strain culture was applied onto a MD plate. One MD plate was applied at every 100-200 µL, kept at a room temperature for 10 minutes, then inverted and incubated at 30°C for 2-5 days until a single colony (a positive transformant) appeared.

2 mL sterile double-distilled water was added to the surface of a MD plate. A His⁺ transformant was gently scraped from the surface of the plate using a sterile triangular spreader and transferred into a 50 mL centrifuge tube. A strain suspension was diluted with sterile double-distilled water. 105 cells were applied onto a YPD plate containing 0.5 mg/mL G418, placed upside down and incubated at 30°C for 3-4 days until a single colony appears. The colony was picked from the YPD plate into a sterile 96-well plate (200 µL YPD/well), mixed thoroughly, and incubated at 30°C for 48 hours. A strain culture was mixed evenly in each well. 10 µL of the strain suspension was put onto one new sterile 96-well plate, and incubated at 30°C for 24 hours, and then this procedure was repeated once more. After 24 hours, 1 µL of the strain suspension was taken out from a third 96-well plate to be dripped onto the YPD plates containing 1.0 mg/mL and 4 mg/mL G418, respectively, and continue incubating at 30°C for 96-120 hours. If Pichia pastoris transformants grew on plates containing a high concentration of G418, it indicates that the transformant can efficiently express an exogenous gene. A further screening step yields a highly efficient recombinant yeast engineered strain.

Samples of 8 engineered strains constructed were deposited at China General Microbiological Culture Collection Center. The corresponding strain deposited numbers are:
A strain expressing the recombinant small-molecule collagen 3A5D1NT has a deposit number: CGMCC No. 25823.

A strain expressing the recombinant small-molecule collagen 3A5D1 has a deposit number: CGMCC No.25811

A strain expressing the recombinant small-molecule collagen 3A5D2NT has a deposit number: CGMCC No.25824

A strain expressing the recombinant small-molecule collagen 3A5D2 has a deposit number: CGMCC No.25812

A strain expressing the recombinant small-molecule collagen 17S1NNT has a deposit number: CGMCC No.25825

A strain expressing the recombinant small-molecule collagen 17S1N has a deposit number: CGMCC No.25813

A strain expressing the recombinant small-molecule collagen 17S3NT has a deposit number: CGMCC No.25826

A strain expressing the recombinant small-molecule collagen 17S3 has a deposit number: CGMCC. 25814;

The deposited address for all 8 engineered strains was as follows: No. 3, Compound 1, Beichen West Road, Chaoyang District, Beijing. The deposited date for all was: September 26, 2022. Classification designation for all strains was *Komagataella phaffii.*

### (3) Expression, purification, and identification of a recombinant small-molecule collagen via a single-sequence recombinant expression strategy

With 8 highly expressive recombinant yeast engineered strains obtained in step (2), high-density fermentation was conducted by employing a fed-batch method to produce a recombinant small-molecule collagen. A fermentation broth containing the recombinant collagen was harvested, and a high-purity recombinant small-molecule collagen freeze-dried sponge was purified. Specific procedures were as follows:
8 highly expressive recombinant yeast strains were selected in step (2) and inoculated into a 1L shake flask containing a seed culture solution YPG, incubated at 220 rpm and 30°C for 18-20 hours until OD₆₀₀ reached 2-10. 200 mL of a seed culture was inoculated into a 5 L fermentation tank (purchased from Shanghai Baoxing Bio-Engineering Equipment Co., Ltd.). A 2 L fermentation medium was filled, and induced growth using a mixed methanol-glycerol carbon source (methanol:50% glycerol = 7:3). The fermentation medium was induced for 40-60 hours. When UV measurements showed no significant increase or a decrease in protein concentration, then the tank can be put. The formula for protein concentration measured by UV: C(mg/mL) = 0.144 × (A215 - A225).

The fermentation broth was collected and centrifuged at 2000 g at 4°C for 30 minutes to separate a strain from a fermentation supernatant. The fermentation supernatant was taken to carry out cation exchange chromatography purification (a chromatography packing material was UniGel-80sp resin from Suzhou NanoMicro Technology Co., Ltd., loaded onto a GCC-50-400 column from Lisure Technology, and processed with a GE AKTA Pure protein separation chromatography purification system), followed by ultrafiltration (using Shandong Bona Biotechnology Group Co., Ltd.'s 1KDa organic membrane filtration system with a model BNUF402-2-A), desalting, and concentration. The product was then lyophilized to obtain a recombinant small-molecule collagen freeze-dried sponge.

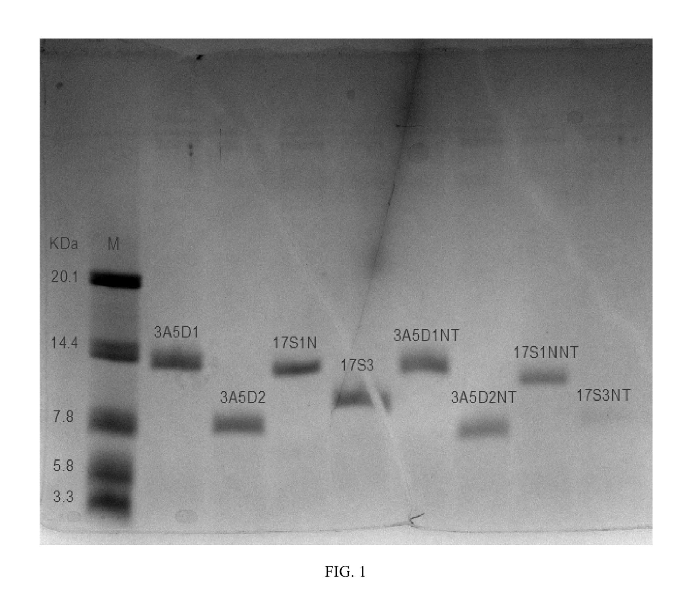

The purified lyophilized sponge was dissolved in ultrapure water, mixed with a 2× small-molecule protein loading buffer, and heated in a 100°C water bath for 10 minutes. Tricine-PAGE inspection was performed (all reagents required by Tricine-PAGE electrophoresis suitable for a small-molecule protein were purchased from Shanghai Wansheng Haotian Biotechnology Co., Ltd., with electrophoresis conducted according to manufacturer's instructions). The detection results were shown in FIG. 1. As depicted, 8 recombinant small-molecule collagens were effectively secreted and expressed in the supernatant, exhibiting a distinct electrophoretic band. There was no issue with uncontrolled sizes of a hydrolyzed small-molecule collagen (The collagen exhibited a certain degree of electrophoretic migration delay during electrophoresis, resulting in an apparent molecular weight that appears larger than expected).

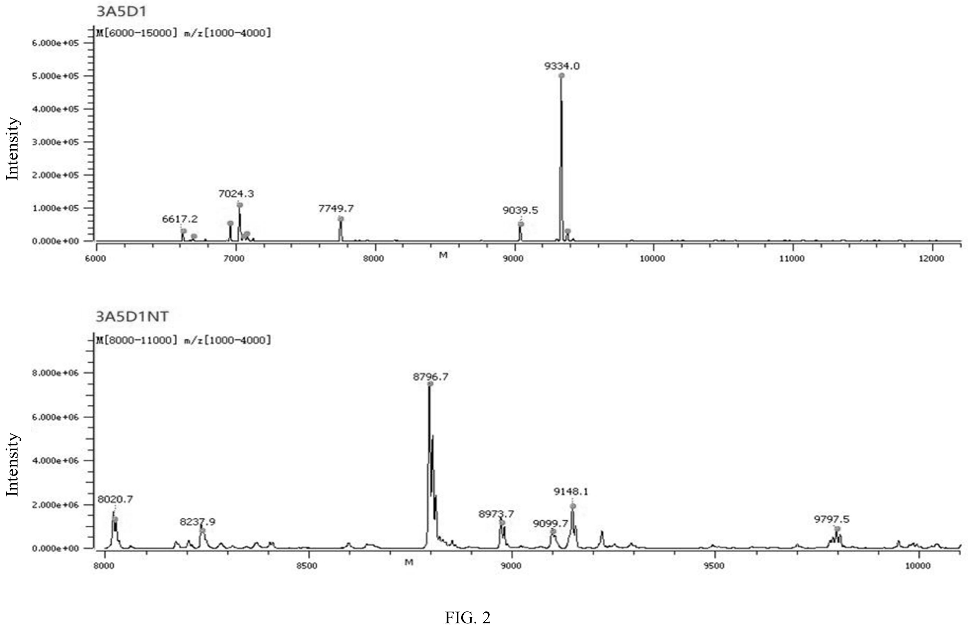

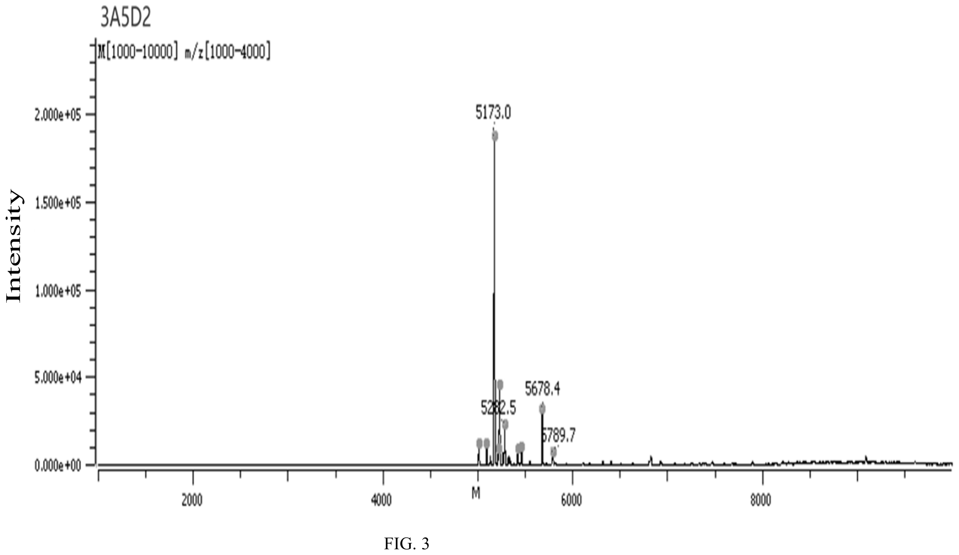

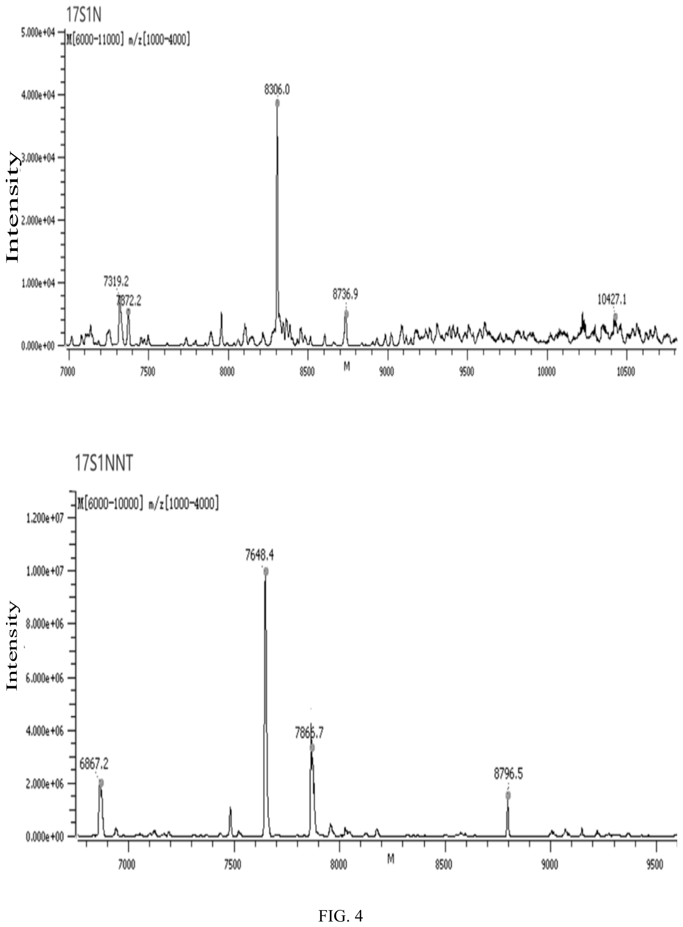

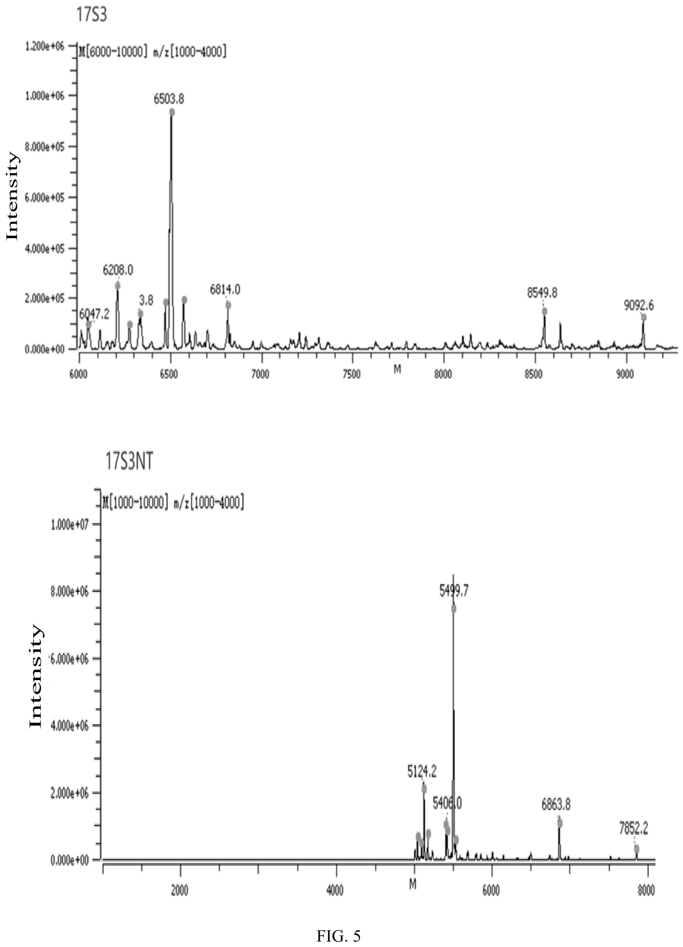

Eight high-purity recombinant small-molecule collagen lyophilized products were subjected to LC-MS analysis to obtain deconvoluted molecular weights (contracted to Beijing Biotech Pack Scientific Co, Ltd.). The results were shown in FIGS. 2-5. In some of the result figures, the deconvoluted molecular weight values were displayed with one decimal place retained, and subject to rounding. As shown in the figures: 3A5D1NT had a theoretical molecular weight of 8596.08 Da and a measured deconvoluted molecular weight of 8796.68 Da; 3A5D1 had a theoretical molecular weight of 9281.78 Da and a measured deconvoluted molecular weight of 9333.96 Da; 3A5D2NT had a theoretical molecular weight of 4532.83 Da and a measured deconvoluted molecular weight of 4591.46 Da; 3A5D2 had a theoretical molecular weight of 5218.54 Da and a measured deconvoluted molecular weight of 5173.04 Da; 17S1NNT had a theoretical molecular weight of 7483.28 Da and a measured deconvoluted molecular weight of 7648.41 Da; 17S1N had a theoretical molecular weight of 8306.13 Da and a measured deconvoluted molecular weight of 8306.00 Da; 17S3NT had a theoretical molecular weight of 5406.08 Da and a measured deconvoluted molecular weight of 5499.72 Da; 17S3 had a theoretical molecular weight of 6228.93 Da and a measured deconvoluted molecular weight of 6503.79 Da. An observed deviation arose from potential glycosylation modification during protein expression, and a detection error. Therefore, the measured deconvoluted molecular weights of the recombinant small-molecule collagens 3A5D1NT, 3A5D1, 3A5D2NT, 3A5D2, 17S1NNT, 17S1N, 17S3NT, and 17S3 were essentially consistent with the theoretical values thereof.

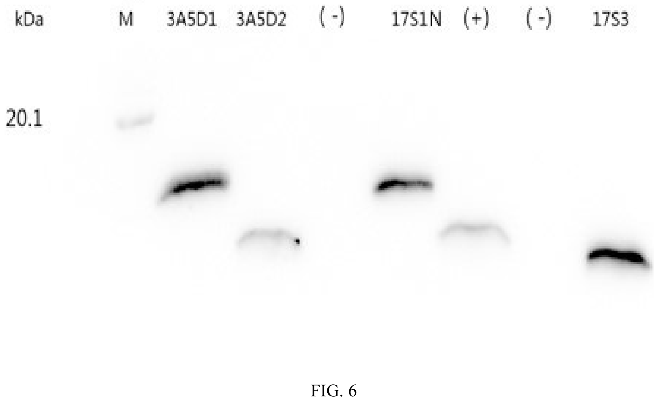

Since 3A5D1, 3A5D2, 17S1N, and 17S3 bore a 6×His tag at carboxyl-termini, Western blot detection was performed using an anti-6×His Tag antibody (purchased from Nanjing GenScript Biotechnology Co., Ltd.) with color development by ECL chemiluminescence and inspection by an automated chemiluminescence imaging system (Tanon 5200). The results were shown in FIG. 6. 6×His tags were successfully detected in 3A5D1, 3A5D2, 17S1N, and 17S3 (negative control (-) was a culture supernatant of a GS115 empty vector strain. Positive control (+) was His-tagged recombinant human MIF purchased from Sangon Biotech (Shanghai) Co., Ltd., both were normal), and the corresponding band positions matched expected sizes.

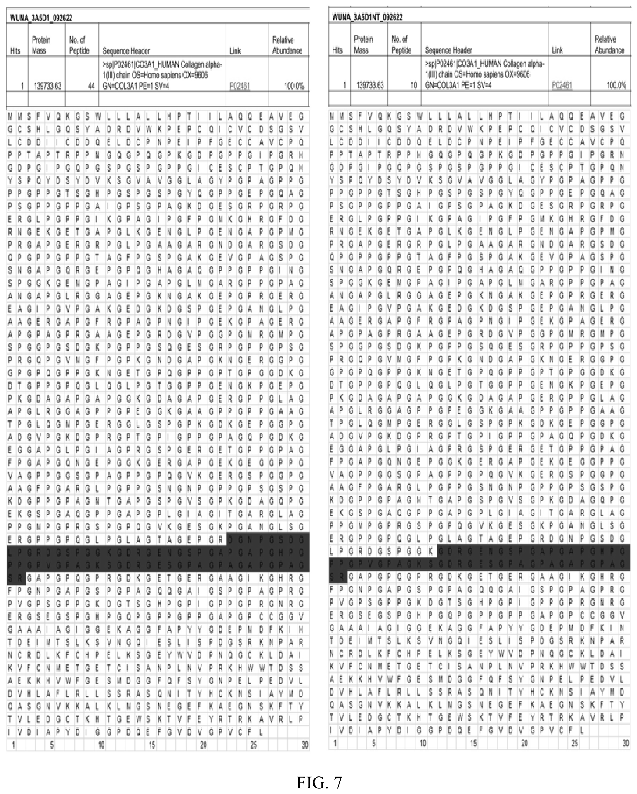

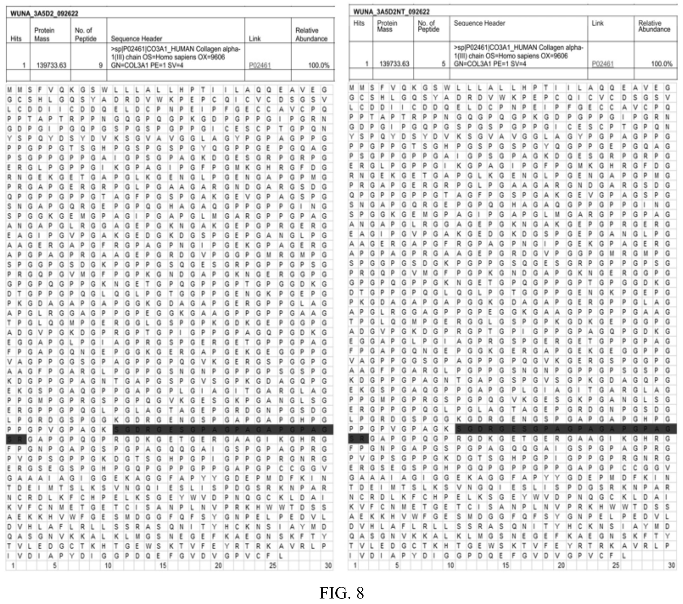

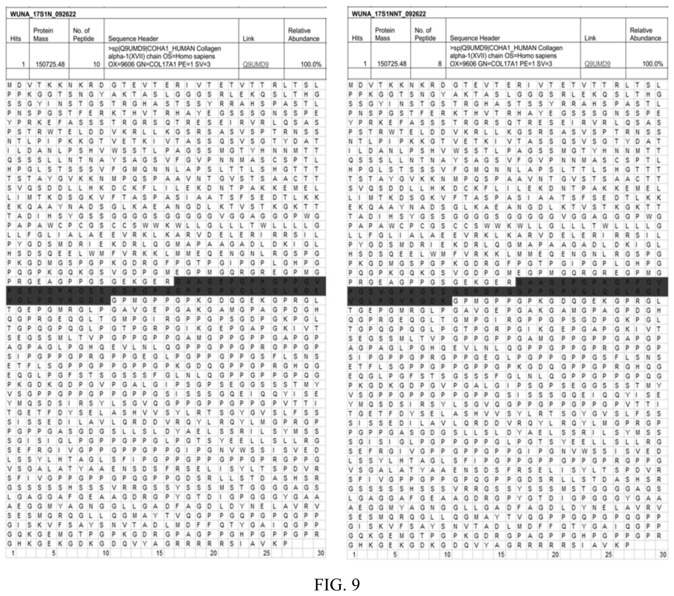

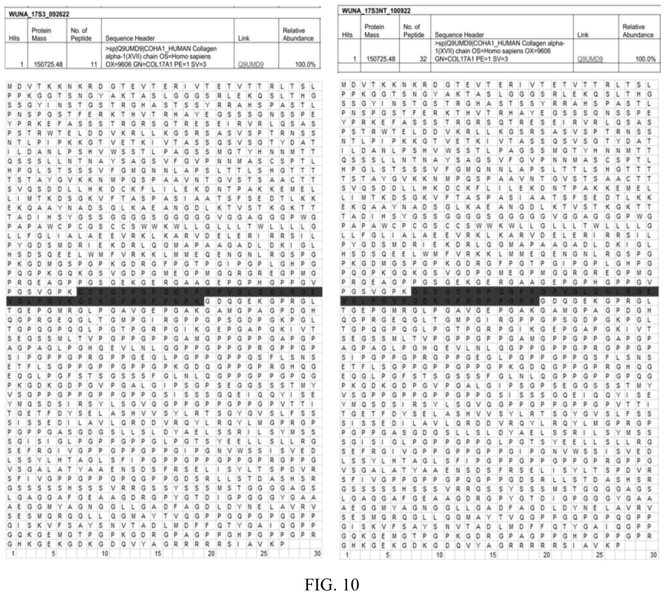

A lyophilized product was enzymatically digested with trypsin. Nano-HPLC-MS/MS mass spectrometry was used to detect an enzymatically digested trypsin peptide fragment of the recombinant collagen (contracted to Suzhou ProtTech Biotechnology Co., Ltd.). The detected peptide fragment was then compared with sequences of various natural proteins in Uniprot database. Results were shown in FIGS 7-10. Mass spectrometry inspection results indicated that peptide fragments obtained by the tryptic digestion of recombinant small-molecule collagens 3A5D1, 3A5D2, 17S1N, 17S3, 3A5D1NT, 3A5D2NT, 17S1NNT, and 17S3NT and sequences covered by the peptide fragments all belonged to relevant regions of human collagen sequences, confirming successful expression of the recombinant small-molecule collagens.

### Embodiment 2: Construction of a highly expressive system, a highly expressive chassis cell or a chassis engineered strain

### (1) Design of a CPB positioning functional fusion protein

The present invention used Pichia pastoris as a starting cell and was combined with the design of a specialized expression sequence. The present invention first constructed a dedicated chassis cell or a dedicated chassis engineered strain. The chassis cell or the chassis engineered strain included a positioning fusion functional protein. The positioning fusion functional protein was configured to sequentially cleave and remove basic amino acid residues (K andR) at the C-termini of the recombinant small-molecule collagens after the plurality of recombinant small-molecule collagens for tandem repeat were cleaved by a Kex2 enzyme into monomers.

The positioning fusion functional protein included a mature peptide sequence of the CPB enzyme, a functional region sequence with intracellular membrane positioning or conversion and transport functions among respective organelles and a connecting sequence for connecting the CPB enzyme to the functional region sequence with the intracellular membrane positioning or the conversion and transport functions among the respective organelles during fusion expression;

The CPB enzyme may be derived from any species and preferably derived from a human or a rat in the present invention.

The functional region sequence with the intracellular membrane positioning or the conversion and transport functions among the respective organelles was preferably derived from Saccharomyces cerevisiae or Pichia pastoris. The functional region was preferably derived from the Kex2 enzyme or other functional regions of a protein with similar functions, such as a Ste13 protease.

The connecting sequence, that is, a linker sequence, was not limited, as long as the sequence could play a connecting role that did not interfere with the functional region with the intracellular membrane positioning or the conversion and transport functions among the respective organelles.

In this embodiment, the CPB enzyme preferred amino acid sequences at positions 111-417 (i.e., a mature peptide sequence of a human CPB enzyme) in a full-length (including a signal peptide, a propeptide, and a mature peptide) human CPB enzyme sequence (Uniprot database ID was P15086, ) as a preferable human CPB enzyme amino acid sequence in the present invention, as shown in SEQ ID NO: 17:

ATGHSYEKYNKWETIEAWTQQVATENPALISRSVIGTTFEGRAIYLLKVGKAGQN
KPAIFMDCGFHAREWISPAFCQWFVREAVRTYGREIQVTELLDKLDFYVLPVLNIDGYI
YTWTKSRFWRKTRSTHTGSSCIGTDPNRNFDAGWCEIGASRNPCDETYCGPAAESEKE
TKALADFIRNKLSSIKAYLTIHSYSQMMIYPYSYAYKLGENNAELNALAKATVKELASL
HGTKYTYGPGATTIYPAAGGSDDWAYDQGIRYSFTFELRDTGRYGFLLPESQIRATCEE
TFLAIKYVASYVLEHLY

In this embodiment, the CPB enzyme further preferred amino acid sequences at positions 109-415 (i.e., a mature peptide sequence of a rat CPB enzyme) in a full-length (including the signal peptide, the propeptide, and the mature peptide) rat CPB enzyme sequence (Uniprot database ID was P19223, https://www.uniprot.org/uniprotkb/P19223) as, a preferable rat CPB enzyme amino acid sequence in the present invention, as shown in SEQ ID NO: 18:

ASGHSYTKYNKWETIEAWIQQVATDNPDLVTQSVIGTTFEGRNMYVLKIGKTRPN
KPAIFIDCGFHAREWISPAFCQWFVREAVRTYNQEIHMKQLLDELDFYVLPVVNIDGYV
YTWTKDRMWRKTRSTMAGSSCLGVRPNRNFNAGWCEVGASRSPCSETYCGPAPESE
KETKALADFIRNNLSTIKAYLTIHSYSQMMLYPYSYDYKLPENYEELNALVKGAAKEL
ATLHGTKYTYGPGATTIYPAAGGSDDWSYDQGIKYSFTFELRDTGFFGFLLPESQIRQT

CEETMLAVKYIANYVREHLY

In this embodiment, the functional region with the intracellular membrane positioning or the conversion and transport functions among the respective organelles further preferred amino acid sequences at positions 679-814 in a full-length amino acid sequence (Uniprot database ID was P13134 protein, https://www.uniprot.org/uniprotkb/ P13134) of a Kex2 enzyme of *Saccharomyces cerevisiae ,* that is, the region with the intracellular membrane positioning or the conversion and transport functions among the respective organelles positioned at a carboxyl-terminus, with the sequence as shown in SEQ ID NO: 19:

YFLTIFLIGATFLVLYFMFFMKSRRRIRRSRAETYEFDIIDTDSEYDSTLDNGTSGITE
PEEVEDFDFDLSDEDHLASLSSSENGDAEHTIDSVLTNENPFSDPIKQKFPNDANAESAS
NKLQELQPDVPPSSGRS

In this embodiment, the functional region with the intracellular membrane positioning or the conversion and transport functions among the respective organelles further preferred amino acid sequences at positions 681-777 in a full-length amino acid sequence of a Kex2 enzyme of *Komagataella phaffii* (i.e., *Pichia pastoris*) (Uniprot database ID was C4R095 protein, https://www.uniprot.org/uniprotkb/C4R095), that is, the region with the intracellular membrane positioning or the conversion and transport functions among the respective organelles positioned at the carboxyl-terminus, as shown in SEQ ID NO: 20:

YLAFLLGLGFLICIIFLFTNRNKLERRQRRNRRDEYEFDLIPADDDFDTEEDQEANS
QFTLD SDAELMFEDTSQREASPHEYQDSLGSNEHPKRAAL

As connection during fusion expression between the carboxy-terminus sequenced of the CPB enzyme and the Kex2 enzyme, the linker sequence was used, as shown in SEQ ID NO: 21:

### GGSGSGSGGS

A CPB positioning fusion functional protein constructed in the present invention was as follows:
The CPB positioning fusion functional protein constructed in the present invention, which fused an expressive human CPB enzyme with a carboxy-terminus sequence of the Kex2 enzyme of Pichia pastoris, was named HCPB-PPKEX2. A sequence thereof was shown in SEQ ID NO: 22:

ATGHSYEKYNKWETIEAWTQQVATENPALISRSVIGTTFEGRAIYLLKVGKAGQN
KPAIFMDCGFHAREWISPAFCQWFVREAVRTYGREIQVTELLDKLDFYVLPVLNIDGYI
YTWTKSRFWRKTRSTHTGSSCIGTDPNRNFDAGWCEIGASRNPCDETYCGPAAESEKE
TKALADFIRNKLSSIKAYLTIHSYSQMMIYPYSYAYKLGENNAELNALAKATVKELASL
HGTKYTYGPGATTIYPAAGGSDDWAYDQGIRYSFTFELRDTGRYGFLLPESQIRATCEE
TFLAIKYVASYVLEHLYGGSGSGSGGSYLAFLLGLGFLICIIFLFTNRNKLERRQRRNRR
DEYEFDLIPADDDFDTEEDQEANSQFTLDSDAELMFEDTSQREASPHEYQDSLGSNEHP
KRAAL

A DNA sequence for encoding SEQ ID NO: 22 was shown in SEQ ID NO: 23:

gcgacaggacacagctatgagaaatacaacaaatgggagacaatagaggcgtggacgcagcaggtggccaccgaaaacccg
gctcttatatctcgttctgtcatcggaaccactttcgaaggccgagccatctacctacttaaggtcggcaaagcaggtcaaaacaaaccagc
aatctttatggactgtggattccatgctagggagtggatctcaccagcattctgtcagtggttcgtacgggaggcggttcgaacgtatggtag
agaaattcaggtcacagagctgttggacaagctcgatttttatgtactacctgtgctgaacattgatgggtatatttatacctggactaagtccc
gcttttggcgcaagacgcggagtacgcacactgggtcctcttgcatcggaacagaccccaaccgtaatttcgatgcaggctggtgcgaga
taggggctagtcgtaatccgtgcgacgagacgtactgcgggcccgcggctgaatcagaaaaggaaactaaggcgcttgcggatttcatt
agaaataaactttccagtatcaaagcgtatttgaccatacactcgtatagccaaatgatgatctacccctactcctacgcgtataaacttggtg
aaaacaacgccgaactgaatgccctcgccaaggcaactgtgaaggagctagcaagcttgcatggaactaaatacacttacgggcctggt
gccacaaccatatacccggctgccgggggtagtgacgattgggcttatgatcagggtataaggtatagctttacgttcgagctgcgtgata
cgggccgttatggattcttgctccctgagtctcaaattagggctacctgtgaagaaacatttctggctattaaatatgttgcatcttacgtattag
aacatttatacggaggctcgggatctgggtcgggtggctcatacctagccttcttactgggcctcggttttttaatatgtattatcttcctctttac
aaaccggaataagttggaacgccggcaaaggcgaaatcgccgagatgaatatgaatttgaccttattcccgcagatgacgactttgatact
gaggaagatcaagaagcgaattcacaatttaccttagactcagacgccgagctaatgtttgaagatacctcgcaacgagaggcgtcccca
catgagtaccaggactcgttgggcagcaatgagcacccaaaaagagctgcattataa

The CPB positioning fusion functional protein, which fused the expressive human CPB enzyme with a carboxy-terminus sequence of the Kex2 enzyme of saccharomyces cerevisiae, was named HCPB-SCKEX2. A sequence thereof was shown in SEQ ID NO: 24:

ATGHSYEKYNKWETIEAWTQQVATENPALISRSVIGTTFEGRAIYLLKVGKAGQN

KPAIFMDCGFHAREWISPAFCQWFVREAVRTYGREIQVTELLDKLDFYVLPVLNIDGYI
YTWTKSRFWRKTRSTHTGSSCIGTDPNRNFDAGWCEIGASRNPCDETYCGPAAESEKE
TKALADFIRNKLSSIKAYLTIHSYSQMMIYPYSYAYKLGENNAELNALAKATVKELASL
HGTKYTYGPGATTIYPAAGGSDDWAYDQGIRYSFTFELRDTGRYGFLLPESQIRATCEE
TFLAIKYVASYVLEHLYGGSGSGSGGSYFLTIFLIGATFLVLYFMFFMKSRRRIRRSRAE
TYEFDIIDTDSEYDSTLDNGTSGITEPEEVEDFDFDLSDEDHLASLSSSENGDAEHTIDSV
LTNENPFSDPIKQKFPNDANAESASNKLQELQPDVPPSSGRS

A DNA sequence for encoding SEQ ID NO: 24 was shown in SEQ ID NO: 25:

gcgacaggacacagctatgagaaatacaacaaatgggagacaatagaggcgtggacgcagcaggtggccaccgaaaacccg
gctcttatatctcgttctgtcatcggaaccactttcgaaggccgagccatctacctacttaaggtcggcaaagcaggtcaaaacaaaccagc
aatctttatggactgtggattccatgctagggagtggatctcaccagcattctgtcagtggttcgtacgggaggcggttcgaacgtatggtag
agaaattcaggtcacagagctgttggacaagctcgatttttatgtactacctgtgctgaacattgatgggtatatttatacctggactaagtccc
gcttttggcgcaagacgcggagtacgcacactgggtcctcttgcatcggaacagaccccaaccgtaatttcgatgcaggctggtgcgaga
taggggctagtcgtaatccgtgcgacgagacgtactgcgggcccgcggctgaatcagaaaaggaaactaaggcgcttgcggatttcatt
agaaataaactttccagtatcaaagcgtatttgaccatacactcgtatagccaaatgatgatctacccctactcctacgcgtataaacttggtg
aaaacaacgccgaactgaatgccctcgccaaggcaactgtgaaggagctagcaagcttgcatggaactaaatacacttacgggcctggt
gccacaaccatatacccggctgccgggggtagtgacgattgggcttatgatcagggtataaggtatagctttacgttcgagctgcgtgata
cgggccgttatggattcttgctccctgagtctcaaattagggctacctgtgaagaaacatttctggctattaaatatgttgcatcttacgtattag
aacatttatacggaggctcgggatctgggtcgggtggctcatatttcctcacaatattcttaataggcgcaacgtttttagtgctgtattttatgtt
tttcatgaaatcaaggcggagaatccggcgttcacgcgcggagacctacgagttcgacatcatagatactgactcggaatacgattcaacc
ctggataacgggacctcgggtattactgaacccgaggaggtagaggatttcgatttcgacctctctgacgaagatcatctagccagtctatc
gtccagtgaaaatggagatgctgaacacacaatcgacagcgtccttacgaatgagaacccgttttccgatcctattaaacaaaagtttccca
acgacgcgaatgcagagagcgccagtaacaagttgcaggaattgcagccagacgttccgccatcctctggtcgatcttaa

The CPB positioning fusion functional protein, which fused a rat CPB enzyme with a carboxy-terminus sequence of the Kex2 enzyme of Pichia pastoris, was named RCPB-PPKEX2. A sequence thereof was shown in SEQ ID NO: 26:

ASGHSYTKYNKWETIEAWIQQVATDNPDLVTQSVIGTTFEGRNMYVLKIGKTRPN
KPAIFIDCGFHAREWISPAFCQWFVREAVRTYNQEIHMKQLLDELDFYVLPVVNIDGYV

YTWTKDRMWRKTRSTMAGSSCLGVRPNRNFNAGWCEVGASRSPCSETYCGPAPESE
KETKALADFIRNNLSTIKAYLTIHSYSQMMLYPYSYDYKLPENYEELNALVKGAAKEL
ATLHGTKYTYGPGATTIYPAAGGSDDWSYDQGIKYSFTFELRDTGFFGFLLPESQIRQT
CEETMLAVKYIANYVREHLYGGSGSGSGGSYLAFLLGLGFLICIIFLFTNRNKLERRQRR
NRRDEYEFDLIPADDDFDTEEDQEANSQFTLDSDAELMFEDTSQREASPHEYQDSLGSN
EHPKRAAL

A DNA sequence for encoding SEQ ID NO: 26 was shown in SEQ ID NO: 27:

gcaagcggtcactcgtacacaaagtataacaaatgggaaactatcgaggcctggattcaacaagtagccacggataaccctgattt
agtcacgcaatcggtcatagggacaaccttcgagggcagaaacatgtacgtattaaagataggaaagactcgtcccaataaacctgctatt
tttatcgactgtggcttccacgctagagaatggattagccctgccttctgccaatggttcgtgagggaagcagtacggacgtacaaccagg
aaattcatatgaagcaactgctcgacgaattagatttctatgttttaccggtagttaacatcgatggctacgtttacacatggacaaaagaccg
tatgtggcggaagactagatcgacgatggcaggctcatcctgtctgggtgtgcgtccgaataggaacttcaatgcgggttggtgtgaagtg
ggggcgtcccgctctccctgttcagagacatactgcggacctgccccagaatccgaaaaagagacgaaagctcttgctgattttattagga
acaacctaagtaccattaaagcatacttaaccatccatagttacagccagatgatgctgtatccctattcctacgactataagctaccggaga
actatgaagaactcaatgctttagttaaaggagcggccaaggagttggcgacattgcatggaacgaagtacacttatggtccaggggcca
ctaccatctatccggcagctggtggatcagacgactggagttatgaccaaggtataaagtactctttcacctttgaattacgtgatactggtttt
tttggctttctgcttccagagtcacagatccgccagacttgcgaggaaaccatgcttgcggtgaagtatatagctaattacgtccgtgaacat
ctatacggcggatccgggtcggggagtggtgggagttatctagcatttttgctagggctcggcttcctgatctgcataatattcttgtttacca
accgcaataaattggaacgaaggcagcgacgaaatcggcgagacgaatatgagtttgacctcattccggcggatgatgattttgatacgg
aggaggaccaggaggccaatagccaattcacattggactctgacgcggaacttatgttcgaagatacgtcacagagagaggcctcgccc
cacgagtatcaagattctctgggatctaatgaacacccaaaacgggctgcactttaa

The CPB positioning fusion functional protein, which fused the expressive rat CPB enzyme with the carboxy-terminus sequence of the Kex2 enzyme of saccharomyces cerevisiae, was named RCPB-SCKEX2. A sequence thereof was shown in SEQ ID NO: 28:

ASGHSYTKYNKWETIEAWIQQVATDNPDLVTQSVIGTTFEGRNMYVLKIGKTRPN
KPAIFIDCGFHAREWISPAFCQWFVREAVRTYNQEIHMKQLLDELDFYVLPVVNIDGYV
YTWTKDRMWRKTRSTMAGSSCLGVRPNRNFNAGWCEVGASRSPCSETYCGPAPESE
KETKALADFIRNNLSTIKAYLTIHSYSQMMLYPYSYDYKLPENYEELNALVKGAAKEL

ATLHGTKYTYGPGATTIYPAAGGSDDWSYDQGIKYSFTFELRDTGFFGFLLPESQIRQT
CEETMLAVKYIANYVREHLYGGSGSGSGGSYFLTIFLIGATFLVLYFMFFMKSRRRIRR
SRAETYEFDIIDTDSEYDSTLDNGTSGITEPEEVEDFDFDLSDEDHLASLSSSENGDAEHT
IDSVLTNENPFSDPIKQKFPNDANAESASNKLQELQPDVPPSSGRS

A DNA sequence for encoding SEQ ID NO: 28 was shown in SEQ ID NO: 29:

gcaagcggtcactcgtacacaaagtataacaaatgggaaactatcgaggcctggattcaacaagtagccacggataaccctgattt
agtcacgcaatcggtcatagggacaaccttcgagggcagaaacatgtacgtattaaagataggaaagactcgtcccaataaacctgctatt
tttatcgactgtggcttccacgctagagaatggattagccctgccttctgccaatggttcgtgagggaagcagtacggacgtacaaccagg
aaattcatatgaagcaactgctcgacgaattagatttctatgttttaccggtagttaacatcgatggctacgtttacacatggacaaaagaccg
tatgtggcggaagactagatcgacgatggcaggctcatcctgtctgggtgtgcgtccgaataggaacttcaatgcgggttggtgtgaagtg
ggggcgtcccgctctccctgttcagagacatactgcggacctgccccagaatccgaaaaagagacgaaagctcttgctgattttattagga
acaacctaagtaccattaaagcatacttaaccatccatagttacagccagatgatgctgtatccctattcctacgactataagctaccggaga
actatgaagaactcaatgctttagttaaaggagcggccaaggagttggcgacattgcatggaacgaagtacacttatggtccaggggcca
ctaccatctatccggcagctggtggatcagacgactggagttatgaccaaggtataaagtactctttcacctttgaattacgtgatactggtttt
tttggctttctgcttccagagtcacagatccgccagacttgcgaggaaaccatgcttgcggtgaagtatatagctaattacgtccgtgaacat
ctatacggcggatccgggtcggggagtggtgggagttatttcctcacaatattcttaataggcgcaacgtttttagtgctgtattttatgtttttc
atgaaatcaaggcggagaatccggcgttcacgcgcggagacctacgagttcgacatcatagatactgactcggaatacgattcaaccctg
gataacgggacctcgggtattactgaacccgaggaggtagaggatttcgatttcgacctctctgacgaagatcatctagccagtctatcgtc
cagtgaaaatggagatgctgaacacacaatcgacagcgtccttacgaatgagaacccgttttccgatcctattaaacaaaagtttcccaacg
acgcgaatgcagagagcgccagtaacaagttgcaggaattgcagccagacgttccgccatcctctggtcgatcttaa

In the design of the positioning fusion functional protein of the present invention, the preferred Kex2 enzyme was a calcium ion-dependent serine protease naturally expressed by a yeast strain (including Pichia species), which could specifically recognize and cleave a peptide bond at carboxyl-termini of dibasic amino acids such as RR and KR in the amino acid sequence, playing a key role in the protein secretion pathway of a yeast. Concurrently, the yeast strain had an STE13 gene, which could express the Ste13 protease (strictly speaking, a dipeptidyl aminopeptidase) intracellularly and cleave EA and EAEA amino acid sequences at amino-termini in proteins.

The most common application of the Kex2 enzyme in genetic engineering was that in the exogenous secretion pathway of the yeast, the Kex2 enzyme cleaved a signal peptide sequence or a propeptide sequence in an exogenous protein precursor to release a mature secreted protein. The Kex2 enzyme cleaved the peptide bond at a carboxyl-terminus of a dibasic amino acid such as KR or RR in an exogenous protein. To enhance cleavage efficiency at the same time, an amino acid sequence like EA or EAEA was often added after KR or RR (though not mandatory; the sequence could also be the sequence from the exogenous protein, but cleavage efficiency varied depending on the types of the 1-4 amino acid residues following KR or RR). Additionally, the Kex2 enzyme could function as an enzyme cleavage site between the fusion proteins, splitting one whole expression peptide fragment into two or more peptide fragments. This method was frequently used when expressing proteins with different subunits. Correspondingly, this application could be extended to proteins with a plurality of tandem repeat sequences. A Kex2 cleavage site was inserted herein, which could indirectly increase copy number, thereby enhancing the expression level of the exogenous protein. However, when the Kex2 enzyme acted, the dibasic amino acid like KR or RR was prerequisite for effective cleavage. However, after cleavage, KR or RR could not be removed. To enhance cleavage efficiency, 1-2 amino acid residues were often inserted before KR or RR (though not mandatory, the amino acid residues could be adjusted and designed based on actual conditions). For the recombinant expression protein, more additional non-natural amino acids were introduced. A collagen, whose typical sequence structure was a repetitive G-X-Y triplet sequence structure, often had this structural feature disrupted by the introduction of the non-natural amino acids.

In the design of the positioning fusion protein of the present invention, the removal of KR and RR was required to be considered.

A recombinant carboxypeptidase B (the CPB enzyme, an exocrine protease) could specifically cleave basic amino acids (particularly K and R) at the carboxyl terminus of the protein until all basic amino acids at the carboxyl termini were removed, leaving non-basic amino acids exposed at the C-terminus of the protein. In bioengineering or biopharmaceutical fields, the CPB enzyme was generally used by adding the CPB enzyme after the expression and purification of a recombinant protein drug or a protein drug to remove the basic amino acids like K and R at the carboxyl termini, which equivalently introduced an additional exogenous substance into the expression and purification workflow of the recombinant protein. Besides the increased cost of purchasing the CPB enzyme, process steps for CPB removal and residue detection were added. Residual enzyme assessment may be necessitated, which increased process complexity, workflow, costs, and time while introducing residual enzyme contamination risks.

Therefore, the present invention also required establishing a synthetic metabolic pathway for the CPB enzyme in a cell, particularly in the expression and secretion pathways of the protein so that the CPB enzyme was positioned in a cell (the expression and secretion pathways of the protein constituted by an endoplasmic reticulum lumen, trans-Golgi network (TGN), etc.). Following cleavage of a designed long-sequence recombinant collagen amino acid into small fragments and small-molecule proteins by the Kex2 enzyme, the CPB enzyme could then synergistically collaborate with a Ste 13 protease to excise non-collagenous amino acids like C-terminus KR and N-terminus EA, respectively, preserving the G-X-Y triplet repeat structure of the collagen.

In summary, the present invention required to address the challenge that the CPB enzyme was not only expressed in the cell but also directed into the secretion and expression pathways (via a mechanism identical to that of a full-length secreted protein) while being able to remain confined in the secretion pathway of the protein such as the endoplasmic reticulum lumen and the trans-Golgi network without being secreted extracellularly. Furthermore, the CPB enzyme maintained temporal and spatial continuity with the activation time of the Kex2 enzyme.

Therefore, the present invention fused the CPB enzyme with a sequence with the intracellular membrane positioning or the conversion and transport functions among the respective organelles in the cell, created one novel CPB-positioned fusion functional protein capable of secreting and positioning on the intracellular membrane, and accompanying the Kex2 enzyme and the recombinant collagen in the conversion and transport among intracellular secretory organelles-without being secreted (remaining positioned on the intracellular membrane and unable to detach).

The Kex2 enzyme included seven domains as a whole: a signal peptide, a precursor peptide, a catalytic domain, a P-domain, an Ser/Thr-rich domain, a transmembrane region, and an extracellular domain. The enzymatic activity thereof was primarily realized by the catalytic domain, the P-domain, and the Ser/Thr-rich domain. Retaining these three domains preserved the activity of the Kex2 enzyme. However, a C-terminus membrane domain and the extracellular domain primarily governed the conversion and transport of a Kex2 protease among the organelles in the cell. The present invention selected the C-terminus functional region of the Kex2 enzyme to be fused with the CPB enzyme for expression. To preserve the structural integrity and activity of both fused components, a flexible linker sequence (non-essential) was inserted between both fused components.

Furthermore, the identical C-terminus sequence could concentrate the Kex2 enzyme and the CPB-positioned fusion functional protein in the same region. The reaction product of the Kex2 enzyme was a substrate for the CPB-positioned fusion functional protein. This positioned concentration enhanced synergistic activity between the two enzymes.

### (2) Construction and identification of an expression vector and a chassis cell of the CPB-positioned fusion protein

A CPB chassis cell was required to express the CPB positioning fusion functional protein, position the CPB positioning fusion functional protein in the secretion pathway of the cellular protein constituted by the endoplasmic reticulum and the trans-Golgi network, and enable synergistic action with yeast-specific proteases such as Kex2 and Ste13. Therefore, constructing the expression vector of the CPB positioning fusion functional protein required adding a signal peptide sequence upstream to enable secretion into the endoplasmic reticulum and the subsequent protein secretion pathway. The signal peptide used by the protein entering the endoplasmic reticulum lumen shared similar functions. The present invention used the commonly used α-factor secretion signal as an exemplary signal peptide sequence.

Numerous expression vectors were available for Pichia pastoris, with a promoter included in the vector playing a crucial role. Commonly used promoters included: (1) inducible promoters such as an AOX1 promoter, a FLD1 promoter, a FDH1 promoter, a DAS1 promoter, and a DAS2 promoter, which required methanol or methylamine to induce protein expression. The timing of the transcription and expression of the CPB-positioned fusion functional protein could be regulated by adding these inducers; (2) combined-type promoters like GAP and GCW14, which did not require the inducers. These promoters initiated the transcription and expression of the protein as long as the cell remained viable. That is, the CPB-positioned fusion functional protein was continuously transcribed and expressed; (3) promoters like THI4 and THI11, which did not require the specific inducers, activated protein expression by reducing certain culture components (e.g., thiamine), and also allowed for some regulation of the timing of the transcription and expression of the CPB-positioned fusion functional protein; (4) a promoter regulating other culture conditions, such as a HSP82 promoter, was heat-inducible. During the normal growth of a strain, altering the growth temperature to induce a thermal stress triggered transcription initiation via the HSP82 promoter, thereby regulating the transcription and expression of the CPB-positioned fusion functional protein. Regardless of the specific promoter used, the ultimate function remained identical: The final effect of initiating the transcription and expression of the CPB-positioned fusion functional protein was indistinguishable and fell in the scope of protection of the present invention. The common AOX1 promoter was used as an exemplary illustration herein. Numerous expression vectors existed for Pichia pastoris or the yeast. As long as an ultimate goal was to achieve the expression of the CPB-positioned fusion functional protein, the functions thereof were equivalent. The present invention used the commonly used expression vector to construct a plurality of expression vectors.

DNA sequences SEQ ID NO: 23, SEQ ID NO: 25, SEQ ID NO: 27 and SEQ ID NO: 29 were synthesized by Nanjing GenScript Biotechnology Co., Ltd. Synthesized genes were cloned into expression vectors such as pPICZαB, pFLDα, and pPIC9K (all purchased from Thermo Fisher Scientific (China) Co., Ltd.). A cloning site was positioned between a KEX2 cleavage site sequence in a α-factor secretion signal sequence (with a terminus DNA sequence being AAAGAAGAAGGGGTATCTCTCGAGAAAAGA) and a termination codon "TGA" on the vector. Three sets of recombinant expression vectors for constructing a chassis microorganism were obtained:
(1) pPICZαB-HCPB-PPKEX2, pPICZαB-HCPB-SCKEX2, pPICZαB-RCPB-PPKEX2, and pPICZαB-RCPB-SCKEX2. The AOX1 promoter and a α-factor secretion signal peptide sequence were used.
(2) pFLDα-HCPB-PPKEX2, pFLDα-HCPB-SCKEX2, pFLDα-RCPB-PPKEX2, and pFLDα-RCPB-SCKEX2. The FLD promoter and the α-factor secretion signal peptide sequence were used.
(3) pPIC9K-HCPB-PPKEX2, pPIC9K-HCPB-SCKEX2, pPIC9K-RCPB-PPKEX2, and pPIC9K-RCPB-SCKEX2. The AOX1 promoter and the α-factor secretion signal peptide sequence were used.

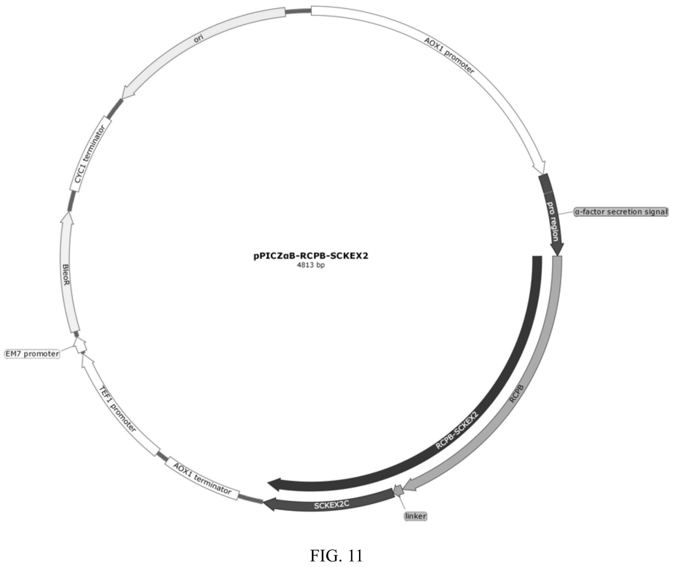

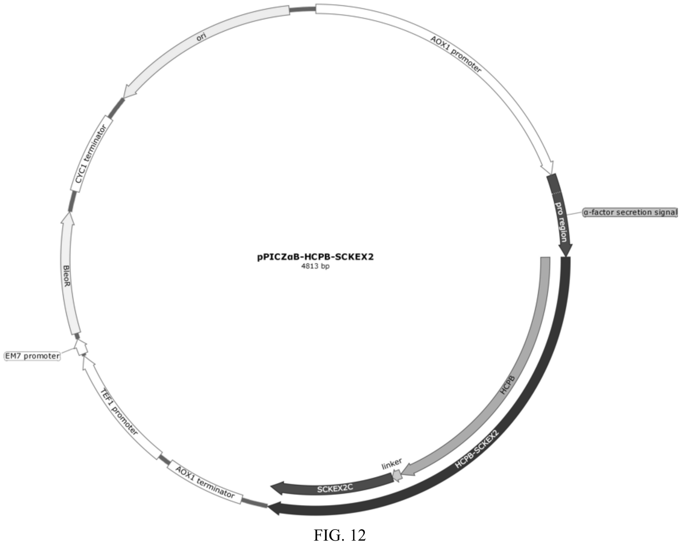

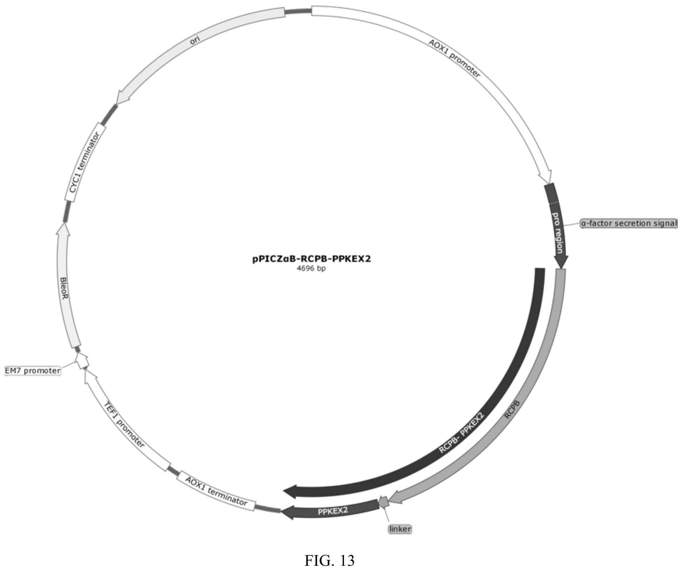

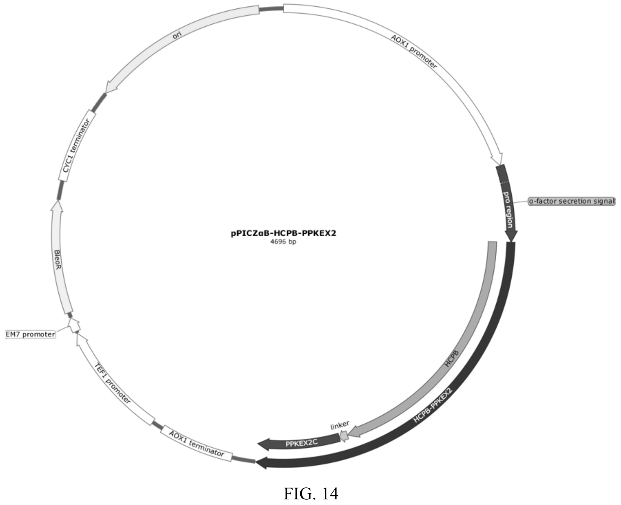

Each of these three sets of the recombinant expression vectors could be used independently and use the α-factor secretion signal peptide sequence. Although the promoters differed, all could be induced with methanol (the FLD promoter could also use methylamine), yielding identical final effects. In an example of the present invention, the first group was used as an example for construction of the subsequent engineered strain. As shown in FIGS 11-14, four recombinant expression vectors constructed in this embodiment were presented.

A blank starting strain used for the chassis cell could be a natural pichia pastoris strain or commercial strains such as X-33, SMD1168, GS115, or other yeast strains. The ultimate effects thereof remained consistent: that is, the expression of CPB-positioned fusion functional protein was constructed in the secretion pathway of the cellular protein and synergized with yeast-specific proteases like Kex2 and Ste13 to express and cleave a long protein. The present invention used the commercial blank starting strain GS115 as an example.

pPICZαB-HCPB-PPKEX2, pPICZαB-HCPB-SCKEX2, pPICZαB-RCPB-PPKEX2, pPICZαB-RCPB-SCKEX2 vector plasmids were digested overnight at 37°C using Pme I (purchased from TaKara (Dalian)) and linearized. The linearized plasmids were recovered using a PCR product purification kit (purchased from Sangon Biotech (Shanghai) Co., Ltd.) so that the volume thereof was controlled under approximately 10 µL. The linearized plasmids were electrotransformed into a competent cell of an empty host strain Pichia pastoris GS115 (purchased from China Center of Industrial Culture Collection) and applied onto a YPD plate containing 0.1 mg/mL Zeocin (purchased from Thermo Fisher Scientific (China) Co., Ltd.). After a single colony grew up, a single clone was picked with a toothpick and inoculated into a 96-well plate.

After 48 hours of incubation at 30°C, the plate was spotted onto the YPD plates containing a 0.5 mg/mL Zeocin and a 1 mg/mL Zeocin, respectively. The strain growing on the high-concentration (1 mg/mL) Zeocin plate was transferred to a BMGY medium shaking flask. After incubation overnight at 30°C and 220 rpm, the medium was replaced with a BMMY medium for induction. 100% methanol was supplemented every 24 hours to a final concentration of 1.0%. After incubation for at least 36 hours, the supernatant and the strain were collected. The strain was lysed with a 8M urea. After a sample was prepared, SDS-PAGE inspection was carried out. An anti-human or anti-rat CPB enzyme antibody (purchased from Sangon Biotech (Shanghai) Co., Ltd.) was used. With a recombinant human CPB enzyme protein or a recombinant rat CPB enzyme protein (purchased from Sangon Biotech (Shanghai) Co., Ltd.) as a positive control and a culture supernatant of an empty host strain as a negative control, detection was performed using the color development by ECL chemiluminescence and an automated chemiluminescence image analysis system (Tanon 5200) to combine an image.

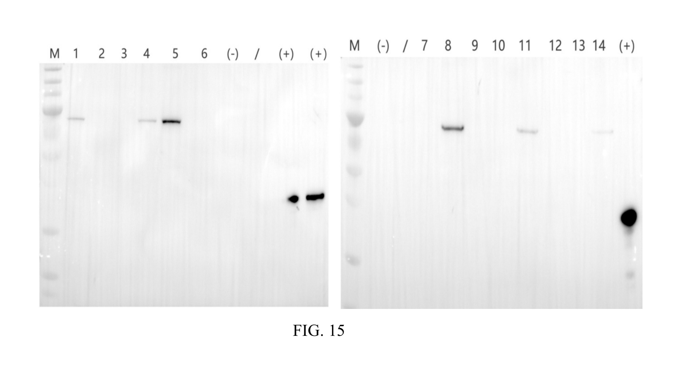

The results were shown in FIG. 15, which showed the detection results of CPB-positioned fusion functional proteins HCPB-PPKEX2, HCPB-SCKEX2, RCPB-PPKEX2, and RCPB-SCKEX2 expressed in an intracellular lysate and a culture supernatant. The panel in the left figure. 1: HCPB-SCKEX2 strain lysate; 2: HCPB-SCKEX2 culture supernatant; 3, 6: HCPB-PPKEX2 culture supernatant; 4, 5: HCPB-PPKEX2 strain lysate; (-): negative control; (+): positive control, a recombinant human CPB enzyme; /: no sample well. Lanes in the right figure: (-): Negative control; (+): Positive control, a CPB enzyme of a recombinant rat ; /: Non-sample well; 7: an RCPB-SCKEX2 strain culture supernatant; 8: a RCPB-SCKEX2 strain lysate; 9, 10, 12, 13: an RCPB-PPKEX2 culture supernatant; 11, 14: an RCPB-PPKEX2 strain lysate.

The human CPB enzyme, serving as the positive control, was successfully detected. However, HCPB-PPKEX2, HCPB-SCKEX2, RCPB-PPKEX2, and RCPB-SCKEX2 were only detected in the intracellular lysate. A band was larger than the positive control, with band sizes consistent with an expected fusion protein (an apparent molecular weight on electrophoresis was naturally larger when fused to other proteins). Most importantly, no corresponding bands were detected in a culture supernatant. This indicates that HCPB-PPKEX2, HCPB-SCKEX2, RCPB-PPKEX2, and RCPB-SCKEX2 expressed as the fusion proteins were not secreted extracellularly but remained intracellular, which aligned with the design objective of the four CPB-positioned fusion functional proteins. That is, the human or rat CPB enzyme was fused with the region with the intracellular membrane positioning or the conversion and transport functions among the respective organelles derived from the carboxy-terminus of the Kex2 enzyme and expressed. Under the influence of the signal peptide, the human or rat CPB enzyme could enter the secretion pathway of the intracellular protein constituted by the endoplasmic reticulum lumen and the trans-Golgi network. However, like the Kex2 enzyme, the human or rat CPB enzyme remained confined in the cell and could not be secreted extracellularly.

Following Invitrogen's instructions, by using YPD plate gradients with varying concentrations of Zeocin antibiotic, a plate was spotted and screened. Combined with intracellular Western blot (WB) detection results, the following engineered strains of the chassis cells fusing the synthetic metabolic pathway of the functional protein were successively screened: HCPB-PPKEX2, HCPB-SCKEX2, RCPB-PPKEX2, and RCPB-SCKEX2. These engineered strains served as a host strain for the subsequent recombinant tandem expression strategy.

All engineered chassis cell samples of the functional pathways of the constructed CPB-positioned fusion functional protein were deposited at China General Microbiological Culture Collection Center (CGMCC), with respective strain deposit numbers:
The strain expressing HCPB-PPKEX2 was deposited as CGMCC No. 25815;
The strain expressing RCPB-PPKEX2 was deposited as CGMCC No.25817;
The strain expressing HCPB-SCKEX2 was deposited as CGMCC No.25816;
The strain expressing RCPB-SCKEX2 was deposited as CGMCC No.25818;

The deposited address for the engineered strains was as follows: No. 3, Compound 1, Beichen West Road, Chaoyang District, Beijing. The deposited date for all was: September 26, 2022. Classification designation for all strains was *Komagataella phaffii.*

**Embodiment 3: Design of an amino acid sequence of a small-molecule collagen and characterization of a tandem repeat expression small-molecule collagen** To achieve the efficient expression of the small-molecule collagen described in the present invention, the amino acid sequence of the target small-molecule collagen was designed according to the following requirements:
(1) Among the tandem repeat small-molecule collagen monomers (or fragments), the site that could be effectively recognized and cleaved by the yeast Kex2 enzyme was designed, which was characterized by the presence of dibasic amino acids such as KR and RR.
(2) Following the dibasic amino acid residues, sequences such as EA, EAEA, or other amino acid sequences (e.g., D or A) that facilitate the recognition and cleavage of the Kex2 enzyme may be present, or the sequence may directly be amino acids inherent to the small-molecule collagen monomer (or fragment) itself (a relevant example was provided in the present invention).
(3) The amino acid sequence of the tandem repeat small-molecule collagen monomer (or fragment) may be any sequence. The specific order and the sequence length of amino acid residues thereof, and the number of tandem repeats for the monomer (or the fragment) were freely configurable and unrestricted. Of course, as the small-molecule collagen, the sequence generally required a typical G-X-Y triplet structure, with the amino acid sequence length controlled in 100 amino acids.

**An exemplary embodiment of the present invention was illustrated as follows:)**
(1) Referring to the amino acid sequence of 3A5D2NT, a repeat tandem sequence was designed and repeated nine times, designated as 3A5D29N-9. This sequence included 522 amino acids, as shown in SEQ ID NO: 30:

EAGKSGDRGESGPAGPAGAPGPAGSRGAPGPQGPRGDKGETGERGAAGIKGHRG
LEKREAGKSGDRGESGPAGPAGAPGPAGSRGAPGPQGPRGDKGETGERGAAGIKGHR
GLEKREAGKSGDRGESGPAGPAGAPGPAGSRGAPGPQGPRGDKGETGERGAAGIKGH
RGLEKREAGKSGDRGESGPAGPAGAPGPAGSRGAPGPQGPRGDKGETGERGAAGIKG
HRGLEKREAGKSGDRGESGPAGPAGAPGPAGSRGAPGPQGPRGDKGETGERGAAGIK
GHRGLEKREAGKSGDRGESGPAGPAGAPGPAGSRGAPGPQGPRGDKGETGERGAAGI
KGHRGLEKREAGKSGDRGESGPAGPAGAPGPAGSRGAPGPQGPRGDKGETGERGAA
GIKGHRGLEKREAGKSGDRGESGPAGPAGAPGPAGSRGAPGPQGPRGDKGETGERGA
AGIKGHRGLEKREAGKSGDRGESGPAGPAGAPGPAGSRGAPGPQGPRGDKGETGERG
AAGIKGHRGLEKR

When 3A5D29N-9 entered the protein secretion pathway constituted by the endoplasmic reticulum and the trans-Golgi network via transcription and translation, the Kex2 enzyme cleaved 3A5D29N-9 between KR and EA in each **KREA** sequence into identical small protein sequences as shown in SEQ ID NO: 31:

EAGKSGDRGESGPAGPAGAPGPAGSRGAPGPQGPRGDKGETGERGAAGIKGHRG
LEKR

Two EA amino acids at amino-termini were cleaved and removed by the Ste 13 protease, while the two amino acids at the carboxy-terminus (KR) were cleaved and removed by the CPB protease. A final secreted product was a fragment of 54-amino acid recombinant small collagen (designated 3A5D29N), whose amino acid sequence was the typical G-X-Y triplet collagen sequence, as shown in SEQ ID NO: 32:
GKSGDRGESGPAGPAGAPGPAGSRGAPGPQGPRGDKGETGERGAAGIKGHRGLE

(2) Referring to the amino acid sequence of 17S3NT, the repeat tandem sequence was designed and repeated 8 times, and designated as 17S28-8. This sequence included 536 amino acids, as shown in SEQ ID NO: 33:

EAGVPGSVGPKGSSGSPGPQGPPGPVGLQGLRGEVGLPGVKGDKGPMGPPGPKG
DQGEKGPRGLEKREAGVPGSVGPKGSSGSPGPQGPPGPVGLQGLRGEVGLPGVKGDK
GPMGPPGPKGDQGEKGPRGLEKREAGVPGSVGPKGSSGSPGPQGPPGPVGLQGLRGE

VGLPGVKGDKGPMGPPGPKGDQGEKGPRGLEKREAGVPGSVGPKGSSGSPGPQGPPG
PVGLQGLRGEVGLPGVKGDKGPMGPPGPKGDQGEKGPRGLEKREAGVPGSVGPKGSS
GSPGPQGPPGPVGLQGLRGEVGLPGVKGDKGPMGPPGPKGDQGEKGPRGLEKREAGV
PGSVGPKGSSGSPGPQGPPGPVGLQGLRGEVGLPGVKGDKGPMGPPGPKGDQGEKGP
RGLEKREAGVPGSVGPKGSSGSPGPQGPPGPVGLQGLRGEVGLPGVKGDKGPMGPPG
PKGDQGEKGPRGLEKREAGVPGSVGPKGSSGSPGPQGPPGPVGLQGLRGEVGLPGVK
GDKGPMGPPGPKGDQGEKGPRGLEKR

When 17S28-8 entered the protein secretion pathway constituted by the endoplasmic reticulum and the trans-Golgi network via transcription and translation, the Kex2 enzyme cleaved 17S28-8 between KR and EA in each **KREA** sequence into identical small protein sequences as shown in SEQ ID NO: 34:

EAGVPGSVGPKGSSGSPGPQGPPGPVGLQGLRGEVGLPGVKGDKGPMGPPGPKG
DQGEKGP RGLEKR

The two EA amino acids at the amino-termini were cleaved and removed by the Ste 13 protease, while the two amino acids at the carboxy-terminus (KR) were cleaved and removed by the CPB protease. A final secreted product was a fragment of 63-amino acid recombinant small collagen (designated 17S28), whose amino acid sequence was the typical G-X-Y triplet collagen sequence, as shown in SEQ ID NO: 35:

GVPGSVGPKGSSGSPGPQGPPGPVGLQGLRGEVGLPGVKGDKGPMGPPGPKGDQ
GEKGPRG LE

(3) Referring to the amino acid sequence of 17S1NNT, the repeat tandem sequence was designed and repeated 6 times anddesignated as 17S1N6-6. This sequence included 540 amino acids, as shown in SEQ ID NO: 36:

EAGPPGSGEKGERGAAGEPGPHGPPGVPGSVGPKGSSGSPGPQGPPGPVGLQGLR
GEVGLPGVKGDKGPMGPPGPKGDQGEKGPRGLEKREAGPPGSGEKGERGAAGEPGPH
GPPGVPGSVGPKGSSGSPGPQGPPGPVGLQGLRGEVGLPGVKGDKGPMGPPGPKGDQ
GEKGPRGLEKREAGPPGSGEKGERGAAGEPGPHGPPGVPGSVGPKGSSGSPGPQGPPG
PVGLQGLRGEVGLPGVKGDKGPMGPPGPKGDQGEKGPRGLEKREAGPPGSGEKGERG

AAGEPGPHGPPGVPGSVGPKGSSGSPGPQGPPGPVGLQGLRGEVGLPGVKGDKGPMGP
PGPKGDQGEKGPRGLEKREAGPPGSGEKGERGAAGEPGPHGPPGVPGSVGPKGSSGSP
GPQGPPGPVGLQGLRGEVGLPGVKGDKGPMGPPGPKGDQGEKGPRGLEKREAGPPGS
GEKGERGAAGEPGPHGPPGVPGSVGPKGSSGSPGPQGPPGPVGLQGLRGEVGLPGVKG
DKGPMGPPGPKGDQGEKGPRGLEKR

When 17S1N6-6 entered the protein secretion pathway constituted by the endoplasmic reticulum and the trans-Golgi network via transcription and translation, the Kex2 enzyme cleaved 17S1N6-6 between KR and EA in each **KREA** sequence into identical small protein sequences as shown in SEQ ID NO: 37:

EAGPPGSGEKGERGAAGEPGHGPPGVPGSVGPKGSSGSPGPQGPPGPVGLQGLR
GEVGLPG VKGDKGPMGPPGPKGDQGEKGPRGLEKR

The two EA amino acids at the amino-termini were cleaved and removed by the Ste 13 protease, while the two amino acids at the carboxy-terminus (KR) were cleaved and removed by the CPB protease. A final secreted product was a fragment of 86-amino acid recombinant small collagen (designated 17S1N6), whose amino acid sequence was the typical G-X-Y triplet collagen sequence, as shown in SEQ ID NO: 38:

GPPGSGEKGERGAAGEPGPHGPPGVPGSVGPKGSSGSPGPQGPPGPVGLQGLRGE
VGLPGVK GDKGPMGPPGPKGDQGEKGPRGLE

(4) Referring to the amino acid sequence of 17S1NNT, the repeat tandem sequence was designed and repeated 7 times and designated as 17S1N7-7. This sequence included 528 amino acids, as shown in SEQ ID NO: 39:

EAGPPGSGEKGERGAAGEPGPHGPPGVPGSVGPKGSSGSPGPQGPPGPVGLQGLR
GEVGLPGVKGDKGPMGPPGPKGDQGEKGPRGKREAGPPGSGEKGERGAAGEPGPHG
PPGVPGSVGPKGSSGSPGPQGPPGPVGLQGLRGEVGLPGVKGDKGPMGPPGPKGDQGE
KGPRGKREAGPPGSGEKGERGAAGEPGPHGPPGVPGSVGPKGSSGSPGPQGPPGPVGL
QGLRGEVGLPGVKGDKGPMGPPGPKGDQGEKGPRGKREAGPPGSGEKGERGAAGEP
GPHGPPGVPGSVGPKGSSGSPGPQGPPGPVGLQGLRGEVGLPGVKGDKGPMGPPGPKG
DQGEKGPRGKREAGPPGSGEKGERGAAGEPGPHGPPGVPGSVGPKGSSGSPGPQGPPG

PVGLQGLRGEVGLPGVKGDKGPMGPPGPKGDQGEKGPRGKREAGPPGSGEKGERGA
AGEPGPHGPPGVPGSVGPKGSSGSPGPQGPPGPVGLQGLRGEVGLPGVKGDKGPMGPP
GPKGDQGEKGPRGKR

When 17S1N7-7 entered the protein secretion pathway constituted by the endoplasmic reticulum and the trans-Golgi network via transcription and translation, the Kex2 enzyme cleaved 17S1N7-7 between KR and EA in each **KREA** sequence into identical small protein sequences as shown in SEQ ID NO: 40:

EAGPPGSGEKGERGAAGEPGHGPPGVPGSVGPKGSSGSPGPQGPPGPVGLQGLR
GEVGLPG VKGDKGPMGPPGPKGDQGEKGPRGKR

The two EA amino acids at the amino-termini were cleaved and removed by the Ste 13 protease, while the two amino acids at the carboxy-terminus (KR) were cleaved and removed by the CPB protease. A final secreted product was a fragment of 84-amino acid recombinant small collagen (designated 17S1N7), whose sequence was as shown in SEQ ID NO: 41:

GPPGSGEKGERGAAGEPGPHGPPGVPGSVGPKGSSGSPGPQGPPGPVGLQGLRGE
VGLPG VKGDKGPMGPPGPKGDQGEKGPRG

(5) Referring to the amino acid sequence of 17S1NNT, the repeat tandem sequence was designed and repeated 7 times and designated as 17S1NK-7. This sequence included 595 amino acids, as shown in SEQ ID NO: 42:

EAGPPGSGEKGERGAAGEPGPHGPPGVPGSVGPKGSSGSPGPQGPPGPVGLQGLR
GEVGLPGVKGDKGPMGPPGPKGDQGEKGKREAGPPGSGEKGERGAAGEPGPHGPPG
VPGSVGPKGSSGSPGPQGPPGPVGLQGLRGEVGLPGVKGDKGPMGPPGPKGDQGEKG
KREAGPPGSGEKGERGAAGEPGPHGPPGVPGSVGPKGSSGSPGPQGPPGPVGLQGLRG
EVGLPGVKGDKGPMGPPGPKGDQGEKGKREAGPPGSGEKGERGAAGEPGPHGPPGVP
GSVGPKGSSGSPGPQGPPGPVGLQGLRGEVGLPGVKGDKGPMGPPGPKGDQGEKGKR
EAGPPGSGEKGERGAAGEPGPHGPPGVPGSVGPKGSSGSPGPQGPPGPVGLQGLRGEV
GLPGVKGDKGPMGPPGPKGDQGEKGKREAGPPGSGEKGERGAAGEPGPHGPPGVPGS
VGPKGSSGSPGPQGPPGPVGLQGLRGEVGLPGVKGDKGPMGPPGPKGDQGEKGKREA
GPPGSGEKGERGAAGEPGPHGPPGVPGSVGPKGSSGSPGPQGPPGPVGLQGLRGEVGL

PGVKGDKGPMGPPGPKGDQGEKGKR

When 17S1NK-7 entered the protein secretion pathway constituted by the endoplasmic reticulum and the trans-Golgi network via transcription and translation, the Kex2 enzyme cleaved 17S1NK-7 between KR and EA in each **KREA** sequence into identical small protein sequences as shown in SEQ ID NO: 43:

EAGPPGSGEKGERGAAGEPGHGPPGVPGSVGPKGSSGSPGPQGPPGPVGLQGLR
GEVGLPG VKGDKGPMGPPGPKGDQGEKGKR

The two EA amino acids at the amino-termini were cleaved and removed by the Ste 13 protease, while the two amino acids at the carboxy-terminus (KR) were cleaved and removed by the CPB protease. A final secreted product was a fragment of 84-amino acid recombinant small collagen (designated 17S1NK), whose sequence was as shown in SEQ ID NO: 44:

GPPGSGEKGERGAAGEPGPHGPPGVPGSVGPKGSSGSPGPQGPPGPVGLQGLRGE
VGLPG VKGDKGPMGPPGPKGDQGEKGPRG

(6) Referring to the amino acid sequence of 3A5D1, the repeat tandem sequence was designed and repeated 5 times and designated as 3A5D15D-5. This sequence included 510 amino acids, as shown in SEQ ID NO: 45:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIKGHRGLEKRDGNPGSDGL
PGRDGSPGGKGDRGENGSPGAPGAPGHPGPPGPVGPAGKSGDRGESGPAGPAGAPGP
AGSRGAPGPQGPRGDKGETGERGAAGIKGHRGLEKRDGNPGSDGLPGRDGSPGGKGD
RGENGSPGAPGAPGHPGPPGPVGPAGKSGDRGESGPAGPAGAPGPAGSRGAPGPQGPR
GDKGETGERGAAGIKGHRGLEKRDGNPGSDGLPGRDGSPGGKGDRGENGSPGAPGAP
GHPGPPGPVGPAGKSGDRGESGPAGPAGAPGPAGSRGAPGPQGPRGDKGETGERGAA
GIKGHRGLEKRDGNPGSDGLPGRDGSPGGKGDRGENGSPGAPGAPGHPGPPGPVGPA
GKSGDRGESGPAGPAGAPGPAGSRGAPGPQGPRGDKGETGERGAAGIKGHRGLEKR

When 3A5D15D-5 entered the protein secretion pathway constituted by the endoplasmic reticulum and the trans-Golgi network via transcription and translation, the Kex2 enzyme cleaved 3A5D15D-5 between KR and D in each **KRD** sequence into identical small protein sequences as shown in SEQ ID NO: 46:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIKGHRGLEKR

The two amino acids at the carboxy-termini (KR) were cleaved and removed by the CPB protease. A final secreted product was a fragment of 100-amino acid recombinant small collagen (designated 3A5D15D), which was the typical G-X-Y triplet collagen sequence, as shown in SEQ ID NO: 47:

DGNPGSDGLPGRDGSPGGKGDRGENGSPGAPGAPGHPGPPGPVGPAGKSGDRGE
SGPAG PAGAPGPAGSRGAPGPQGPRGDKGETGERGAAGIKGHRGLE

(7) Similarly, using other cleavage sites could also achieve the same effect as 3A5D15D. The enzyme cleavage site region sequence was designed as **KREA** to create a tandem repeat sequence, which was repeated 5 times and named 3A5D15E-5, with a total of 520 amino acids. A sequence thereof was as shown in SEQ ID NO: 48.

EADGNPGSDGLPGRDGSPGGKGDRGENGSPGAPGAPGHPGPPGPVGPAGKSGDR
GESGPAGPAGAPGPAGSRGAPGPQGPRGDKGETGERGAAGIKGHRGLEKREADGNPG
SDGLPGRDGSPGGKGDRGENGSPGAPGAPGHPGPPGPVGPAGKSGDRGESGPAGPAG
APGPAGSRGAPGPQGPRGDKGETGERGAAGIKGHRGLEKREADGNPGSDGLPGRDGS
PGGKGDRGENGSPGAPGAPGHPGPPGPVGPAGKSGDRGESGPAGPAGAPGPAGSRGA
PGPQGPRGDKGETGERGAAGIKGHRGLEKREADGNPGSDGLPGRDGSPGGKGDRGEN
GSPGAPGAPGHPGPPGPVGPAGKSGDRGESGPAGPAGAPGPAGSRGAPGPQGPRGDKG
ETGERGAAGIKGHRGLEKREADGNPGSDGLPGRDGSPGGKGDRGENGSPGAPGAPGH
PGPPGPVGPAGKSGDRGESGPAGPAGAPGPAGSRGAPGPQGPRGDKGETGERGAAGIK
GHRGLEKR

When 3A5D15E-5 entered the protein secretion pathway constituted by the endoplasmic reticulum and the trans-Golgi network via transcription and translation, the Kex2 enzyme cleaved 3A5D15E-5 between KR and EA in each **KRD** sequence into identical small protein sequences as shown in SEQ ID NO: 49:

EADGNPGSDGLPGRDGSPGGKGDRGENGSPGAPGAPGHPGPPGPVGPAGKSGDR
GESGPAGP AGAPGPAGSRGAPGPQGPRGDKGETGERGAAGIKGHRGLEKR

The two EA amino acids at the amino-termini were cleaved and removed by the Ste 13 protease, while the two amino acids at the carboxy-terminus (KR) were cleaved and removed by the CPB protease. A final secreted product was a fragment of 100-amino acid recombinant small collagen (same as the sequence obtained after expression and cleavage of 3A5D15D, designated 3A5D15E), which was the typical G-X-Y triplet collagen sequence. A sequence thereof wasas shown in SEQ ID NO: 50:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIKGHRGLE

(8) Referring to the amino acid sequence of 3A5D1NT, the repeat tandem sequence was designed and repeated 6 times and designated as 3A5D15R-6, with a total of 570 amino acids. A sequence thereof wasas shown in SEQ ID NO: 51:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIKRDGNPGSDGLPGRDGSP
GGKGDRGENGSPGAPGAPGHPGPPGPVGPAGKSGDRGESGPAGPAGAPGPAGSRGAP
GPQGPRGDKGETGERGAAGIKRDGNPGSDGLPGRDGSPGGKGDRGENGSPGAPGAPG
HPGPPGPVGPAGKSGDRGESGPAGPAGAPGPAGSRGAPGPQGPRGDKGETGERGAAGI
KRDGNPGSDGLPGRDGSPGGKGDRGENGSPGAPGAPGHPGPPGPVGPAGKSGDRGES
GPAGPAGAPGPAGSRGAPGPQGPRGDKGETGERGAAGIKRDGNPGSDGLPGRDGSPG
GKGDRGENGSPGAPGAPGHPGPPGPVGPAGKSGDRGESGPAGPAGAPGPAGSRGAPG
PQGPRGDKGETGERGAAGIKRDGNPGSDGLPGRDGSPGGKGDRGENGSPGAPGAPGH
PGPPGPVGPAGKSGDRGESGPAGPAGAPGPAGSRGAPGPQGPRGDKGETGERGAAGIK
R

When 3A5D15R-6 entered the protein secretion pathway constituted by the endoplasmic reticulum and the trans-Golgi network via transcription and translation, the Kex2 enzyme cleaved 3A5D15R-6 between KR and D in each **KRD** sequence into identical small protein sequences as shown in SEQ ID NO: 52:

DGNPGSDGLPGRDGSPGGKGDRGENGSPGAPGAPGHPGPPGPVGPAGKSGDRGE
SGPAG PAGAPGPAGSRGAPGPQGPRGDKGETGERGAAGIKR

The two amino acids at the carboxy-termini (KR) were cleaved and removed by the CPB protease. A final secreted product was a fragment of 93-amino acid recombinant small collagen (designated 3A5D15R). A sequence thereof was as shown in SEQ ID NO: 53:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGI

(9) Referring to the amino acid sequence of 3A5D1NT, the repeat tandem sequence was designed and repeated 6 times and designated as 3A5D15KR-6, with a total of 582 amino acids. A sequence thereof was as shown in SEQ ID NO: 54:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIKGKRDGNPGSDGLPGRDG
SPGGKGDRGENGSPGAPGAPGHPGPPGPVGPAGKSGDRGESGPAGPAGAPGPAGSRG
APGPQGPRGDKGETGERGAAGIKGKRDGNPGSDGLPGRDGSPGGKGDRGENGSPGAP
GAPGHPGPPGPVGPAGKSGDRGESGPAGPAGAPGPAGSRGAPGPQGPRGDKGETGER
GAAGIKGKRDGNPGSDGLPGRDGSPGGKGDRGENGSPGAPGAPGHPGPPGPVGPAGK
SGDRGESGPAGPAGAPGPAGSRGAPGPQGPRGDKGETGERGAAGIKGKRDGNPGSDG
LPGRDGSPGGKGDRGENGSPGAPGAPGHPGPPGPVGPAGKSGDRGESGPAGPAGAPGP
AGSRGAPGPQGPRGDKGETGERGAAGIKGKRDGNPGSDGLPGRDGSPGGKGDRGENG
SPGAPGAPGHPGPPGPVGPAGKSGDRGESGPAGPAGAPGPAGSRGAPGPQGPRGDKGE
TGERGAAGIKGKR

When 3A5D15KR-6 entered the protein secretion pathway constituted by the endoplasmic reticulum and the trans-Golgi network via transcription and translation, the Kex2 enzyme cleaved 3A5D15KR-6 between KR and D in each **KRD** sequence into identical small protein sequences as shown in SEQ ID NO: 55:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIKGKR

The two amino acids at the carboxy-termini (KR) were cleaved and removed by the CPB protease. A final secreted product was a fragment of 95-amino acid recombinant small collagen (designated 3A5D15KR). A sequence thereof was as shown in SEQ ID NO: 56:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIKG

(10) Referring to the amino acid sequence of 3A5D1NT, the repeat tandem sequence was designed and repeated 6 times and designated as 3A5D15EKR-6, with a total of 576 amino acids. A sequence thereof was as shown in SEQ ID NO: 57:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIEKRDGNPGSDGLPGRDGS
PGGKGDRGENGSPGAPGAPGHPGPPGPVGPAGKSGDRGESGPAGPAGAPGPAGSRGA
PGPQGPRGDKGETGERGAAGIEKRDGNPGSDGLPGRDGSPGGKGDRGENGSPGAPGA
PGHPGPPGPVGPAGKSGDRGESGPAGPAGAPGPAGSRGAPGPQGPRGDKGETGERGA
AGIEKRDGNPGSDGLPGRDGSPGGKGDRGENGSPGAPGAPGHPGPPGPVGPAGKSGD
RGESGPAGPAGAPGPAGSRGAPGPQGPRGDKGETGERGAAGIEKRDGNPGSDGLPGR
DGSPGGKGDRGENGSPGAPGAPGHPGPPGPVGPAGKSGDRGESGPAGPAGAPGPAGS
RGAPGPQGPRGDKGETGERGAAGIEKRDGNPGSDGLPGRDGSPGGKGDRGENGSPGA
PGAPGHPGPPGPVGPAGKSGDRGESGPAGPAGAPGPAGSRGAPGPQGP
RGDKGETGERGAAGIEKR

When 3A5D15EKR-6 entered the protein secretion pathway constituted by the endoplasmic reticulum and the trans-Golgi network via transcription and translation, the Kex2 enzyme cleaved 3A5D15EKR-6 between KR and D in each **KRD** sequence into identical small protein sequences as shown in SEQ ID NO: 58:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIEKR

The two amino acids at the carboxy-termini (KR) were cleaved and removed by the CPB protease. A final secreted product was a fragment of 94-amino acid recombinant small collagen (designated 3A5D15EKR), which was the typical G-X-Y triplet collagen sequence. A sequence thereof was as shown in SEQ ID NO: 59:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIE

(11) Referring to the amino acid sequence of 3A5D1NT, the repeat tandem sequence was designed and repeated 6 times and designated as 3A5D15G-6, with a total of 576 amino acids. A sequence thereof was as shown in SEQ ID NO: 60:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIGKRDGNPGSDGLPGRDGS
PGGKGDRGENGSPGAPGAPGHPGPPGPVGPAGKSGDRGESGPAGPAGAPGPAGSRGA
PGPQGPRGDKGETGERGAAGIGKRDGNPGSDGLPGRDGSPGGKGDRGENGSPGAPGA
PGHPGPPGPVGPAGKSGDRGESGPAGPAGAPGPAGSRGAPGPQGPRGDKGETGERGA
AGIGKRDGNPGSDGLPGRDGSPGGKGDRGENGSPGAPGAPGHPGPPGPVGPAGKSGD
RGESGPAGPAGAPGPAGSRGAPGPQGPRGDKGETGERGAAGIGKRDGNPGSDGLPGR
DGSPGGKGDRGENGSPGAPGAPGHPGPPGPVGPAGKSGDRGESGPAGPAGAPGPAGS
RGAPGPQGPRGDKGETGERGAAGIGKRDGNPGSDGLPGRDGSPGGKGDRGENGSPGA
PGAPGHPGPPGPVGPAGKSGDRGESGPAGPAGAPGPAGSRGAPGPQGPRGDKGETGER
GAAGIGKR

When 3A5D15G-6 entered the protein secretion pathway constituted by the endoplasmic reticulum and the trans-Golgi network via transcription and translation, the Kex2 enzyme cleaved 3A5D15G-6 between KR and D in each **KRD** sequence into identical small protein sequences as shown in SEQ ID NO: 61:

DGNPGSDGLPGRDGSPGGKGDRGENGSPGAPGAPGHPGPPGPVGPAGKSGDRGE
SGPAGPAG APGPAGSRGAPGPQGPRGDKGETGERGAAGIGKR

The two amino acids at the carboxy-termini (KR) were cleaved and removed by the CPB protease. A final secreted product was a fragment of 94-amino acid recombinant small collagen (designated 3A5D15G), which was the typical G-X-Y triplet collagen sequence. A sequence thereof was as shown in SEQ ID NO: 62:

DGNPGSDGLPGRDGSPGGKGDRGENGSPGAPGAPGHPGPPGPVGPAGKSGDRGE
SGPAGPAGAPGPAGSRGAPGPQGPRGDKGETGERGAAGIG

Besides one region from a human type-III collagen or a human type-XVII collagen being taken as the basis to design a tandem repeat sequence, sequences spliced by two or more regions from a human collagen sequence could also be used as monomers for tandem repeat. This method could also be applicable to the tandem repeat expression system described in the present invention (including the tandem repeat expression sequence design method and chassis cell usage) as well as a monomer expression system.

(12) Referring to several distinct functional regions of the human type-III collagen sequence (as per the protein sequence with ID P02461 in the Uniprot database, https://www.uniprot.org/uniprotkb/P02461), the amino acid sequences at positions 880-915, 949-966, 1012-1038, and 1060-1074 were combined to design the recombinant small-molecule collagen. A total of 96 amino acids could be used for was a tandem monomer. A sequence thereof was as shown as SEQ ID NO: 63:

GPPGPAGNTGAPGSPGVSGPKGDAGQPGEKGSPGAQGPQGVKGESGKPGANGLS
GENGSPGA PGAPGHPGPPGPVGPAGKSGAPGPQGPRGDKGET

Therefore, the repeat tandem sequence was designed and repeated six times and designated as 3A5D16M-6 with a total of, 500 amino acids. A sequence thereof was shown in SEQ ID NO: 64:

EAGPPGPAGNTGAPGSPGVSGPKGDAGQPGEKGSPGAQGPQGVKGESGKPGANG
LSGENGSPGAPGAPGHPGPPGPVGPAGKSGAPGPQGPRGDKGLEKREAGPPGPAGNTG
APGSPGVSGPKGDAGQPGEKGSPGAQGPQGVKGESGKPGANGLSGENGSPGAPGAPG
HPGPPGPVGPAGKSGAPGPQGPRGDKGLEKREAGPPGPAGNTGAPGSPGVSGPKGDA
GQPGEKGSPGAQGPQGVKGESGKPGANGLSGENGSPGAPGAPGHPGPPGPVGPAGKS
GAPGPQGPRGDKGLEKREAGPPGPAGNTGAPGSPGVSGPKGDAGQPGEKGSPGAQGP
QGVKGESGKPGANGLSGENGSPGAPGAPGHPGPPGPVGPAGKSGAPGPQGPRGDKGL
EKREAGPPGPAGNTGAPGSPGVSGPKGDAGQPGEKGSPGAQGPQGVKGESGKPGANG
LSGENGSPGAPGAPGHPGPPGPVGPAGKSGAPGPQGPRGDKGLEKR

When 3A5D16M-6 entered the protein secretion pathway constituted by the endoplasmic reticulum and the trans-Golgi network via transcription and translation, the Kex2 enzyme cleaved 3A5D16M-6 between KR and EA in each **KREA** sequence into identical small protein sequences as shown in SEQ ID NO: 65:

EAGPPGPAGNTGAPGSPGVSGPKGDAGQPGEKGSPGAQGPQGVKGESGKPGANG
LSGENGSP GAPGAPGHPGPPGPVGPAGKSGAPGPQGPRGDKGLEKR

The two EA amino acids at the amino-termini were cleaved and removed by the Ste 13 protease, while the two amino acids at the carboxy-terminus (KR) were cleaved and removed by the CPB protease. A final secreted product was a fragment of 96-amino acid recombinant small collagen (designated 3A5D16M). A sequence thereof was as shown in SEQ ID NO: 66:

GPPGPAGNTGAPGSPGVSGPKGDAGQPGEKGSPGAQGPQGVKGESGKPGANGLS
GENGSPGA PGAPGHPGPPGPVGPAGKSGAPGPQGPRGDKGLE

(13) Referring to the two functional regions of the human type-III collagen sequence (based on protein sequence ID P02461 in the Uniprot database, https://www.uniprot.org/uniprotkb/P02461), namely, the amino acid sequences at positions 1060-1074 and 1015-1038 were combined. A total of 39 amino acids could be used for monomer expression. A sequence thereof was shown in SEQ ID NO: 67:
GSPGAPGAPGHPGPPGPVGPAGKSGAPGPQGPRGDKGET

Therefore, the repeat tandem sequence was designed and repeated 12 times and designated as 3A5D15M-12, with a total of 516 amino acids. A sequence thereof was shown in SEQ ID NO: 68:

EAGSPGAPGAPGHPGPPGPVGPAGKSGAPGPQGPRGDKGLEKREAGSPGAPGAPG
HPGPPGPVGPAGKSGAPGPQGPRGDKGLEKREAGSPGAPGAPGHPGPPGPVGPAGKSG
APGPQGPRGDKGLEKREAGSPGAPGAPGHPGPPGPVGPAGKSGAPGPQGPRGDKGLE
KREAGSPGAPGAPGHPGPPGPVGPAGKSGAPGPQGPRGDKGLEKREAGSPGAPGAPGH
PGPPGPVGPAGKSGAPGPQGPRGDKGLEKREAGSPGAPGAPGHPGPPGPVGPAGKSGA
PGPQGPRGDKGLEKREAGSPGAPGAPGHPGPPGPVGPAGKSGAPGPQGPRGDKGLEKR
EAGSPGAPGAPGHPGPPGPVGPAGKSGAPGPQGPRGDKGLEKREAGSPGAPGAPGHPG
PPGPVGPAGKSGAPGPQGPRGDKGLEKREAGSPGAPGAPGHPGPPGPVGPAGKSGAPG
PQGPRGDKGLEKREAGSPGAPGAPGHPGPPGPVGPAGKSGAPGPQGPRGDKGLEKR

When 3A5D15M-12 entered the protein secretion pathway constituted by the endoplasmic reticulum and the trans-Golgi network via transcription and translation, the Kex2 enzyme cleaved 3A5D15M-12 between KR and EA in each **KREA** sequence into identical small protein sequences as shown in SEQ ID NO: 69:
EAGSPGAPGAPGHPGPPGPVGPAGKSGAPGPQGPRGDKGLEKR

The two EA amino acids at the amino-terminus were cleaved and removed by the Ste 13 protease, while the two amino acids at the carboxy-terminus (KR) were cleaved and removed by the CPB protease. A final secreted product was a fragment of 39-amino acid recombinant small collagen (designated 3A5D15M). A sequence thereof was as shown in SEQ ID NO: 70:
GSPGAPGAPGHPGPPGPVGPAGKSGAPGPQGPRGDKGLE

An artificially designed non-natural collagen sequence could theoretically also be applied to a repeat tandem expression system of the present invention. The principle was as described above and will not be repeated here.

### Construction of a recombinant expression vector of the repeat tandem expression system and identification of expression efficiency:

In this embodiment, based on the design of the chassis cell and the above amino acid sequence, the expression vector used for the recombinant small-molecule collagen was not restricted, as long as the selected expression vector had a signal peptide sequence capable of importing the translated protein into the endoplasmic reticulum. Commonly used expression vectors such as pPICZαB, pFLDα, and pPIC9K were suitable. Construction based on other expression vectors with similar functions were also in the scope of protection of the present invention. Similarly, a DNA sequence for encoding the corresponding recombinant small-molecule collagen may be optimized. As long as the final encoded amino acid sequences remained identical, such optimization fell in the scope of protection of the present invention.

A DNA sequence for encoding 3A5D29N-9 was shown in SEQ ID NO: 71:

gaagctggaaagtccggcgaccggggagagtcagggcctgcagggcctgctggtgccccaggaccggccggcagtagaggt
gctcctggtcctcaaggaccacggggcgacaaaggtgaaacaggtgaacgaggtgcggcaggtataaaagggcacagagggctaga
aaagcgggaagcaggaaaaagtggcgatcgcggggaatcaggtcccgcggggccggcgggtgcgcccggaccggcggggtccag

aggtgcccctggaccgcagggacctcggggtgacaaaggtgaaactggggaaaggggggctgcgggcataaagggacaccgcgga
ctcgagaagagagaggcaggtaaaagtggggatagaggcgaatctggtccggcaggaccagcgggcgcccccggtcctgctggatc
acgaggtgccccgggtccgcaaggtcctaggggtgacaaaggcgagacgggcgaacgaggggcagcaggaattaaaggccaccga
gggctggagaagcgggaagcaggtaaatcaggagataggggggaaagcggaccggccggacccgctggtgcgccgggacctgct
ggatcgagaggtgcaccgggtccccaagggcctcgtggagataaaggggagaccggggagcgcggggctgcagggattaagggac
accgcggcttggagaagcgagaggctggtaaaagcggcgaccgcggcgaatcgggtccggcgggtcccgcgggcgcacctgggcc
tgctgggtctaggggagcgccagggcctcaaggtcctcgcggtgacaagggagaaacaggcgagcgtggggcagcgggcattaaag
gtcatcgtgggttagaaaagcgtgaggctggcaaatctggcgatcgcggggagagcggtccagcaggtccggctggtgctccaggtcc
agctggtagccgtggcgccccaggtcctcaaggtcctcgtggcgacaaaggggagaccggcgagcgtggagcggcaggcatcaaag
gtcatcgtggacttgaaaagagggaggcgggaaagtcgggcgatcggggtgaatccggcccagcgggtcctgctggagccccaggc
cccgctggatcgcggggcgcacctggcccccaaggaccgcgtggggataaaggggagactggcgagagaggcgccgccggaatc
aaaggtcaccggggattagaaaagagggaggcagggaagtcgggtgatcgaggcgaatcaggtcctgctggacccgctggcgcgcc
gggtcctgctggtagcagaggagcccctgggcctcaaggcccgaggggtgataagggtgaaaccggagaacgaggcgccgctggta
taaagggacatcgtggactcgagaagcgagaagcggggaaaagtggggacagaggagagtctggtcctgccggacccgccggtgct
cctggtccagcaggctcccgcggcgcgcctggtccgcaaggtcctaggggagacaaaggagaaacgggcgagcgaggcgccgccg
ggatcaaggggcatcgggggttggagaagaggtaa

A DNA sequence for encoding 17S28-8 was shown in SEQ ID NO: 72:

gaggccggtgtacccggaagcgtggggccgaagggcagctcgggttcaccaggcccacaggggccaccaggcccggtagg
cctacaaggtttgcgcggtgaggtggggttacccggagtaaaaggtgataaggggcctatggggcctcccggtcctaaaggcgaccag
ggtgaaaagggtccgcggggactggagaaaagagaagcgggtgttccagggtcggttggtcctaagggaagttcgggttctccgggg
ccgcaaggtcctccaggtcccgtcggtttacagggcctacgtggagaagtcggactccccggagttaagggcgataagggacccatgg
gtcctcctggtcccaaaggtgaccaaggcgagaaaggcccaaggggactcgagaagcgggaagcgggagtccctggctccgttggc
cctaagggaagttccggaagtccgggaccgcaaggtcctcctggccctgtcggcctccaaggtttgaggggagaggttggactccccg
gcgtcaagggcgacaaaggccctatggggccacctgggcctaagggcgaccaaggcgagaaaggccctcgcgggcttgagaagcg
tgaagctggagtgcctgggtcagtaggcccaaaaggatcgtccggatcaccgggacctcaggggccaccgggtcctgtaggtctacag
ggcctcaggggcgaggtaggtttacctggagtcaagggagataagggacctatggggccgccaggtccgaaaggcgatcaagggga
aaaagggcctcgtggactagaaaaacgtgaggcaggcgtccctggatcggttggtcctaaaggttcttcaggtagtcccggtccccagg
gacctcccgggccggtagggctacagggcttacgaggggaggtgggcctcccaggggttaaaggggataaaggtcctatgggtcctc

cgggtccaaaaggtgatcagggtgagaagggtcctcgtgggctggaaaagagagaagcaggtgttccaggctctgttggaccgaagg
ggtcttctggaagccctggtcctcaaggtcctcctggtccggtcggacttcagggattgcgaggcgaggtaggtctgcccggggttaagg
gtgacaaagggccaatgggaccgccagggccgaaaggcgaccaaggtgaaaaaggccctcgaggtctggaaaaaagagaagctgg
agtaccagggagcgtcggccccaaaggaagttcggggtcccctggaccgcaagggccaccaggtcccgtagggttacaaggtctgcg
gggggaggttgggcttcccggagttaaaggtgataagggaccaatgggtcctcccggtccaaagggtgatcaaggagagaaaggacc
gcgcgggctagaaaagagggaggccggtgtcccaggtagcgttggtcctaagggcagctcaggctccccaggaccccaagggccgc
cggggcctgtggggttgcaaggtttgcgaggagaagtgggccttcccggggtgaagggggacaagggacctatgggaccacccggtc
ccaaaggtgaccagggtgaaaagggtcctcgtggacttgaaaaaagataa

A DNA sequence for encoding 17S1N6-6 was shown in SEQ ID NO: 73:

gaagcgggacctcccggcagtggtgaaaagggcgaacgaggcgcagccggagagcctggacctcatgggcctccgggcgtc
ccgggaagtgttggtcctaaaggtagctccggatctccaggtccacagggaccacctggcccggtaggccttcaaggactgagaggtg
aggtcggccttccaggcgtgaagggcgataaaggtcctatgggtccacctggacccaagggagatcagggggagaaagggccgagg
ggcttagaaaaacgcgaagcagggcctcctgggagcggagaaaagggcgagcgcggagccgcaggcgagccaggtcctcatggtc
ctccaggagtgcctgggtccgtcggcccaaaggggtcttctggttcgcctgggcctcaaggaccaccaggtcccgttggtttgcagggtc
tgcgtggagaagtgggccttccgggagttaagggcgacaagggtcctatggggcctccggggccgaagggcgatcagggcgaaaaa
ggcccccgcgggttagaaaaaagagaggcaggaccacccggctcaggtgagaagggtgaaagaggagccgctggggagccagga
ccacacggcccccccggagtcccgggatctgttggacccaaaggttcttctgggagcccgggtccccagggaccgccaggcccagta
ggtctacaaggactcagaggggaggtgggtctaccgggggtcaaaggagataagggtcctatgggtcctcccggtccaaaaggggac
caaggagagaagggacctcggggtttggaaaaacgggaagctggtcctcccggtagcggtgagaaaggcgagcgaggtgctgctgg
ggaaccggggccacacggtcctcctggcgtgcccggctcggtcggacctaaaggttcttctggttctccgggtcctcaggggccgcctg
gtcctgtcgggcttcaaggtctccgcggtgaggttgggctccccggtgtcaagggagacaaagggccgatggggccgccaggtcccaa
gggagaccagggagaaaaaggtcctcgtgggttagaaaaacgggaggcgggaccgcctggttccggcgagaagggggaacgagg
cgcggccggggaaccaggaccccatggaccgccaggtgttccggggtccgtaggtccgaaaggctcatcgggttcgcctggcccaca
aggaccaccaggcccagtgggcctacaaggtctgaggggggaagttgggctccctggcgtaaagggagacaaagggccgatgggtc
ctcctggacccaaaggtgaccaaggagaaaaaggtccgaggggcttagagaaacgtgaagcaggtccgccaggttcaggtgagaag
ggggaacgtggagctgccggggagcctggcccgcacggccctcccggcgttcccggctcagttggaccaaagggatcgagtggaag
tcctggtcctcaaggcccacctggtccagtaggtctacagggcttgaggggggaggtaggactgccgggcgtaaagggcgataagggt
cccatgggtcctccagggcctaaaggtgatcagggggagaagggtccccgagggttggagaagcggtaa

A DNA sequence for encoding 17S1N7-7 was shown in SEQ ID NO: 74:

gaggcaggtcctccgggttctggtgagaagggcgaaaggggagctgcgggagaacctggcccacatggtcctcctggtgtacc
cgggtcggttggtcctaaagggtcttcaggctcccccggtccccagggaccaccgggtcctgttggtctccagggcctgcgaggggag
gtgggacttccgggagtcaaaggagacaaaggtcctatgggtcctcctggtcctaaaggcgatcagggtgaaaagggaccgcgaggc
aagagagaggctggtcctcccggaagcggtgagaagggtgagcgaggggctgctggagagccaggtccacacggacctcccggtgt
tcccggcagcgtaggcccaaaagggtcgtctgggtccccggggccacaaggtccccctggacccgtagggttgcaaggcctacgggg
agaagtaggattaccgggtgtgaagggcgacaaaggtcctatgggaccccccggtcccaagggtgatcagggtgagaagggtccgag
gggtaaacgcgaagctggtccaccaggatctggcgaaaagggagaacgtggtgctgcgggagagcctgggccacacggtcccccgg
gagtacctgggagtgttgggccgaagggcagtagtggctcaccggggccgcaaggcccccccggaccggtaggactacaaggcttac
ggggggaggtaggtcttcctggggtgaagggggataaaggtcctatgggaccacccggcccaaaaggggaccagggagagaaagg
gccacgcggaaaaagggaggcaggcccaccggggtcgggtgaaaagggtgaacgcggagcggcgggggaaccggggcctcatg
ggccaccgggcgtgccaggttcggttggtcctaagggttcaagcggatctccaggcccgcagggacctcctggacccgttgggcttcag
ggactgcgtggcgaggtcggtctgcccggcgtcaagggagacaagggtcctatgggtcctccgggtccaaagggagatcagggtgag
aagggacccagagggaaaagagaagctggccctccggggtccggggagaagggggaacgtggggcagccggtgagccgggacct
cacggcccaccaggcgtgcctgggtccgttggcccaaaggggtcctcgggctcgccaggccctcagggaccaccgggtccagtcgg
attacaaggtttgcggggagaggttgggctccctggagttaaaggtgacaaaggccctatgggtcccccgggtcctaaaggcgaccaag
gcgaaaaaggaccgagagggaaacgtgaagcaggtccacctgggtcaggtgagaaaggcgagcgcggggccgccggggaaccg
gggccacacggtcctcctggcgtccccggcagcgtcggcccgaaaggaagttctggaagtccgggaccacagggtccgcccggtcct
gtagggctacaaggtttaagaggcgaagtgggactccctggcgtgaaaggtgacaaaggccccatgggaccaccaggtcctaaaggg
gatcagggagaaaaggggcctaggggtaaacgagaggcaggtccccccgggagcggagaaaagggtgagcgaggggccgccggt
gaaccaggcccgcatggtccaccaggcgtccccggatcagtcggaccaaaaggcagttctggctcacctggaccgcaaggtccgcct
ggacctgtaggacttcaaggcctgcgtggtgaagttgggttgcctggggttaagggggataagggtcctatgggtccccctgggccaaa
aggggatcaaggcgaaaagggtccccggggaaaaaggtaa

A DNA sequence for encoding 17S1NK-7 was shown in SEQ ID NO: 75:

gaagcaggtccgccagggagcggtgaaaaaggagaacgcggagcggcgggcgagccaggcccgcacgggccgcccggt
gttcccggctctgttggaccgaaaggtagttctggttcacccggacctcaaggtcctcctgggcctgttggacttcaggggcttagaggtg
aggtcggactccctggtgtgaaaggggataaagggcctatgggtcctcctgggccaaaaggggaccagggagagaaggggaagagg
gaggctggtcctccaggatcgggggaaaaaggagaacgtggagctgccggcgagccggggccacacggtccaccgggcgtccccg

ggtccgtgggacccaagggctcatcaggatcgccaggaccgcagggtcctccaggtcctgttgggttgcaggggttacgtggagaagt
gggtctccccggtgttaaaggtgacaagggtcccatgggaccccccggtcctaaaggagaccaaggagagaaaggtaaacgagaggc
cgggccacctgggtccggcgagaagggcgaacgtggtgccgctggcgagcccgggcctcacggtccaccgggagtgcccgggtca
gtaggcccgaaaggaagttccggaagtccaggtcctcaaggtcccccgggacctgtaggcctgcaagggttgcgtggtgaggtcggtc
tccctggggttaagggtgacaaaggtcctatgggtcctcccggacctaaaggggatcaaggggaaaaaggaaaacgagaggctggac
caccaggttctggcgaaaaaggtgagcgaggcgccgcgggcgaacccggcccgcatggccccccaggcgtaccaggctctgtgggt
ccgaagggctcgagcggttcacctggacctcaagggccacctggaccggttgggctacaaggcctacggggggaggtgggactacc
gggcgtcaaaggtgacaaaggcccgatgggaccaccaggccctaagggagatcagggcgagaagggtaagagagaagcaggacc
gccgggaagcggcgaaaaaggtgagcgcggagcagcaggggaacccgggccgcatggtccccctggcgtacctggtagcgttggt
cctaaagggtcgtcgggttccccaggtcctcagggtccccctggtcctgtcggtctgcaagggttaaggggggaagtaggattaccggg
cgtgaaaggagataaaggtcctatggggccacctggtcctaagggcgaccagggtgagaagggtaagcgcgaagctggcccgccgg
gtagtggtgagaaaggcgagcggggggcagcgggcgaaccaggtcctcatggtcctcctggtgtgccaggctccgtcggccctaagg
gatctagcggaagcccgggtccgcagggaccaccaggtcctgttggccttcaagggttgagaggtgaagtcggcttaccaggggtcaa
aggtgataaaggcccgatgggtccacctgggcctaagggggatcagggggagaagggaaagcgggaagccggtccccccggaagt
ggagaaaagggggagaggggggctgcaggtgaacctgggcctcacggaccaccgggtgttccaggatcagttggacccaagggtag
ttcgggatctcctggccctcaaggcccccctggtcctgtaggacttcaagggctgcggggggaggtaggtctccctggtgtcaaaggag
acaaaggaccgatgggtcctcctggtcctaagggagatcaaggagaaaaagggaagcgctaa

A DNA sequence for encoding 3A5D15D-5 was shown in SEQ ID NO: 76:

gatgggaacccggggtcggacggcctgccgggtcgcgacggctcaccaggtggcaaaggcgaccgtggtgagaatggatctc
cgggagcacccggtgcccctggacatcctggtccccccgggcctgtcggtcctgctggcaagtcgggtgatagaggagaatccgggc
ctgccggtcctgctggggcaccagggccagcaggaagccgtggggctcccggtcctcaaggtccccggggggacaagggtgagacc
ggagaacgcggagctgcgggaatcaaggggcatcgcggcttagagaaacgtgatggcaacccgggctctgacgggcttcccggtcg
ggacggcagccccggtgggaaaggtgatcgaggggaaaacggctcgcctggggctccaggtgcccccggccaccctggaccgcca
ggtcctgtcggccctgccggtaagagcggagatcgaggtgaatcaggacctgccggcccagccggtgctcctggtccagctggaagc
cgtggtgctcccggcccgcagggtcctcgtggcgataaaggcgagacgggtgaacggggcgcagccggaattaaaggacacagagg
tcttgaaaagagggatggaaacccaggctcagatggtctaccaggcagggacgggtcacccggaggaaagggtgatcggggcgaaa
acggctccccgggagcgccaggtgcccctgggcatcccgggccacccggaccggtgggtcccgcagggaaatcgggggatagggg
cgagtctggaccagcggggcctgcgggagcacctgggccagcaggtagtaggggggcgccgggtccgcaagggccacggggcga

caaaggggagactggcgagaggggggctgccggcatcaagggccaccgcggcctagaaaagagagacggcaatcctggttctgac
ggattgcctggtcgagatggttctcctggtggaaagggggatagaggggagaatggatctcctggcgccccaggtgcgccggggcatc
caggcccgcccgggccggttggaccagcgggtaaatcgggcgacaggggggaaagcggtcctgcaggaccagcgggcgcaccgg
ggcctgctgggagtagaggcgctccaggtccgcaaggtccccgaggggacaagggcgaaactggggagcgtggtgcggccggcat
aaagggacaccgaggactcgaaaaacgcgatggaaatcccggctccgacggactccctgggcgggatggcagtcccggaggtaagg
gagacagaggcgagaatggaagtcctggggcgcctggcgctccgggacatccagggccacccggaccggtaggaccggcaggaaa
atccggtgaccgcggcgagtcaggtcccgcaggaccagcgggtgcacctgggccggcgggtagtcgtggggctcccgggccgcag
ggtccgagaggtgataagggagagacaggagaacgcggtgccgccggcattaaagggcaccgaggcctggagaaacgataa

A DNA sequence for encoding 3A5D15E-5 was shown in SEQ ID NO: 77:

gaagccgatggtaacccaggaagtgatggcctcccggggcgcgacggttctcctggcggaaaaggcgatcgtggagaaaatg
ggtcgccaggtgccccgggagcgcccgggcatcctggtccaccaggcccggttggtcctgcagggaagagtggggaccgcggaga
gtcgggtccagcgggacctgcaggcgctcctggcccagccggcagtcgcggtgcccctgggccacagggtcccagaggagacaag
ggggaaaccggggaaagaggagctgctggaataaagggacaccgggggctagagaaaagggaggccgacgggaacccaggatca
gacggtctgccgggtcgggatgggagccctggcggaaaaggggatagaggcgaaaacggtagtccgggggcgcctggagcacccg
gccacccaggtcctcctggaccagttggtccggcaggtaaatccggggatcgcggcgagagcggccccgctggacctgcgggggcg
ccaggcccggcgggatcgcgaggtgcccctggtccgcaggggccgcgtggcgataagggtgagacaggtgagcggggagccgcg
ggcatcaaaggtcataggggcttagagaagcgtgaagcagatggaaatcccggatctgatggactgccgggacgagatggctcccctg
ggggcaaaggcgacagaggagaaaatggttcaccgggcgcaccaggtgctccaggccatccaggtccccccggcccagttggtcctg
ctggcaaaagtggagacagaggtgagtcgggacccgctgggcctgcgggagcaccaggtccggccggctcgcgcggtgctccagg
cccccagggtccgcggggcgataagggcgagacaggagagcggggagccgcaggtattaaagggcatcgaggtttggagaaacgt
gaagcagacggaaacccgggttcagatggactaccaggccgtgacggcagcccggggggaaagggtgaccgaggtgaaaacggtt
ctcctggagcccccggtgccccggggcatcctgggcctccaggccccgtaggacccgcaggcaaatctggggaccgaggggaaagc
ggccccgctggtcctgcgggcgcccccggtcccgcgggttctcgtggtgcacctggcccccaaggtcctaggggtgacaagggggaa
acgggggagcgtggggcagctgggataaaaggccaccggggtcttgaaaaacgcgaggcggatgggaatccgggaagcgacggct
taccgggacgggacggctcacccggggggaagggtgatcgcggggagaatggttcccccggtgctcctggggctcctgggcaccct
ggtccgcctggtcccgtcggaccggcagggaaatcaggggacagaggggaatccggacctgctggtcctgccggggctcccggtcct
gctggttctcgtggagcccctggtcctcaagggccaaggggtgataagggtgaaactggcgagaggggagctgcagggatcaagggc
caccgaggactcgaaaagaggtaa

A DNA sequence for encoding 3A5D15R-6 was shown in SEQ ID NO: 78:

gacggaaatcccggaagcgacggtcttcccggaagggatggctcccctggcgggaaaggtgatcgcggcgaaaacgggtctc
ccggcgcgcccggcgccccgggccaccctgggccacctggtccggttggtcctgctggcaaatcaggtgaccgtggggaatcgggg
ccagctggtcctgctggggcgccaggacccgcaggcagtcgtggcgcgcccgggcctcaagggccacgaggtgataagggagaga
ccggcgagaggggggcggctggaatcaaacgggacggtaatcccggttcagacggcctcccgggacgcgacggctccccgggggg
taaaggggatcgcggtgaaaacggatcgccaggtgcgccaggggcgcccggacaccctggtcctccggggcctgtcggcccggcag
gcaagtctggcgacaggggcgagtctggtcctgcaggccccgcaggtgctcctggcccagcaggatcacgtggcgcccccggacca
caggggccgagaggggataagggcgaaactggtgaacggggggcggctggaattaagagagatggtaatcccggtagtgatgggct
accgggacgagacggttcacccgggggtaagggagatcgaggtgagaatggcagtccaggcgcccctggtgctcctgggcatcccg
gcccacccggtccggttggacccgcgggcaaatccggggaccggggggagagtggcccggccggtcctgcgggtgccccgggacc
agcgggaagtaggggggctccggggccacaaggtcctcgtggagataagggcgaaacaggtgagagaggagctgcagggattaag
agggacggaaatcccgggtcggatgggttgcctggacgggatggtagccccggtggtaaaggagaccgaggcgaaaacggatctcc
cggtgcgcctggagccccaggtcacccggggccacctggtcctgtaggccctgcaggtaagtcgggtgaccgcggagagagcggac
cggccggtcctgcaggagcccctggacctgccggttcccggggagcaccaggcccccaaggaccgagaggtgacaagggggagac
aggtgagcgtggggctgctgggataaaaagagatggtaacccgggctctgatgggctgcctggaagggatgggagcccaggtggaaa
aggagatcggggtgagaacggttctcctggagccccgggagcccccggccatccggggccgccgggacctgtcggtcctgctggaa
aatcaggcgatcgtggggaatcaggaccagctgggcctgctggagcaccggggccagcagggagcagaggtgctcctggacctcag
ggaccgcgaggcgacaaaggggagacgggcgaacgcggtgcggcaggtataaaacgagacgggaacccaggctctgatggtttac
caggtcgtgacggctcgcctggaggcaagggagatcgcggtgaaaatggttcccccggtgctcctggagcgcctgggcatccggggc
ctccaggtccggtaggtccagcaggcaagagcggggacaggggagaatccggaccagctggtcctgccggagcgccaggccccgc
aggaagtcgaggggctcctggtccccaaggtcctcgtggcgacaaaggcgagaccggtgaacgcggcgctgcaggcatcaagagat
aa

A DNA sequence for encoding 3A5D15KR-6 was shown in SEQ ID NO: 79:

gacggtaaccctggaagtgacggattgccggggcgagacggttcacccggaggaaagggcgatcgcggcgaaaatgggtca
ccgggcgctcctggggcaccagggcatccgggtccccccggtcctgttgggccggcgggtaagtctggtgatcgaggcgagtctggc
cctgcgggtcctgctggtgcacctggacctgccggatcgagaggggctccagggccgcaaggacctaggggtgacaaaggcgaaac
gggtgagcgaggagccgccggaattaaggggaaaagggatggcaaccccgggtccgatggcctgccaggacgcgatgggtcgccc
ggaggcaagggtgacagaggggagaatggctctccaggtgcaccaggtgccccggggcaccccggtccgcctggaccagttggacc

ggcgggaaagtctggagatcgcggggagagtggaccggctggtcctgcaggggcaccgggtcccgctgggtctcgcggagcgcctg
gcccccaaggacccagaggggataagggcgaaactggagagcgtggcgcggcaggaataaagggcaaacgtgatggcaatccagg
gtcggacggacttcctggccgggacggttccccagggggaaagggtgacagaggcgaaaacggctcgcccggtgcgccaggggcg
cctggtcatccggggcctccgggtccagtgggaccagccggaaaaagcggggatcggggggaatctggaccggctggaccagccg
gcgcaccaggccccgccggatcgaggggggcgcccggaccgcagggtcctagaggggataaaggtgagaccggcgagaggggc
gcagctgggataaagggtaagcgggacggtaaccccggctccgacggtctcccaggacgagacgggtcaccgggtggtaagggaga
ccgaggcgaaaatgggtcacctggtgcacccggcgcccctggacaccccggtcctcctggaccagtgggacccgcagggaagagcg
gtgacagaggggagagtgggcctgcaggaccagctggcgctccgggaccggccggaagtcggggtgctcccggcccccaagggcc
tagaggggataaaggtgagaccggggaacgtggcgcggccggtatcaagggcaaacgcgacggcaacccaggttcggatggactac
cagggcgagatggttcacctggcggtaaaggcgatcggggggaaaatggcagccccggtgctcctggagcaccgggtcatcctggtc
cgcctggtcctgttggaccggctgggaaatccggtgaccgcggagaatccggcccggccggtccggctggagcccctgggccggctg
gcagtcgtggcgcgcctggcccacagggtccgcgtggagataaaggggaaacaggtgagaggggcgcagcgggaatcaagggga
aacgggatggcaacccggggagcgacgggttacccgggcgagacggttccccaggaggcaagggcgatcgtggcgagaatggtag
ccccggtgctccaggagcacctggccacccagggccaccaggaccggtaggacccgctggtaaatcaggcgatcggggtgagagtg
gccctgcgggacccgcgggcgcccccggacctgccgggagccgcggtgctcctggaccacaaggtcctaggggcgacaaaggtga
aacaggggaacgtggcgctgcggggattaaaggaaaaaggtaa

A DNA sequence for encoding 3A5D15EKR-6 was shown in SEQ ID NO: 80:

gacggaaaccctggctcggatggtcttccaggacgggatggcagtcccggcggaaaaggcgaccggggagaaaacggttcac
ccggtgctcctggagcaccaggacatcctgggccacccggccccgttggtccggcggggaaatcaggcgacaggggcgaaagtggg
cctgccgggcctgcgggagccccgggtccggcaggtagccgaggggcaccaggccctcaagggccacgaggtgacaagggagaa
acaggcgaaaggggtgctgctggaatcgagaaacgtgatgggaatcccgggtcagacggcctaccgggacgggatgggtcccccgg
cggcaaaggggaccgaggggagaatggttcgcctggtgccccaggcgcgccgggtcatcctggtcccccgggacccgtcggtcctg
ctggcaaaagtggggatcgtggagaaagtggacctgccggacctgccggggctcctggtcctgctggatcgcgcggcgcaccaggac
ctcaaggtcctagaggagacaaaggggagacgggagaaagaggagctgcagggatagaaaaacgggatgggaatcctggaagcga
tgggttaccaggcagggacggaagccccggcgggaagggggacaggggggagaatggttcaccaggtgcgcccggagccccggg
acatcccgggccgcctggtccggtagggcctgcaggtaaatccggtgatagaggagaatccggacctgcggggccagccggagcgc
cggggccggcgggtagcaggggcgcaccgggacctcaagggccacggggtgataagggagaaaccggtgagaggggcgcggct
ggaattgagaagcgtgatggtaaccctgggtccgatggactgcccggccgagacggctcgcctggcggcaagggcgaccgcggcga

gaatgggtctcccggtgctcctggtgctcctggacaccctggtcctcccggtccggtgggtcctgccggcaagagtggggacagaggg
gaatcggggccggctggaccggcgggtgcaccgggtcccgctggctcacgaggtgcgccggggccacaaggcccacgcggcgata
agggggagactggtgagcgtggagctgcaggaatcgagaagcgcgatggcaacccaggtagcgacggattgccaggtcgtgacgga
tctcctgggggtaagggtgatcgaggagaaaatggctcaccgggtgctcctggggcgcccggccacccgggacctcctggtcctgtag
gtcccgccggcaaatcgggcgaccggggtgagtctgggcctgcaggcccggccggtgccccaggtccagcagggtctcgtggcgcg
cccggtccccagggtcctcgtggggataagggcgagacgggagaaagaggtgcggcaggcatagaaaagcgcgatgggaaccctg
gatctgatgggctcccagggagagacggaagccccggcggaaaaggtgatcggggcgaaaacggaagtccaggtgcgcccggagc
acccggccacccaggtccacccggaccggttggtcccgctgggaaatctggtgacagaggggagtccgggccagcagggccggcc
ggcgcaccaggaccagctggttctcgtggtgcccctggacctcaaggtcctaggggggacaaaggcgagaccggggaacgcggtgc
ggctgggattgagaagcgctaa

A DNA sequence for encoding 3A5D15G-6 was shown in SEQ ID NO: 81:

gacggcaatcctggatctgacgggctgcctggccgtgacgggagcccagggggtaaaggcgaccgcggagaaaatggtagc
cctggcgctcctggtgctcccggtcatcctggtcccccgggaccagtggggccggctggaaaaagcggtgaccgtggtgaatctggcc
cagcgggacctgcgggagcccctggtcctgccggttctcgtggcgcacccggacctcaaggtccgcggggtgataaaggcgagacag
gggagcgaggtgccgcaggcattggaaaaagggacggcaatccgggttcagacggcttgccaggaagggatggctcgccgggtggt
aagggagacaggggagaaaacggttccccaggtgctcctggcgcacctggccaccctggtcctccggggcctgtaggaccagctggc
aagagtggagatcgcggagagtctgggcctgcgggtccggcaggtgctcccggacccgccggatcacgcggtgctcctggaccgca
aggtccgcggggtgacaagggagagactggggagaggggtgcggcgggtattggtaagcgtgatggcaatccaggcagcgacggtc
taccggggcgggatgggagtcccggtgggaagggggataggggtgagaacggctctccaggggctcccggtgcccctgggcatcct
ggtcctcctgggcctgtgggtcctgccggcaagtcaggggatcgaggcgagagtggtcctgctggtcctgctggggcgccaggaccag
cggggtctcgaggcgcccctggcccgcagggtccaagaggtgataagggggagaccggggaacgaggagccgctgggataggga
agcgcgacggaaatcccgggagtgatgggcttccaggccgtgatggaagtcccggagggaagggagatcggggcgaaaacggttct
cccggtgctcctggagcgcccggacaccccggcccaccaggccctgttgggccggccgggaaaagcggcgatcgtggcgaatctgg
tcctgcgggtccagctggtgctcctgggccggcaggttctcgtggtgcacccggaccgcagggacccagaggagataaaggggaaac
gggtgagcgaggggcagcaggtatcgggaaaagggacggtaaccctggctccgatggcttaccggggcgagacggatcgccgggg
ggtaaaggagaccggggagagaacggttcaccaggcgcacccggcgctccagggcatcctgggccacccggcccagttggtcctgc
tggtaaatcgggggaccgaggcgaatcaggacctgcaggccctgcgggtgcccccggccccgctgggagtcgtggtgcacctgggc
cacaaggcccgcgcggcgacaagggggagacgggagagcgcggtgccgctggaatcggcaagagagatggtaaccccgggagc

gacggactcccaggcagagatgggtccccgggcggcaaaggagacagaggtgaaaatggatcacctggtgcgcctggtgctcctgga
cacccaggaccgccaggtccagtcggtcctgccgggaaatcgggtgatcgtggtgaatccgggcctgcaggtcccgctggagcgccg
ggtcccgcagggtcgaggggcgcgccaggtccacaaggtcctcggggcgataagggcgaaacaggtgaaagaggcgcggccggc
ataggaaaaagataa

The above DNA sequences SEQ ID NO: 71 to SEQ ID NO: 81 were synthesized by Nanjing GenScript Biotechnology Co., Ltd. A synthesized gene was cloned into a pPIC9K vector at position 1203 bp (i.e., the α-factor secretion signal/cleavage site 1203 between a checking sequence AAAGAAGAAGGGGTATCTCTCGAGAAAAGA) and an enzyme cleavage site Not I. Recombinant expression vectors pPIC9K-3A5D29N-9, pPIC9K-17S28-8, pPIC9K-17S1N6-6, pPIC9K-17S1N7-7, pPIC9K-17S1NK-7, pPIC9K-3A5D15D-5, pPIC9K-3A5D15E-5, pPIC9K-3A5D15G-6, pPIC9K-3A5D15EKR-6, pPIC9K-3A5D15KR-6 and pPIC9K-3A5D15R-6 were obtained. Other expression vectors suitable for Pichia pastoris, such as pPICZαB and pFLDα, exhibited similar effects to pPIC9K.

The above recombinant expression vector plasmids (pPIC9K-3A5D29N-9, pPIC9K-17S28-8, pPIC9K-17S1N6-6, pPIC9K-17S1N7-7, pPIC9K-17S1NK-7, pPIC9K-3A5D15D-5, pPIC9K-3A5D15E-5, pPIC9K-3A5D15G-6, pPIC9K-3A5D15EKR-6, pPIC9K-3A5D15KR-6, and pPIC9K-3A5D15R-6), 10 µg each, were digested overnight at 37°C with SacI (purchased from TaKara (Dalian)), and linearized. The linearized plasmids were then purified using a PCR Purification Kit (purchased from Sangon Biotech (Shanghai) Co., Ltd.), and the final volume was adjusted to approximately 10 µL.

The linearized plasmids were electrotransformed into competent cells of the four chassis engineered strains (HCPB-PPKEX2 strain with the deposit number of CGMCC No. 25815; RCPB-PPKEX2 strain with the deposit number of CGMCC No. 25817; HCPB-SCKEX2 strain with the deposit number of CGMCC No. 25816; RCPB-SCKEX2 strain with the deposit number of CGMCC No. 25818). An electrotransformed strain culture was applied onto a MD plate. One MD plate was applied at every 100-200 µL, kept at a room temperature for 10 minutes, then inverted and incubated at 30°C for 2-5 days until a single colony (a positive transformant) appeared.

2 mL sterile double-distilled water was added to the surface of a MD plate. A His⁺ transformant was gently scraped from the surface of the plate using a sterile triangular spreader and transferred into a 50 mL centrifuge tube. A strain suspension was diluted with sterile double-distilled water. 105 cells were applied onto a YPD plate containing 0.5 mg/mL G418, placed upside down and incubated at 30°C for 3-4 days until a single colony appeared. The colony was picked from a YPD plate into a sterile 96-well plate (200 µL YPD/well), mixed thoroughly, and incubated at 30°C for 48 hours. A strain culture was mixed evenly in each well. 10 µL of the strain culture was put onto one new sterile 96-well plate, and incubated at 30°C for 24 hours, and then this procedure was repeated once more. After 24 hours, 1 µL of the strain culture was taken out from a third 96-well plate to be dripped onto the YPD plates containing 1.0 mg/mL and 4 mg/mL G418, respectively, and continued incubating at 30°C for 96-120 hours. If Pichia pastoris transformants grew on plates containing a high concentration of G418, it indicates that the transformant could efficiently express an exogenous gene. A further screening step yielded a highly efficient recombinant yeast engineered strain.

Subsequently, the chassis cell engineered strain expressing HCPB-PPKEX2 (the deposit number of CGMCC No. 25815) and the chassis cell engineered strain expressing RCPB-PPKEX2 (the deposit number of CGMCC No. 25817) were taken as expression host cells for description.

The repeat tandem expression system of the present invention constructed the plurality of engineered strains expressing the recombinant small-molecule collagen. Engineered strains constructed using five chassis cell engineered strains expressing HCPB-PPKEX2 as expression host cells were deposited at China General Microbiological Culture Collection Center with the corresponding strain deposit numbers:
A strain expressing the recombinant small-molecule collagen 3A5D29N has a deposit number: CGMCC No.25819
A strain expressing the recombinant small-molecule collagen 17S28 has a deposit number: CGMCC No.25821
A strain expressing the recombinant small-molecule collagen 3A5D15D has a deposit number: CGMCC No.25827
A strain expressing the recombinant small-molecule collagen 17S1N6 has a deposit number: CGMCC No.25829
A strain expressing the recombinant small-molecule collagen 3A5D15E has a deposit number: CGMCC No.25828.

The engineered strains constructed using two chassis cell engineered strains expressing RCPB-PPKEX2 as the expression host cells were deposited at China General Microbiological Culture Collection Center with the corresponding strain deposit numbers
A strain expressing the recombinant small-molecule collagen 3A5D29N has a deposit number: CGMCC No.25820;
A strain expressing the recombinant small-molecule collagen 17S28 has a deposit number: CGMCC No.25822

The deposited address for the above engineered strains was as follows: No. 3, Compound 1, Beichen West Road, Chaoyang District, Beijing. The deposited date for all was: September 26, 2022. Classification designation for all strains was *Komagataella phaffii.*

A single colony grown on a high-concentration G418 plate were selected and transferred to a 100 mL conical flask containing a 10 mL BMGY medium and cultured at 28-30°C and 220 rpm until OD600 reached 2-6 (16-18 h). The single colony was centrifuged at 1500-3000 g for 5 minutes at a room temperature. A strain was collected and resuspended in a BMMY medium to an OD600 of approximately 2, and continued growth on a shaker at 28-30°C and 220 rpm for 3 days. A 100% methanol was added to the medium every 24 hours to maintain a final concentration of 1.0%. 1 mL of a strain culture sample was collected at time points (sampling every 24 hours after induction), placed into a 1.5 mL EP tube, and centrifuged at 12,000 rpm for 2-3 minutes. A supernatant was collected, added with a 5× loading buffer (250 mM Tris-HCl, pH 6.8; 10% SDS; 0.5% bromophenol blue; 50% glycerol; 5% β-mercaptoethanol), heated in a 100°C metal bath for 10 minutes and subject to SDS-PAGE inspection.

SDS-PAGE detection results were shown in FIGS 16-19. pPIC9K-3A5D29N-9, pPIC9K-17S28-8, pPIC9K-17S1N6-6, pPIC9K-17S1N7-7, pPIC9K-17S1NK-7, pPIC9K-3A5D15D-5, pPIC9K-3A5D15E-5, pPIC9K-3A5D15G-6, pPIC9K-3A5D15EKR-6, pPIC9K-3A5D15KR-6, and pPIC9K-3A5D15R-6 successfully and efficiently expressed the recombinant small-molecule collagen of corresponding sizes in two chassis cell engineered strains and were effectively secreted and expressed into the supernatant, with electrophoresis showing a single band. This indicated the achievement of the present invention's design objective for the tandem repeat amino acid sequence of a small-molecule collagen. Specifically, a macromolecular protein expressing hundreds or more amino acid sequences (a tandem repeat structure of a small-molecule collagen monomer) could ultimately express and obtain the recombinant small-molecule collagen with identical sequences and a single band (the collagen exhibited a certain degree of electrophoretic migration delay during electrophoresis, resulting in an apparent molecular weight that appeared larger) in the chassis cell engineered strain.

Lyophilized products of small-molecule collagens 3A5D29N, 17S28, 3A5D15D, and 17S1N6 were enzymatically digested with trypsin. An enzymatically-digested-trypsin peptide fragment of the recombinant collagen was detected by Nano-HPLC-MS/MS (performed by Suzhou ProtTech Biotechnology Co., Ltd.), and the detected peptide fragment was subjected to sequence alignment (Uniprot database). As shown in FIGS 20 and 21, mass spectrometry inspection results indicated that the peptide fragments detected by the enzymolysis of the recombinant small-molecule collagens 3A5D29N, 17S28, 3A5D15D, and 17S1N6 and sequences covered by the peptide fragments all belonged to the relevant regions of the human collagen sequences selected during the design of the amino acid sequence, confirming successful expression of the recombinant small-molecule collagens.

Lyophilized products of 3A5D29N and 17S28 were subjected to LC-MS analysis (capillary HPLC: Thermo Fisher Scientific Ultimate 3000; electrospray ionization-quadrupole time-of-flight mass spectrometer: AB SCIEX TripleTOF 5600 Mass Spectrometer; chromatographic column: ACQUITY UPLC Protein BEH C4 Column) to obtain deconvoluted molecular weights thereof (contracted to Beijing Biotech Pack Scientific Co, Ltd.). Partial results were shown in FIG. 22 (deconvoluted molecular weight values were displayed with one decimal place, rounded as appropriate). 3A5D29N had a theoretical molecular weight of 4988.35 Da and a measured deconvoluted molecular weight of 4987.5 Da. 17S28 had a theoretical molecular weight of 5861.60 Da and a deconvoluted molecular weight of 5833.92 Da. Considering potential glycosylation modification during protein expression, and detection errors, the measured deconvoluted molecular weights of the recombinant small-molecule collagens 3A5D29N and 17S28 were essentially consistent with theoretical values.

### Effect of the chassis cell engineered strain and validation of the expression system:

The chassis cell engineered strain constructed in the present invention not only cleaved a long protein sequence into individual short collagen monomers but also performed a crucial additional function. That is, the chassis cell engineered strain cleaved the non-collagen amino acid sequences at both amino and carboxyl termini of these single short cleaved small-molecule collagen monomers. With expression examples of 3A5D29N-9 and 17S28-8 in the chassis cell engineered strains RCPB-PPKEX2 and HCPB-PPKEX2, the expressed recombinant small-molecule collagens 3A5D29N and 17S28 were detected at the amino and carboxyl termini to verify the synergistic function of Kex2, Ste 13, and CPB enzymes in the chassis cell engineered strains. Beijing Biotech Pack Scientific Co., Ltd. was commissioned to sequence and validate the N-terminus (amino-terminus) and the C-terminus (carboxy-terminus) of a lyophilized product of the expression recombinant small-molecule collagens in the chassis cell engineered strains HCPB-PPKEX2 and RCPB-PPKEX2 for 3A5D29N-9 and 17S28-8, respectively and analyze the full sequence of the protein based on LC-MS/MS.

N-Terminus sequencing: A N-terminus sequence of a sample was analyzed using a Shimadzu fully automated protein/polypeptide sequencer (PPSQ-33A) via an Edman degradation method. An appropriate amount of the lyophilized product of the recombinant small-molecule collagen sample was dissolved. The sample solution was spotted onto a PVDF membrane. The membrane was placed into a reactor, which was assembled and positioned in the fixed position of an instrument. Settings were configured via PPSQ-30 Analysis software: Sample name, sample number, test cycle number, and method file selection. After configuration, a test began. Standard peaks of raw data generated by the PPSQ-33A and a map were identified by PPSQ-30 DataProcessing software, and a corresponding map was exported. After data analysis, a protein N-terminus sequence was determined.

Results indicated that the N-terminus sequences of the expression recombinant small-molecule collagens in the chassis cell engineered strains HCPB-PPKEX2 and RCPB-PPKEX2 for 3A5D29N-9 were all NH₂-Gly-Lys-Ser-Gly-Asp-Arg-Gly (GKSGDRG), which was consistent with the N-terminus amino acid sequence of a theoretical sequence
(GKSGDRGESGPAGPAGAPGPAGSRGAPGPQGPRGDKGETGERGAAGIKGHRGLE ). It was detected that the N-terminus sequences of the expression recombinant small-molecule collagens in the chassis cell engineered strains HCPB-PPKEX2 and RCPB-PPKEX2 for 17S28-8 were all NH2-Gly-Val-Pro-Gly-Ser-Val-Gly (GVPGSVG), which was consistent with the N-terminus amino acid sequence of a theoretical sequence. 
(GVPGSVGPKGSSGSPGPQGPPGPVGLQGLRGEVGLPGVKGDKGPMGPPGPKGDQ
GEK GPRGLE).
 C-terminus sequencing: An appropriate amount of lyophilized recombinant small-molecule collagen sample was dissolved and subjected to enzymolysis of trypsin and pepsin. A treated sample was then analyzed by liquid chromatography-mass spectrometry (LC-MS/MS), obtaining a raw mass spectrometry data file. The file was analyzed using Byonic software for data matching. Mass spectrometric data were subject to database searching to obtain identification results. A secondary mass spectrum for the detected C-terminus peptide fragment was shown in FIGS 23 and 24 below.

Results indicate: (1) The detected C-terminus sequences of the recombinant small-molecule collagen 3A5D29N-9 expressed in both the chassis cell engineered strains HCPB-PPKEX2 and the RCPB-PPKEX2 were both listed as GHRGLE, which was consistent with the C-terminus of the theoretical sequence
(GKSGDRGESGPAGPAGAPGPAGSRGAPGPQGPRGDKGETGERGAAGIKGHRGLE ). (2) A detected C-terminus sequence of a recombinant small-molecule collagen of 17S28-8 in the chassis cell engineered strain HCPB-PPKEX2 was VGLPGVKGDKGPMGPPGPKGDQGEKGPRGLE. A detected C-terminus sequence of the expression recombinant small-molecule collagen of 17S28-8 in the chassis cell engineered strain RCPB-PPKEX2 was KGPMGPPGPKGDQGEKGPRGLE, which was both consistent with C-terminus of a theoretical sequence

(GVPGSVGPKGSSGSPGPQGPPGPVGLQGLRGEVGLPGVKGDKGPMGPPGPKGDQ
GEK GPRGLE).

LC-MS/MS-based full-sequence protein analysis: Furthermore, a sample of a lyophilized product of the recombinant small-molecule collagen was subjected to enzymolysis of trypsin, chymotrypsin, pepsin, trypsin & Glu-C protease, and trypsin & Asp-N protease. The treated samples were then analyzed via liquid chromatography-mass spectrometry (LC-MS/MS) to obtain a raw file of an initial mass spectrometry result. The raw file was analyzed using Byonic software for matching data, ultimately obtaining the full-sequence sequencing verification results. Analysis of comprehensive detection results indicated that coverage rates of the amino acid sequences of the samples of the lyophilized products of the expression recombinant small-molecule collagens of 3A5D29N-9 and 17S28-8 in the chassis cell engineered strains HCPB-PPKEX2 and RCPB-PPKEX2 reached 100%, respectively. The amino acid sequence of the small-molecule collagen expressed by the present invention was identical to that of the target small-molecule collagen.

In this embodiment, when 3A5D29N-9 entered the protein secretion pathway constituted by the endoplasmic reticulum and the trans-Golgi network via transcription and translation, the Kex2 enzyme cleaved 3A5D29N-9 into identical small protein sequence monomers:
EAGKSGDRGESGPAGPAGAPGPAGSRGAPGPQGPRGDKGETGERGAAGIKGHRG LEKR. The two EA amino acids at the amino-termini were cleaved and removed by the Ste 13 protease, while the two amino acids at the carboxy-terminus (KR) were cleaved and removed by the CPB protease. A final secreted product was a fragment of 54-amino acid recombinant small collagen 3ASD29N:
GKSGDRGESGPAGPAGAPGPAGSRGAPGPQGPRGDKGETGERGAAGIKGHRGLE_{∘}

In this embodiment, when 17S28-8 entered the protein secretion pathway constituted by the endoplasmic reticulum and the trans-Golgi network via transcription and translation, the Kex2 enzyme cleaved 17S28-8 into an identical small protein sequence monomer:
EAGVPGSVGPKGSSGSPGPQGPPGPVGLQGLRGEVGLPGVKGDKGPMGPPGPKG DQGEK GPRGLEKR. The two EA amino acids at the amino-termini were cleaved and removed by the Ste 13 protease, while the two amino acids at the carboxy-terminus (KR) were cleaved and removed by the CPB protease. A final secreted product was a fragment of 63-amino acid recombinant small collagen 17S28:

GVPGSVGPKGSSGSPGPQGPPGPVGLQGLRGEVGLPGVKGDKGPMGPPGPKGDQ
GEKGPRGLE。

The accuracy of the N- and C-terminus amino acid sequences directly indicated whether the synthetic metabolic pathway for the CPB enzyme in the chassis cell engineered strain was effectively established, whether the synthetic metabolic pathway possessed biological activity after establishment, and how the synthetic metabolic pathway interacted synergistically with the yeast's own Kex2 enzyme and Ste13 protease. The results of this example demonstrated that the CPB enzyme (whether a human or rat CPB) in the chassis cell engineered strain successfully established a synthetic metabolic pathway in Pichia pastoris, exhibited normal biological enzyme activity, and synergistically interacted with the yeast's own Kex2 enzyme and Ste13 protease. After successful cleavage of the long-sequence large-molecule recombinant collagen designed with specific amino acid during expression, the non-collagen sequences at both termini were removed, secreting and obtaining the recombinant small-molecule collagen.

### Embodiment 4: 500L-scale production and validation of a small-molecule collagen

A small fermentation tank or a shake flask could not accurately reflecte actual large-scale industrial production conditions. Only a 500L-scale fermentation tank had the potential for scale-up and characterized actual large-scale industrial production conditions. The present invention optimized the fermentation process of a high-expression engineered strain to verify the yield of each engineered strain in a 500L fermentation tank.

Two recombinant small-molecule collagens 3A5D2NT and 3A5D29N exhibited highly similar lengths, sequence homology, and identical purification methods. The present invention specifically compared small-molecule collagen expression using these two strains.

As shown in the table below, after fermentation parameter optimization, the corresponding indicators of both engineered strains (the engineered strain expressing 3A5D29N had CGMCC No. 25819 and the engineered strain expressing 3A5D2NT had CGMCC No. 25812) were largely consistent. However, significant differences were observed in a collagen expression amount in the fermentation supernatant (a UV method, a UV protein quantification empirical formula: C(mg/mL) = 0.144 × (A215 - A225) for protein concentration measurement) and the yield of the final purified lyophilized product. The expression amount in the fermentation supernatant of 3A5D29N was generally about twice that of 3A5D2NT (the UV method was highly susceptible to pigment interference in the fermentation broth, resulting in a lower ratio), and the final yield of the purified lyophilized product was 4-5 times that of 3A5D2NT (direct weight measurement). During an optimal fermentation batch, the protein yield in the fermentation supernatant (the UV method) for 3A5D29N was twice that of 3ASD2NT. The SDS-PAGE of the fermentation supernatant was shown in FIG. 25. Comparing the purified lyophilized product yields obtained from the same volume of the fermentation broth, the yield of 3A5D29N was nearly five times that of 3A5D2NT (direct weighing), representing a substantial increase in yield. In other repeat tandem expression systems of the recombinant small-molecule collagen, the yield of the recombinant small-molecule collagens was also 4-6 times that of the single-sequence expression of the small-molecule collagen.

| Reco mbinant Small-Mo lecule Collagen | Multi-batch Fermentation and Purification Indicators | | | | | Optimal Batch (Highest Lyophilized Yield) | | |
|---|---|---|---|---|---|---|---|---|
| | In ductio n Time | C ell Densi ty (OD_{60 0}) | C ell Wet Weigh t | Expres sion Amount of Fermentatio n Supernatant (the UV Method) | Pur ified Lyophili zed Product Yield | Fer mentation Supernata nt Volume | Supe rnatant Protein Yield (the UV Method) | Puri fied Lyophiliz ed Product Yield |
| 3A5 | 4 | 2 | 3 | 3.6-4.7 | 50~ | 190 | 888. | 60 g |
| D2NT | 0-48 h | 90-35 0 | 20-35 0 g/L | g/L | 60 g | L | 3 g | |
| 3A5 D29N | 4 0-43 h | 3 00-36 4 | 3 20-35 1 g/L | 8.1-9.0 g/L | 257 ~295 g | 190 L | 1682 .1 g | 295 g |

### Embodiment 5: Biological activity and transdermal absorption detection of the recombinant small-molecule collagen

### (1) Cell adhesion activity detection

References for Detection Method for Cell Adhesion Activity of Recombinant Small-Molecule Collagen: Juming Yao, Satoshi Yanagisawa, Tetsuo Asakura. Design, Expression and Characterization of Collagen-Like Proteins Based on the Cell Adhesive and Crosslinking Sequences Derived from natural Collagens, J Biochem. 136, 643-649 (2004).

Specific implementation method: A NIH/3T3 cell was cultured under standard conditions (purchased from Cell Bank of the Chinese Academy of Sciences, with catalog number of GNM6. Culture and passage methods followed a cell instruction). Lyophilized products of recombinant small-molecule collagens 3A5D29N, 3A5D2NT, 3A5D1NT, 17S1NNT, 17S3NT, and 17S28 were used. A recombinant type-III collagen (Patent Application No. CN201310033299.6, Date of patent: May 15, 2013, Molecular Weight: 43.6 kDa) produced by our company (Jiangsu Trautec Medical Technology Co., Ltd.) was compared to a recombinant type-XVII collagen lyophilized sponge (Patent Application No. CN202110520499.9, Publication Date: July 30, 2021, Molecular Weight: 23.8 kDa). A natural human collagen (Sigma, Catalog No. C7774) was compared to a bovine serum albumin solution (BSA, purchased from Sangon Biotech (Shanghai) Co., Ltd.) (in ultrapure water or 1M HCl solution). Protein concentration was determined using the UV protein quantification empirical formula: C(mg/mL) = 0.144 × (A215 - A225). Then, the protein was diluted to 0.5 mg/mL with PBS (pH 7.4).

100 µL of various protein solutions and blank PBS solution were added to a 96-well cell culture plate, and set aside 60 minutes at room temperature. Then, 105 NIH/ 3T3 cells with good culture status were added to each well, and incubated at 37°C and 5% CO₂ for 60 minutes. The cells were cleaned in the wells 4 times with PBS. An absorbance value at OD492nm was detected using a LDH detection kit (Roche, 04744926001). Data were analyzed and subject to significant differential analysis (SPSS 22 software, Duncan's method, P<0.05).

The absorbance value at OD492nm characterized the cell adhesion activity of a collagen sample: higher adhesion activity indicated more cells adhering to the protein.The collagen could rapidly facilitate the attachment of the cell to a wall or the adhesion of the cell to an extracellular matrix, thereby promoting the establishment of a more favorable extracellular environment. As shown in FIG. 26, the cell adhesion activity of recombinant small-molecule collagens 3A5D29N, 3A5D2NT, 3A5D1NT, 17S1NNT, 17S3NT, and 17S28 was comparable to or superior to that of a natural human collagen. At equivalent concentrations, the cell adhesion activity of the three recombinant small-molecule collagens 3A5D29N, 3A5D2NT, and 3A5D1NT significantly outperformed that of a larger-molecular-weight recombinant type-III collagen. At equivalent concentrations, the cell adhesion activity of 17S1NNT, 17S3NT, and 17S28 showed no significant difference compared to the larger-molecular-weight recombinant type-XVII collagen. This indicated that although the recombinant small-molecule collagen possessed fewer amino acid residues and the shorter sequence, designed amino acid sequences thereof achieved a balance between low molecular weight (i.e., short sequence) and preservation of biological activity (i.e., retention of a specific biologically active site sequence).

### (2) Cell proliferation detection

The cell proliferation activity detection for the recombinant small-molecule collagen referenced the "Method for Determining Biological Activity of Human Epidermal Growth Factor" (General Rules 3528) from the 2020 edition of the Pharmacopoeia of the People's Republic of China.

### Brief of implementation method:

Lyophilized products of recombinant small-molecule collagens 3A5D29N and 17S28 were dissolved in pure water, adjusted to have pH 7.2-7.4, sterilized by filtration through a 0.22µm membrane, and diluted in a sterile maintenance medium (0.5% FBS + 95.5% DMEM(H)) to concentrations of 1 mg/mL, 0.5 mg/mL, 0.25 mg/mL, and 0.125 mg/mL. hEGF (purchased from Lonza) was used as a positive control at concentrations of 1 ng/mL, 0.5 ng/mL, 0.25 ng/mL, and 0.125 ng/mL to maintain a medium as a negative control. A HaCat cell strain (purchased from Cell Bank of Chinese Academy of Sciences, with catalog number of SCSP-5091) was cultured in a complete culture solution (88% DMEM + 10% FBS + 1% glutamine + 1% sodium pyruvate) at 37°C and 5% CO₂, maintained at a cell density of 1.0×10⁵ to 5.0×10⁵ cells per mL, and detected for biological activity within 24-36 hours after passage. The culture solution was discarded from a culture flask. Cells were digested, collected, prepared as a cell suspension with the complete culture solution to a concentration of 5.0 × 10⁴ cells/mL, inoculated into a 96-well cell culture plate at 100 µL per well, and cultured at 37°C under 5% CO₂. After 24 hours, the culture solution was replaced with a maintenance culture solution and incubated at 37°C, 5% CO₂ for 24 hours. The maintenance culture solution was discarded from a prepared cell culture plate, added with 100 µL of a control sample solution and a test sample solution to each well, and cultured at 37°C and 5% CO₂ for 24-72 hours. A 96-well plate was removed and observed for cell morphology under a microscope. A supernatant was discarded. 50 µL of MTT solution (purchased from Beyotime Biotech Inc. (Shanghai), prepared in DPBS at a concentration of 1 mg/mL) to each well and cultured at 37°C in a 5% CO₂ incubator. After 4 hours, the supernatant was removed. 100 µL of isopropanol was added to each well to dissolve a crystal. An absorbance value was measured at 570 nm of a wavelength using a microplate reader with 650 nm as a reference wavelength. Detection results were recorded.

As shown in FIG. 27, hEGF, serving as a positive control, promoted HaCat cell proliferation. Recombinant small-molecule collagens 3A5D29N and 17S28 also promoted HaCat cell proliferation at concentrations ranging from 0.125 to 1 mg/mL. Particularly, the proliferation-promoting activity of 17S28 exhibited an increasing trend with rising sample concentration. At a higher concentration, 17S28 demonstrated proliferation-promoting effects comparable to those of 0.5 ng/mL hEGF.

### (3) Transdermal absorption detection

With a full-thickness skin model as a vector, a sample was labeled with FITC fluorescein and applied uniformly to a model surface via surface administration. A sample was applied evenly to the surface of the model. A skin penetration behavior was assessed by counting a fluorescence intensity in a fluorescence section and calculating diffusion percentage using a fluorescence microplate to measure a collection fluid. Detection was conducted by Shaanxi BioCell General Testing Co., Ltd.

### Brief of implementation method:

A recombinant small-molecule collagen 3A5D29N, a recombinant type-III collagen (Jiangsu Trautec Medical Technology Co., Ltd., Patent Application No. CN201310033299.6, Date of patent: May 15, 2013, Molecular Weight: 43.6 kDa), a hydrolyzed hyaluronic acid (molecular weight ≤5000 Da), and a fish skin collagen (Nitta Gelatin Inc. Maringen SP03(PF)) were labeled with FITC (fluorescein isothiocyanate). Unconjugated FITC was purified and removed via a molecular sieve. FulKutis^{®} full-thickness skin model tissue (Guangdong BioCell Biotechnology Co., Ltd.) was transferred to a 6-well plate, with 2 mL of a 3D full-thickness skin model culture solution added to each well.

Sample preparations at different concentrations were performed according to a test group. A sample group received surface administration with an administration volume of 20 µL. After being cultured for an appropriate duration, a residual test substance on the surface of a model can be washed off using a sterile PBS solution wash bottle. Residual liquid inside and outside the model was gently wiped away with a sterile cotton swab. The model was then ring-sectioned and immersed in a 4% paraformaldehyde solution for fixation (fixation time ≥ 24 h). After the solution was frozen and sectioned, fluorescence imaging was performed. An accumulated IOD (Intensity of Optical Density) value for a target substance in a picture was statistically analyzed using IPP software. Intergroup comparisons were conducted using a two-tailed t-test. A culture solution was collected for fluorescence IOD detection. Based on fluorescence standard curves for different samples, the amount of the sample permeating into the culture solution at the 12th hour was calculated. The diffusion percentage = Sample amount / Theoretical sample content × 100%, i.e., cumulative transdermal rate.

As shown in FIG. 28, at 1 hour, fluorescent signals were detected by a 1 mg/mL recombinant small-molecule collagen 3A5D29N and the recombinant type-III collagen in the full-thickness skin model, whereas no fluorescent signals were detected for hydrolyzed hyaluronic acid and fish skin gelatin at the same concentrations. At this point, the FTIC-labeled recombinant small-molecule collagen sample had permeated into an epidermis and entered skin but had not yet fully penetrated an entire skin model tissue. Analysis of fluorescence signal intensity revealed that the fluorescence signal of the recombinant small-molecule collagen 3A5D29N was significantly stronger than those of the other three samples, indicating that a transdermal penetration rate thereof was significantly superior to that of the recombinant type-III collagen, the hydrolyzed hyaluronic acid, and the fish skin collagen. Simultaneously, as shown in FIG. 29, at the 12th hour, the recombinant small-molecule collagen sample had fully penetrated the full-thickness skin model tissue. As the administered concentration increased, the diffusion percentage (i.e., the cumulative transdermal rate) of the recombinant small-molecule collagen 3A5D29N continued to rise. At 1 mg/mL, the diffusion percentage (i.e., the cumulative transdermal rate) was essentially consistent with that of the hydrolyzed hyaluronic acid at the same concentration, both exceeding 40%. These results demonstrated that the recombinant small-molecule collagen could achieve rapid transdermal penetration in a short timeframe and maintain a high cumulative transdermal rate over an extended period. The experimental results of the full-thickness skin model tissue in the embodiment validated the excellent skin penetration property of the small-molecule collagen of the present invention. The present invention also supported the broad prospects for the recombinant small-molecule collagen in the development of a related product, such as applications in a cosmetic.

| | | |
|---|---|---|
| 0-1 | PCT/RO/134 Form | |
| | Explanations on microorganisms or other biological materials collected (Clause II of Article 13 of PCT Detailed Rules) | |
| 0-1-1 | Software version | i-system |
| | | Version 1.0.26 20240201 MT/FOP 20140331/0.20.5.21 |
| 0-2 | International application number | PCT/CN2024/082594 |
| 0-3 | Archive number of the applicant or agent | ZZLPCT230098 |
| | | |
| 1 | The following explanations are related to microorganisms or other biological materials mentioned herein in the instructions for this application: | |
| 1-1 | Page | 3 |
| 1-2 | Row No.: | 11 |
| 1-3 | Collection matter | |
| 1-3-1 | Name of the collection organization | China General Microbiological Culture Collection Center (CGMCC) |
| 1-3-2 | Address of the collection organization | Institute of Microbiology, Chinese Academy of Sciences, No.3, Compound 1, Beichen West Road, Chaoyang District, Beijing 100101 |
| 1-3-3 | Collection date | September 26, 2022(26.09.2022) |
| 1-3-4 | Collection No. | CGMCC No.25823 |
| 1-4 | Supplementary explanations | |
| 1-5 | These explanations apply to the following·designated·countries | All designated countries |
| 1-6 | Explanations submitted separately | |
| | These explanations will subsequently be submitted to the International Bureau | |
| 2 | The following explanations are related to microorganisms or other biological materials mentioned herein in the instructions for this application: | |
| 2-1 | Page | 3 |
| 2-2 | Row No.: | 11 |
| 2-3 | Collection matter | |
| 2-3-1 | Name of the collection organization | China General Microbiological Culture Collection Center (CGMCC) |
| 2-3-2 | Address of the collection organization | Institute of Microbiology, Chinese Academy of Sciences, No.3, Compound 1, Beichen West Road, Chaoyang District, Beijing 100101 |
| 2-3-3 | Collection date | September 26, 2022(26.09.2022) |
| 2-3-4 | Collection No. | CGMCC No.25811 |
| 2-4 | Supplementary explanations | |
| 2-5 | These explanations apply to the following·designated·countries | All designated countries |
| 2-6 | Explanations submitted separately | |
| | These explanations will subsequently be submitted to the International Bureau | |
| 3 | The following explanations are related to microorganisms or other biological materials mentioned herein in the instructions for this application: | |
| 3-1 | Page | 3 |
| 3-2 | Row No.: | 11 |
| 3-3 | Collection matter | |
| 3-3-1 | Name of the collection organization | China General Microbiological Culture Collection Center (CGMCC) |
| 3-3-2 | Address of the collection organization | Institute of Microbiology, Chinese Academy of Sciences, No.3, Compound 1, Beichen West Road, Chaoyang District, Beijing 100101 |
| 3-3-3 | Collection date | September 26, 2022(26.09.2022) |
| 3-3-4 | Collection No. | CGMCC No.25824 |
| 3-4 | Supplementary explanations | |
| 3-5 | These explanations apply to the following·designated·countries | All designated countries |
| 3-6 | Explanations submitted separately | |
| | These explanations will subsequently be submitted to the International Bureau | |
| 4 | The following explanations are related to microorganisms or other biological materials mentioned herein in the instructions for this application: | |
| 4-1 | Page | 3 |
| 4-2 | Row No.: | 11 |
| 4-3 | Collection matter | |
| 4-3-1 | Name of the collection organization | China General Microbiological Culture Collection Center (CGMCC) |
| 4-3-2 | Address of the collection organization | Institute of Microbiology, Chinese Academy of Sciences, No.3, Compound 1, Beichen West Road, Chaoyang District, Beijing 100101 |
| 4-3-3 | Collection date | September 26, 2022(26.09.2022) |
| 4-3-4 | Collection No. | CGMCC No.25812 |
| 4-4 | Supplementary explanations | |
| 4-5 | These explanations apply to the following·designated·countries | All designated countries |
| 4-6 | Explanations submitted separately | |
| | These explanations will subsequently be submitted to the International Bureau | |
| 5 | The following explanations are related to microorganisms or other biological materials mentioned herein in the instructions for this application: | |
| 5-1 | Page | 3 |
| 5-2 | Row No.: | 12 |
| 5-3 | Collection matter | |
| 5-3-1 | Name of the collection organization | China General Microbiological Culture Collection Center (CGMCC) |
| 5-3-2 | Address of the collection | Institute of Microbiology, Chinese Academy of |
| | organization | Sciences, No.3, Compound 1, Beichen West Road, Chaoyang District, Beijing 100101 |
| 5-3-3 | Collection date | September 26, 2022(26.09.2022) |
| 5-3-4 | Collection No. | CGMCC No.25825 |
| 5-4 | Supplementary explanations | |
| 5-5 | These explanations apply to the following·designated·countries | All designated countries |
| 5-6 | Explanations submitted separately | |
| | These explanations will subsequently be submitted to the International Bureau | |
| 6 | The following explanations are related to microorganisms or other biological materials mentioned herein in the instructions for this application: | |
| 6-1 | Page | 3 |
| 6-2 | Row No.: | 12 |
| 6-3 | Collection matter | |
| 6-3-1 | Name of the collection organization | China General Microbiological Culture Collection Center (CGMCC) |
| 6-3-2 | Address of the collection organization | Institute of Microbiology, Chinese Academy of Sciences, No.3, Compound 1, Beichen West Road, Chaoyang District, Beijing 100101 |
| 6-3-3 | Collection date | September 26, 2022(26.09.2022) |
| 6-3-4 | Collection No. | CGMCC No.25813 |
| 6-4 | Supplementary explanations | |
| 6-5 | These explanations apply to the following·designated·countries | All designated countries |
| 6-6 | Explanations submitted separately | |
| | These explanations will subsequently be submitted to the International Bureau | |
| 7 | The following explanations are related to microorganisms or other biological materials mentioned herein in the instructions for this application: | |
| 7-1 | Page | 3 |
| 7-2 | Row No.: | 12 |
| 7-3 | Collection matter | |
| 7-3-1 | Name of the collection organization | China General Microbiological Culture Collection Center (CGMCC) |
| 7-3-2 | Address of the collection organization | Institute of Microbiology, Chinese Academy of Sciences, No.3, Compound 1, Beichen West Road, Chaoyang District, Beijing 100101 |
| 7-3-3 | Collection date | September 26, 2022(26.09.2022) |
| 7-3-4 | Collection No. | CGMCC No.25826 |
| 7-4 | Supplementary explanations | |
| 7-5 | These explanations apply to the following·designated·countries | All designated countries |
| 7-6 | Explanations submitted separately | |
| | These explanations will subsequently be submitted to the International Bureau | |
| 8 | The following explanations are related to microorganisms or other biological materials mentioned herein in the instructions for this application: | |
| 8-1 | Page | 3 |
| 8-2 | Row No.: | 12 |
| 8-3 | Collection matter | |
| 8-3-1 | Name of the collection organization | China General Microbiological Culture Collection Center (CGMCC) |
| 8-3-2 | Address of the collection | Institute of Microbiology, Chinese Academy of |
| | organization | Sciences, No.3, Compound 1, Beichen West Road, Chaoyang District, Beijing 100101 |
| 8-3-3 | Collection date | September 26, 2022(26.09.2022) |
| 8-3-4 | Collection No. | CGMCC No. 25814 |
| 8-4 | Supplementary explanations | |
| 8-5 | These explanations apply to the following·designated·countries | All designated countries |
| 8-6 | Explanations submitted separately | |
| | These explanations will subsequently be submitted to the International Bureau | |
| 9 | The following explanations are related to microorganisms or other biological materials mentioned herein in the instructions for this application: | |
| 9-1 | Page | 6 |
| 9-2 | Row No.: | 17 |
| 9-3 | Collection matter | |
| 9-3-1 | Name of the collection organization | China General Microbiological Culture Collection Center (CGMCC) |
| 9-3-2 | Address of the collection organization | Institute of Microbiology, Chinese Academy of Sciences, No.3, Compound 1, Beichen West Road, Chaoyang District, Beijing 100101 |
| 9-3-3 | Collection date | September 26, 2022(26.09.2022) |
| 9-3-4 | Collection No. | CGMCC No.25815 |
| 9-4 | Supplementary explanations | |
| 9-5 | These explanations apply to the following·designated·countries | All designated countries |
| 9-6 | Explanations submitted separately | |
| | These explanations will subsequently be submitted to the International Bureau | |
| 10 | The following explanations are related to microorganisms or other biological materials mentioned herein in the instructions for this application: | |
| 10-1 | Page | 6 |
| 10-2 | Row No.: | 17 |
| 10-3 | Collection matter | |
| 10-3-1 | Name of the collection organization | China General Microbiological Culture Collection Center (CGMCC) |
| 10-3-2 | Address of the collection organization | Institute of Microbiology, Chinese Academy of Sciences, No.3, Compound 1, Beichen West Road, Chaoyang District, Beijing 100101 |
| 10-3-3 | Collection date | September 26, 2022(26.09.2022) |
| 10-3-4 | Collection No. | CGMCC No.25816 |
| 10-4 | Supplementary explanations | |
| 10-5 | These explanations apply to the following·designated·countries | All designated countries |
| 10-6 | Explanations submitted separately | |
| | These explanations will subsequently be submitted to the International Bureau | |
| 11 | The following explanations are related to microorganisms or other biological materials mentioned herein in the instructions for this application: | |
| 11-1 | Page | 6 |
| 11-2 | Row No.: | 17 |
| 11-3 | Collection matter | |
| 11-3-1 | Name of the collection organization | China General Microbiological Culture Collection Center (CGMCC) |
| 11-3-2 | Address of the collection | Institute of Microbiology, Chinese Academy of |
| | organization | Sciences, No.3, Compound 1, Beichen West Road, Chaoyang District, Beijing 100101 |
| 11-3-3 | Collection date | September 26, 2022(26.09.2022) |
| 11-3-4 | Collection No. | CGMCC No.25817 |
| 11-4 | Supplementary explanations | |
| 11-5 | These explanations apply to the following·designated·countries | All designated countries |
| 11-6 | Explanations submitted separately | |
| | These explanations will subsequently be submitted to the International Bureau | |
| 12 | The following explanations are related to microorganisms or other biological materials mentioned herein in the instructions for this application: | |
| 12-1 | Page | 6 |
| 12-2 | Row No.: | 18 |
| 12-3 | Collection matter | |
| 12-3-1 | Name of the collection organization | China General Microbiological Culture Collection Center (CGMCC) |
| 12-3-2 | Address of the collection organization | Institute of Microbiology, Chinese Academy of Sciences, No.3, Compound 1, Beichen West Road, Chaoyang District, Beijing 100101 |
| 12-3-3 | Collection date | September 26, 2022(26.09.2022) |
| 12-3-4 | Collection No. | CGMCC No.25818 |
| 12-4 | Supplementary explanations | |
| 12-5 | These explanations apply to the following·designated·countries | All designated countries |
| 12-6 | Explanations submitted separately | |
| | These explanations will subsequently be submitted to the International Bureau | |
| 13 | The following explanations are related to microorganisms or other biological materials mentioned herein in the instructions for this application: | |
| 13-1 | Page | 4 |
| 13-2 | Row No.: | 6 |
| 13-3 | Collection matter | |
| 13-3-1 | Name of the collection organization | China General Microbiological Culture Collection Center (CGMCC) |
| 13-3-2 | Address of the collection organization | Institute of Microbiology, Chinese Academy of Sciences, No.3, Compound 1, Beichen West Road, Chaoyang District, Beijing 100101 |
| 13-3-3 | Collection date | September 26, 2022(26.09.2022) |
| 13-3-4 | Collection No. | CGMCC No.25819 |
| 13-4 | Supplementary explanations | |
| 13-5 | These explanations apply to the following·designated·countries | All designated countries |
| 13-6 | Explanations submitted separately | |
| | These explanations will subsequently be submitted to the International Bureau | |
| 14 | The following explanations are related to microorganisms or other biological materials mentioned herein in the instructions for this application: | |
| 14-1 | Page | 4 |
| 14-2 | Row No.: | 6 |
| 14-3 | Collection matter | |
| 14-3-1 | Name of the collection organization | China General Microbiological Culture Collection Center (CGMCC) |
| 14-3-2 | Address of the collection | Institute of Microbiology, Chinese Academy of |
| | organization | Sciences, No.3, Compound 1, Beichen West Road, Chaoyang District, Beijing 100101 |
| 14-3-3 | Collection date | September 26, 2022(26.09.2022) |
| 14-3-4 | Collection No. | CGMCC No.25821 |
| 14-4 | Supplementary explanations | |
| 14-5 | These explanations apply to the following·designated·countries | All designated countries |
| 14-6 | Explanations submitted separately | |
| | These explanations will subsequently be submitted to the International Bureau | |
| 15 | The following explanations are related to microorganisms or other biological materials mentioned herein in the instructions for this application: | |
| 15-1 | Page | 4 |
| 15-2 | Row No.: | 6 |
| 15-3 | Collection matter | |
| 15-3-1 | Name of the collection organization | China General Microbiological Culture Collection Center (CGMCC) |
| 15-3-2 | Address of the collection organization | Institute of Microbiology, Chinese Academy of Sciences, No.3, Compound 1, Beichen West Road, Chaoyang District, Beijing 100101 |
| 15-3-3 | Collection date | September 26, 2022(26.09.2022) |
| 15-3-4 | Collection No. | CGMCC No.25827 |
| 15-4 | Supplementary explanations | |
| 15-5 | These explanations apply to the following·designated·countries | All designated countries |
| 15-6 | Explanations submitted separately | |
| | These explanations will subsequently be submitted to the International Bureau | |
| 16 | The following explanations are related to microorganisms or other biological materials mentioned herein in the instructions for this application: | |
| 16-1 | Page | 4 |
| 16-2 | Row No.: | 7 |
| 16-3 | Collection matter | |
| 16-3-1 | Name of the collection organization | China General Microbiological Culture Collection Center (CGMCC) |
| 16-3-2 | Address of the collection organization | Institute of Microbiology, Chinese Academy of Sciences, No.3, Compound 1, Beichen West Road, Chaoyang District, Beijing 100101 |
| 16-3-3 | Collection date | September 26, 2022(26.09.2022) |
| 16-3-4 | Collection No. | CGMCC No.25829 |
| 16-4 | Supplementary explanations | |
| 16-5 | These explanations apply to the following·designated·countries | All designated countries |
| 16-6 | Explanations submitted separately | |
| | These explanations will subsequently be submitted to the International Bureau | |
| 17 | The following explanations are related to microorganisms or other biological materials mentioned herein in the instructions for this application: | |
| 17-1 | Page | 4 |
| 17-2 | Row No.: | 7 |
| 17-3 | Collection matter | |
| 17-3-1 | Name of the collection organization | China General Microbiological Culture Collection Center (CGMCC) |
| 17-3-2 | Address of the collection | Institute of Microbiology, Chinese Academy of |
| | organization | Sciences, No.3, Compound 1, Beichen West Road, Chaoyang District, Beijing 100101 |
| 17-3-3 | Collection date | September 26, 2022(26.09.2022) |
| 17-3-4 | Collection No. | CGMCC No.25828 |
| 17-4 | Supplementary explanations | |
| 17-5 | These explanations apply to the following·designated·countries | All designated countries |
| 17-6 | Explanations submitted separately | |
| | These explanations will subsequently be submitted to the International Bureau | |
| 18 | The following explanations are related to microorganisms or other biological materials mentioned herein in the instructions for this application: | |
| 18-1 | Page | 4 |
| 18-2 | Row No.: | 7 |
| 18-3 | Collection matter | |
| 18-3-1 | Name of the collection organization | China General Microbiological Culture Collection Center (CGMCC) |
| 18-3-2 | Address of the collection organization | Institute of Microbiology, Chinese Academy of Sciences, No.3, Compound 1, Beichen West Road, Chaoyang District, Beijing 100101 |
| 18-3-3 | Collection date | September 26, 2022(26.09.2022) |
| 18-3-4 | Collection No. | CGMCC No.25820 |
| 18-4 | Supplementary explanations | |
| 18-5 | These explanations apply to the following·designated·countries | All designated countries |
| 18-6 | Explanations submitted separately | |
| | These explanations will subsequently be submitted to the International Bureau | |
| 19 | The following explanations are related to microorganisms or other biological materials | |
| | mentioned herein in the instructions for this application: | |
| 19-1 | Page | 4 |
| 19-2 | Row No.: | 7 |
| 19-3 | Collection matter | |
| 19-3-1 | Name of the collection organization | China General Microbiological Culture Collection Center (CGMCC) |
| 19-3-2 | Address of the collection organization | Institute of Microbiology, Chinese Academy of Sciences, No.3, Compound 1, Beichen West Road, Chaoyang District, Beijing 100101 |
| 19-3-3 | Collection date | September 26, 2022(26.09.2022) |
| 19-3-4 | Collection No. | CGMCC No.25822 |
| 19-4 | Supplementary explanations | |
| 19-5 | These explanations apply to the following·designated·countries | All designated countries |
| 19-6 | Explanations submitted separately | |
| | These explanations will subsequently be submitted to the International Bureau | |

| Filled in by the receiving office | | |
|---|---|---|
| 0-4 | This form is received with the international application: (Yes or No) | |
| 0-4-1 | Authorized official | |

| Filled in by the International Bureau | | |
|---|---|---|
| 0-5 | Date of receipt of this form by the International Bureau: | |
| 0-5-1 | Authorized official | |

## Claims

1. An expression system of a recombinant small-molecule collagen, **characterized by** comprising a chassis cell or a chassis engineered strain obtained by transforming a host strain with a positioning fusion functional protein linker vector; and a tandem repeat expression sequence of the recombinant small-molecule collagen; wherein
the tandem repeat expression sequences are connected in series and repeated by taking as basic units the recombinant small-molecule collagen, or an artificially designed collagen with a typical G-X-Y triplet structure, a collagen combined by two or more regions of a human collagen sequence; recognition and cleavage sites of Kex2 and CPB enzymes are provided between every two adjacent basic units in the tandem repeat expression sequence;
the positioning fusion functional protein comprises the CPB enzyme and a functional region with intracellular membrane positioning or conversion and transport functions among respective organelles; the positioning fusion functional protein further comprises a connecting sequence for connecting the CPB enzyme to the functional region sequence with the intracellular membrane positioning or the conversion and transport functions among the respective organelles during fusion expression;
the positioning fusion functional protein is an amino acid sequence as shown in SEQ ID NO: 22, SEQ ID NO: 24, SEQ ID NO: 26, or SEQ ID NO: 28;
the chassis cell or the chassis engineered strain is selected from a yeast.

2. The expression system according to claim 1, **characterized in that** recognition and cleavage sites of an Ste13 enzyme is further capable of being comprised between the every two adjacent basic units in the tandem repeat expression sequence.

3. The expression system according to claim 2, **characterized in that** a site recognized and cleaved by a Kex2 enzyme comprises a KR or RR dibasic amino acid residue, followed by an EA, an EAEA, or other amino acid residues that facilitate recognition and cleavage by the Kex2 enzyme or the Ste13 enzyme.

4. The expression system according to claim 1, **characterized in that** a site recognized and cleaved by the CPB enzyme comprises a basic amino acid residue at a carboxy terminus of a protein, comprising K and R.

5. The expression system according to any one of claims 1 to 4, **characterized in that** the recombinant small-molecule collagen is the amino acid sequence shown in SEQ ID NO: 1, SEQ ID NO: 3, SEQ ID NO: 5, SEQ ID NO: 7, SEQ ID NO: 63, or SEQ ID NO: 67.

6. The expression system according to claim 5, **characterized in that** a carboxyl terminus of the recombinant small-molecule collagen comprises a plurality of Hises.

7. The expression system according to claim 6, **characterized in that** the recombinant small-molecule collagen comprises amino acid sequences shown in SEQ ID NO: 2, SEQ ID NO: 4, SEQ ID NO: 6, and SEQ ID NO: 8.

8. The expression system according to claim 1, **characterized in that** the tandem repeat expression sequence comprises the sequence shown in SEQ ID NO: 30, SEQ ID NO: 33, SEQ ID NO: 36, SEQ ID NO: 39, SEQ ID NO: 42, SEQ ID NO: 45, SEQ ID NO: 48, SEQ ID NO: 51, SEQ ID NO: 54, SEQ ID NO: 57, SEQ ID NO: 60, SEQ ID NO: 64, or SEQ ID NO: 68.

9. The expression system according to claim 8, **characterized in that** a nucleic acid for encoding the tandem repeat expression sequence is sequences shown in SEQ ID NOS: 71-81 or degenerate sequences thereof.

10. The expression system according to claim 1, **characterized in that** the CPB enzyme is derived from human or a rat.

11. The expression system according to claim 10, **characterized in that** sequences of the CPB enzyme are as shown in SEQ ID NOS: 17-18.

12. The expression system according to claim 1, **characterized in that** the functional region sequence with the intracellular membrane positioning or the conversion and transport functions among the respective organelles is derived from a Kex2 enzyme of Saccharomyces cerevisiae or Pichia pastoris.

13. The expression system according to claim 12, **characterized in that** the functional region sequences with the intracellular membrane positioning or the conversion and transport functions among the organelles are as shown in SEQ ID NOS: 19-20.

14. The expression system according to claim 1, **characterized in that** the connecting sequence is a Linker sequence such as GGSGSGSGGS as shown in SEQ ID NO: 21.

15. The expression system according to claim 1, **characterized in that** a nucleic acid for encoding the positioning fusion functional protein is nucleotide sequences shown in SEQ ID NO: 23, SEQ ID NO: 25, SEQ ID NO: 27, or SEQ ID NO: 29, or degenerate sequences thereof.

16. The expression system according to claim 1, **characterized in that** the chassis cell or the chassis engineered strain is deposited at China General Microbiological Culture Collection Center (CGMCC), with deposit numbers: CGMCC No. 25815, CGMCC No. 25817, CGMCC No. 25816, CGMCC No. 25818.

17. Application of the expression system according to any one of claims 1-16 for obtaining a recombinant small-molecule collagen.

18. A method for preparing a recombinant small-molecule collagen, **characterized in that** the method employs the expression system according to any one of claims 1-16, the method comprises:
constructing a tandem repeat expression sequence of the recombinant small-molecule collagen; wherein the tandem repeat expression sequences are connected in series and repeated by taking as basic units the recombinant small-molecule collagen, an artificially designed collagen with a typical G-X-Y triplet structure, or a collagen combined by two or more regions of a human collagen sequence; and recognition and cleavage sites of Kex2 and CPB enzymes are provided between every two adjacent basic units in the tandem repeat expression sequence;
constructing a positioning fusion functional protein; and transforming a positioning fusion functional protein-linked vector into a host strain to obtain a chassis cell or a chassis engineered strain;
transferring the tandem repeat expression sequence of the recombinant small-molecule collagen into a chassis cell or a chassis engineered strain after being connected to an expression vector, to obtain a recombinant engineered strain containing or expressing the recombinant small-molecule collagen; and inducing an expression through fermentation to obtain the recombinant small-molecule collagen.

19. The method according to claim 18, **characterized in that** the recombinant engineered strain for containing or expressing the recombinant small-molecule collagen is deposited at China General Microbiological Culture Collection Center, with deposit numbers of CGMCC No. 25819, CGMCC No. 25821, CGMCC No. 25827, CGMCC No. 25829, CGMCC No. 25828, CGMCC No. 25820, CGMCC No. 25822.

20. The method according to claim 18, **characterized in that** the recombinant small-molecule collagen obtained by the method is the amino acid sequence shown in SEQ ID NO: 32, SEQ ID NO: 35, SEQ ID NO: 38, SEQ ID NO: 41, SEQ ID NO: 44, SEQ ID NO: 47, SEQ ID NO: 50, SEQ ID NO: 53, SEQ ID NO: 56, SEQ ID NO: 59, SEQ ID NO: 62, SEQ ID NO: 66, or SEQ ID NO: 70.

21. A recombinant small-molecule collagen obtained by the method according to any one of claims 18-20.

22. Use of the recombinant small-molecule collagen obtained by the method according to any one of claims 18-20 or the recombinant small-molecule collagen according to claim 21 in preparation of a biomaterial, a tissue engineering product, or a cosmetic.
